# EUROPEAN PATENT SPECIFICATION

(11) **EP 4 219 463 B1**
(45) Date of publication and mention of the grant of the patent: **15.01.2025**
(21) Application number: 22855515.7
(22) Date of filing: 12.08.2022
(51) Int. Cl.: C07D 401/14, C07D 213/64, C07D 405/14, A61P 29/00, A61K 31/33

(54) **TERPYRIDINE DIKETONE COMPOUND OR SALT THEREOF, PREPARATION METHOD THEREFOR AND APPLICATION THEREOF**
TERPYRIDINDIKETONVERBINDUNG ODER SALZ DAVON, HERSTELLUNGSVERFAHREN DAFÜR UND ANWENDUNG DAVON
COMPOSÉ DE TERPYRIDINE DICÉTONE OU SON SEL, SON PROCÉDÉ DE PRÉPARATION ET SON UTILISATION

(30) Priority: 13.08.2021 CN 202110931085; 30.09.2021 CN 202111156542; 02.11.2021 CN 202111288455; 28.07.2022 CN 202210898817
(43) Date of publication of application: 02.08.2023
(73) Proprietor: Shenzhen Salubris Pharmaceuticals Co., Ltd., Shenzhen, Guangdong 518017 (CN)
(72) Inventor: WU, Junjun, Shenzhen, Guangdong 518017 (CN); LU, Yinsuo, Shenzhen, Guangdong 518017 (CN); XIAO, Ying, Shenzhen, Guangdong 518017 (CN); HONG, Zexin, Shenzhen, Guangdong 518017 (CN); WANG, Liulin, Shenzhen, Guangdong 518017 (CN); SONG, Zenan, Shenzhen, Guangdong 518017 (CN)
(74) Representative: karo IP
(86) International application number: PCT/CN2022/111949
(87) International publication number: WO 2023/016535

(56) References cited:
- WO-A1-2023/283338
- CN-A- 103 391 718
- CN-A- 105 263 326
- US-A1- 2013 143 906

## Description

### Technical Field

The present invention belongs to the technical field of chemical drugs, and relates to a terpyridinedione compound, or a racemate, an isomer, or a pharmaceutically acceptable salt thereof, and a preparation method and application thereof, wherein isomer means geometric or stereoisomeric isomer.

### Background

Mitogen-Activated Protein Kinase (MAPK) is a conserved family of enzymes that use a phosphorylation cascade to transmit and deliver external stimuli to produce a coordinated cellular response to the environment. The MAPK is a proline-induced serine/threonine-specific protein kinase that regulates cellular activities such as gene expression, mitosis, differentiation and cell survival/apoptosis. Up to now, mammal MAPKs of four different categories have been identified, which are extracellular signal transduction kinase (ERK1 and ERK2), c-jun N-terminal kinase-1 (JNK1-3), p38MAPK (p38α, p38β, p38γ, and p38δ), and ERK5.

Scientific exploration of such pathway from biological, cellular, and in vivo perspectives is primarily implemented by the availability of a good-performance, selective small-molecule inhibitor of the p38MAPK. The small-molecule inhibitor targets an α subtype of the p38MAPK and targets β subtype to a lesser extent. The p38αMAPK is a main subtype participating immune reaction and inflammatory response. Therefore, functions of the p38MAPK are critical to the generation and activities of various pro-inflammatory cytokines in cells such as macrophages, monocytes, synoviocytes and endothelial cells. The pro-inflammatory cytokines include TNFα, IL-1, IL-6, and IL-8. The p38MAPK is also responsible for inducing key inflammatory enzymes, such as COX2 and iNOS, which respectively are main resources of eicosanoid and nitric oxide at sites of inflammation. In addition, the p38MAPK pathway regulates the expression of matrix metalloproteinase (MMP). The MMP includes MMP2, MMP9, and MMP13.

The use of selective and effective inhibitors has facilitated the discovery of a plurality of families of p38MAPK substrates. The p38MAPK substrates include transcription factors, MAPKAP kinases and other enzymes. The MAPKAP kinases (MK2, MK-3, and PRAK) are selectively phosphorylated by the p38MAPK, and the phosphorylation of MSK1/2, MNK1/2, and RSKb is catalyzed by the p38MAPK and ERK. Although the identification of the substrates is difficult due to the absence of specific inhibitors, it is thought that activation of the RSKb plays a role in cell survival.

Once being phosphorylated and activated by the p38MAPK, the MK-2, the MK-3, and the PRAK share similar substrate specificities. All of these kinases can phosphorylate small heat shock proteins Hsp27. Studies have shown that PRAK- and MK3-deficient mice do not show any tolerance to endotoxic shock or lipopolysaccharide (LPS)-induced reduction in cytokine production. In contrast, MK-2-deficient mice show a resistance to endotoxic shock and an impaired inflammatory response, as well as a significantly decreased production of cytokines such as TNFα, IFNy and IL-6. Thus, the p38/MK2 axis specifically is necessary and sufficient for mediating pro-inflammatory responses.

By means of p38:MK2 interaction and by using the MK2 as a p38 substrate, a new p38α inhibitor showing properties of interest is discovered (Davidson et al.). The inhibitor shows substrate selectivity by preventing p38α-dependent phosphorylation of MK2 (Ki app 300nM) and simultaneously retaining p38α-dependent phosphorylation of ATF2 (Ki app>20uM). Compared with a conventional p38ATP competitive inhibitor that blocks the p38-dependent phosphorylation of all of the p38 substrates, the new inhibitor is unique in function. A second independent research also describes the p38 inhibitor having unique mechanism performance. The work shows new mechanisms of selectively inhibiting the p38-dependent phosphorylation of the MK2. Different from the previous research by Davidson et al., unique compounds of these mechanisms compete with the ATP and stabilize p38/MK2 compounds.

CN105263326 and CN103391718 describe both p38:MK2 inhibitors differing structurally from the compounds of the present invention by the presence of a pyrimidine instead of pyridinone ring.

To sum up, the two research clearly prove such a concept that p38/MK2 axis block may be selectively implemented by using small molecule inhibitors. Compared with the conventional p38MAPK inhibitors, these p38/MK2 inhibitors should retain or improve efficiency and show improved safety features in animal models of disease or in human body clinical environments.

### Summary

In view of problems in the prior art, this application provides a compound, as a p38/MK2 inhibitor. The compound may inhibit the production of cytokine TNFα, so as to regulate related diseases such as inflammatory response.

According to a first aspect, this application provides a compound shown in a general formula (I), or a racemate, an isomer, or a pharmaceutically acceptable salt thereof:

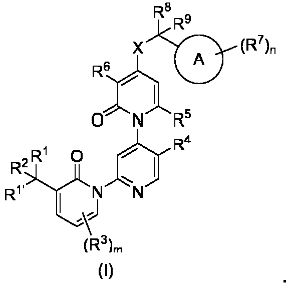

According to a second aspect, the present invention further provides a pharmaceutical composition. The pharmaceutical composition includes any of the therapeutically effective amount of the compound described above or a pharmaceutically acceptable salt thereof and a pharmaceutically acceptable carrier.

According to a third aspect, the present invention further provides an application of the therapeutically effective amount of the compound described above or a pharmaceutically acceptable salt thereof in the preparation of drugs for treating medical conditions. The disease is a p38/MK2 related disease. The compound may inhibit the production of cytokine TNFα, so as to regulate related diseases such as inflammatory response. Specifically, the medical conditions are selected from chronic inflammatory disease, acute inflammatory disease, or self-inflammatory disease.

Specifically, the present invention is achieved by the following technical solutions.

A compound shown in a general formula (I), or a racemate, or a geometric or stereoisomeric isomer, or a pharmaceutically acceptable salt thereof is provided. 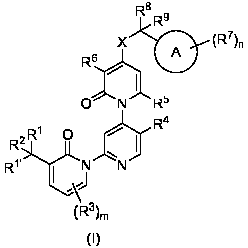
R¹ and R^{1'} are independently selected from hydrogen, halogen, alkyl, cycloalkyl, heterocycloalkyl, alkylcycloalkyl, alkyl heterocycloalkyl, phenyl, heteroaryl, haloalkyl, or cyano, or R¹ and R^{1'} are cyclized to the cycloalkyl or the heterocycloalkyl together.
R² is selected from hydrogen, halogen, hydroxyl, cyano, alkyl, alkoxy, alkoxyalkyl, or haloalkyl.
R³ is independently selected from hydrogen, alkyl, cycloalkyl, heterocycloalkyl, halogen, alkoxy, cyano, haloalkoxy, or sulfonyl.
m is 0, 1, 2, or 3.
R⁴ is selected from hydrogen, alkyl, cycloalkyl, alkoxy, or haloalkyl.
R⁵ is selected from hydrogen, alkyl, cycloalkyl, alkoxy, or haloalkyl.
R⁶ is selected from hydrogen, halogen, cyano, alkyl, cycloalkyl, or haloalkyl.
an A ring is selected from an aromatic ring or a heteroaromatic ring.
R⁷ is independently selected from hydrogen, halogen, cyano, alkyl, cycloalkyl, haloalkyl, alkoxy, haloalkoxy, or sulfonyl.
n is 0, 1, 2, 3, 4, or 5.
X is O, CH₂ or NH.
R⁸ and R⁹ are independently selected from hydrogen, alkyl, cycloalkyl, alkylcycloalkyl, or haloalkyl, or R⁸ and R⁹ are cyclized to the cycloalkyl or the heterocycloalkyl together.

As a preferred technical solution of the present invention, the alkyl is selected from alkyl of C₁₋₆, and the alkyl of C1-6 is selected from methyl, ethyl, propyl, isopropyl, n-butyl, isobutyl, sec-butyl, tert-butyl, n-pentyl, sec-pentyl, 1-ethylpropyl, 2-methylbutyl , tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, or 1-ethylbutyl.

Alkoxy is selected from alkoxy of C₁₋₆, and the alkoxy of C₁₋₆ is selected from methoxy, ethoxy, propoxy, isopropoxy, n-butoxy, isobutoxy, sec-butoxy, tert-butoxy, n-pentyloxy, sec-pentyloxy, 1-ethylpropoxy, 2-methylbutoxy, tert-pentyloxy, 1,2-dimethylpropoxy, isopentyloxy, neopentyloxy, n-hexyloxy, isohexyloxy, sec-hexyloxy, tert-hexyloxy, neohexyloxy, 2-methylpentyloxy, 1,2-dimethylbutoxy, or 1-ethylbutoxy. Alkoxyalkyl is selected from C₁₋₄alkoxy-C₁₋₄alkyl, and the C₁₋₄alkoxy-C₁₋₄alkyl is further selected from methoxymethyl, methoxyethyl, methoxypropyl, methoxybutyl, ethoxymethyl, ethoxyethyl, ethoxypropyl, ethoxybutyl, propoxymethyl, propoxyethyl, propoxypropyl, propoxybutyl, butoxymethyl, butoxyethyl, butoxypropyl, or butoxybutyl.

As a preferred technical solution of the present invention, the cycloalkyl is selected from cycloalkyl of C₃₋₆, and the cycloalkyl of C₃₋₆ is selected from cyclopropyl, cyclobutyl, cyclopentyl, or cyclohexyl.

As a preferred technical solution of the present invention, the aromatic ring is selected from a four-membered ring, a fused ring containing the four-membered ring, a five-membered ring, a fused ring containing the five-membered ring, a six-membered ring, a fused ring containing the six-membered ring, a biphenyl type aromatic ring. The heteroaromatic ring means that one or more carbon atoms on the aromatic ring are substituted by a heteroatom.

The aromatic ring includes a benzene ring and a naphthalene ring.

The heteroaromatic ring includes indazole, quinoline, isoquinoline, quinoxaline, indole, isoindole, cinnoline, quinazoline, phthalazine, purine, naphthyridine, pteridine, benzofuran, benzothiophene, benzoxazole, benzothiazole, benzisoxazole, benzisothiazole, benzoxadiazole, benzothiadiazole, benzotriazole, benzotriazine, benzimidazole, pyrazinopyrazole, pyrazinopyrimidine, pyrazinopyridazine, pyrazinotriazine, pyrimidopyrazole, pyrimidazole, pyrimidotriazole, pyrimidotriazine, pyrimidopyridazine, pyridazinoimidazole, pyridazinopyrazole, pyridazinotriazole, pyridazinotriazine, triazinoimidazole, triazinopyrazole, triazinotriazole, pyridooxazole, pyridothiazole, pyridoisoxazole, pyridoisothiazole, pyridooxadiazole, pyridothiadiazole, pyridofuran, pyridopyrrole, pyrazinooxazole, pyrazinothiazole, pyrazinoisoxazole, pyrazinoisothiazole, pyrazinooxadiazole, pyrazinothiadiazole, pyrazinofuran, pyrazinopyrrole, pyrimidooxazole, pyrimidothiazole, pyrimidoisoxazole, pyrimidoisothiazole, pyrimidooxadiazole, pyrimidothiadiazole, pyrimidofuran, pyrimidopyrrole, pyridazinooxazole, pyridazinothiazole, pyridazinoisoxazole, pyridazinoisothiazole, pyridazinooxadiazole, pyridazinothiadiazole, pyridazinofuran, pyridazinopyrrole, triazinooxazole, triazinothiazole, triazinoisoxazole, triazinoisothiazole, triazinooxadiazole, triazinothiadiazole, triazinofuran, and triazinopyrrole.

Specifically, for example, the naphthidine is selected from 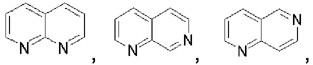 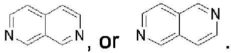 The pyridoimidazole is selected from 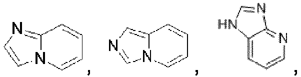 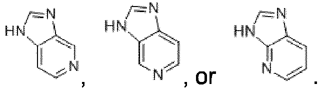 The pyrazinoimidazole is selected from 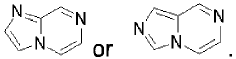 The pyrazinotriazole is selected from 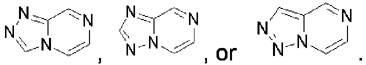 The pyrimidopyrazole is selected from 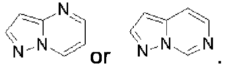 The pyrimidoimidazole is selected from 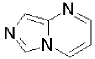 or 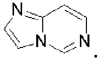 The pyrimidotriazole is selected from 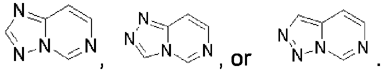 The pyridazinoimidazole is selected from 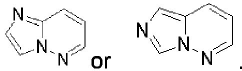 The pyridazinotriazole is selected from 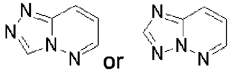 . The triazinoimidazole is selected from 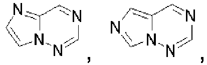 or 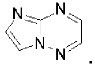 The pyridopyridazine is selected from 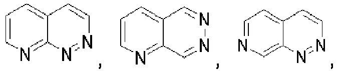 or 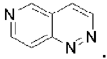 The pyridopyrazole is selected from 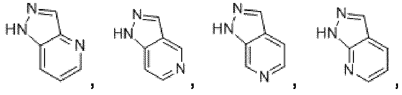 or 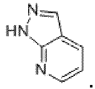 The pyridopyrimidine is selected from 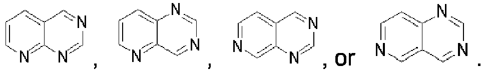 The pyridotriazine is selected from 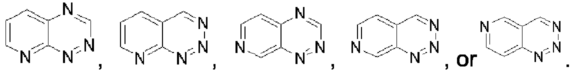 The pyrimidotriazine is selected from

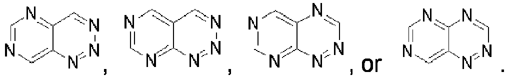

Specifically, for example, the heterocyclic alkyl 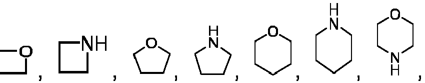 or

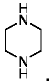

The heteroaryl is selected from

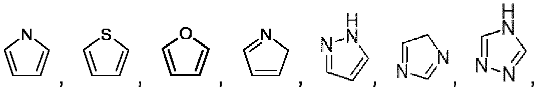

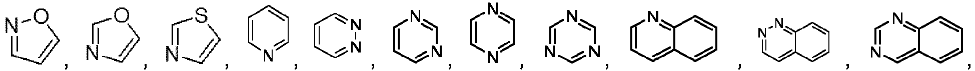

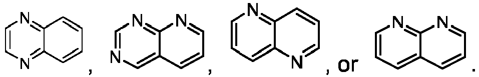

As a preferred technical solution of the present invention, the halogen is selected from fluorine, chlorine, bromine, or iodine.

The haloalkyl means that one or more hydrogen atoms on the alkyl aresubstituted by the halogen. The haloalkoxy means that one or more hydrogen atoms on the alkoxy aresubstituted by the halogen.

The heterocycloalkyl means that one or more carbon atoms on the cycloalkyl aresubstituted byaaheteroatom.

The alkylcycloalkylmeans that one or more hydrogen atoms on the cycloalkyl are substituted by an alkyl, and the alkylheterocycloalkylmeans that one or more hydrogen atoms on the heterocycloalkyl are substituted by an alkyl.

As a preferred technical solution of the present invention, a heteroatom is selected from nitrogen, oxygen, or sulfur, and there are one or more heteroatoms.

As a preferred technical solution of the present invention, the A ring is selected from

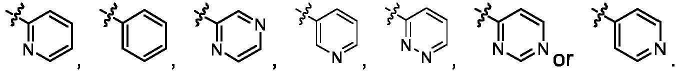

As a preferred technical solution of the present invention, the compound, or a racemate, a geometric or stereoisomeric isomer, or a pharmaceutically acceptable salt thereof has a structure of a formula (Ia), a formula (Ib), a formula (Ic), a formula (Id), a formula (le), a formula (If), or a formula (Ig).

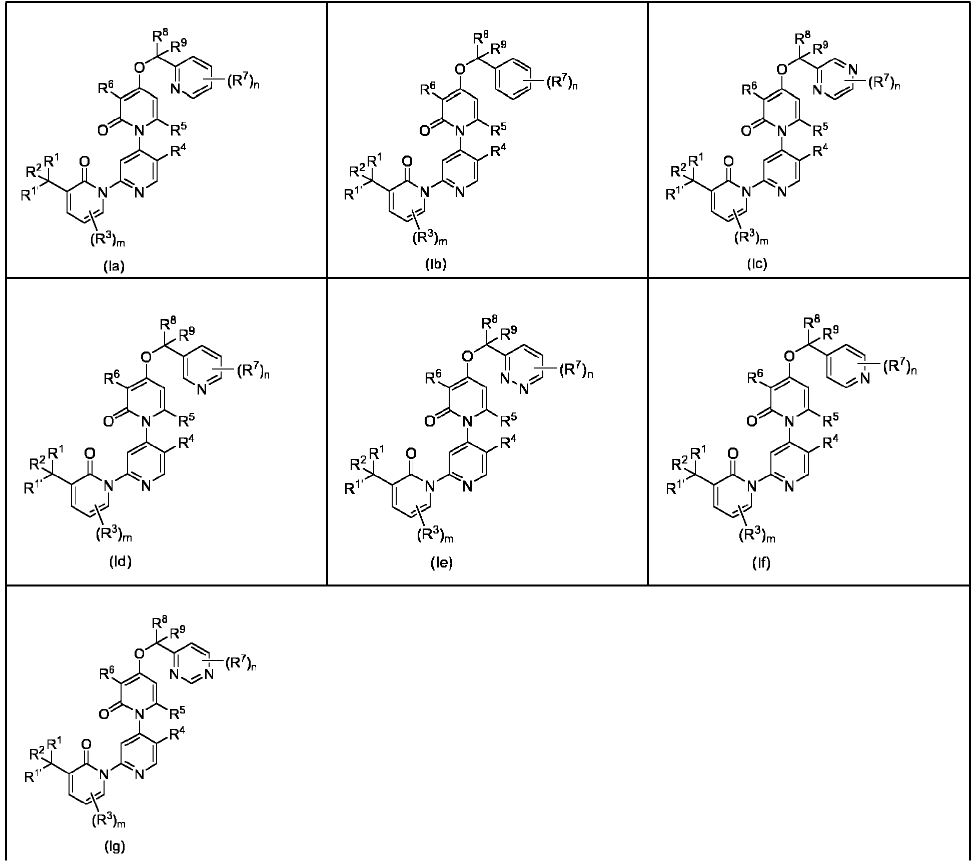

Wherein, m is 0, 1, 2, or 3; n is 0, 1, 2, 3, 4, or 5; and R¹, R^{1'}, R², R³, R⁴, R⁵, R⁶, R⁷, R⁸, and R⁹ are defined as above.

As a preferred technical solution of the present invention, m is 0 or 1; and n is 0, 1, or 2.

The A ring is selected from 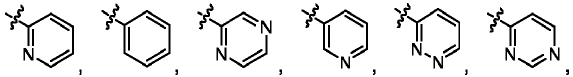 or 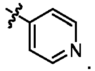
R¹ and R^{1'} are selected from hydrogen, methyl, or ethyl, and R¹ and R^{1'} are cyclized to 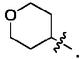
R² is selected from hydrogen, hydroxyl, difluoromethyl, or cyano.
R³ is selected from hydrogen, methyl, methoxy, chlorine, or bromine.
R⁴ is selected from methyl, cyclopropyl, methoxy, ethyl, fluoromethyl, or trifluoromethyl.
R⁵ is selected from methyl.
R⁶ is selected from chlorine, hydrogen, or bromine.
R⁷ is selected from hydrogen, fluorine, chlorine, bromine, cyclopropyl, methoxy, cyano, methyl, or trifluoromethyl.
R⁸ and R³ are hydrogen.

As a preferred technical solution of the present invention, the compound, or a racemate, a geometric or stereoisomeric isomer, or a pharmaceutically acceptable salt thereof is selected from the following.

| No. | Compound structure | No. | Compound structure |
|---|---|---|---|
| 1 | 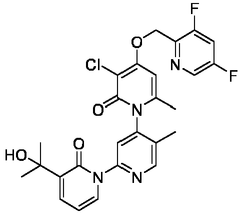 | 2 | 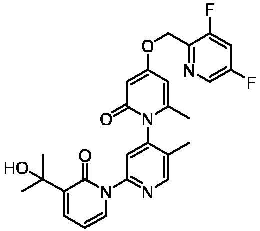 |
| 1A | 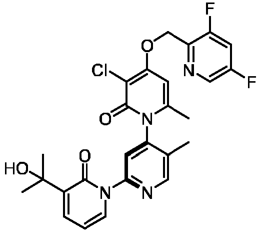 | 1B | 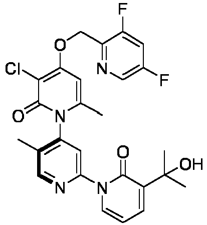 |
| 3 | 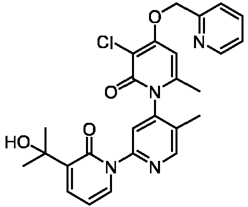 | 4 | 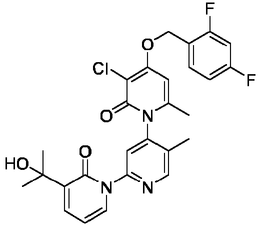 |
| 5 | 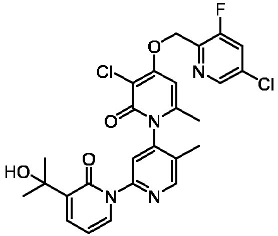 | 6 | 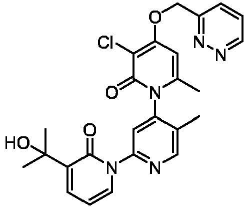 |
| 7 | 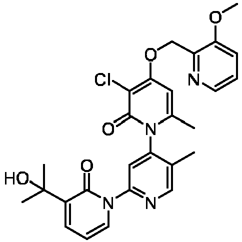 | 8 | 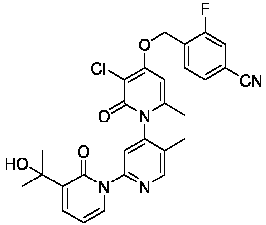 |
| 9 | 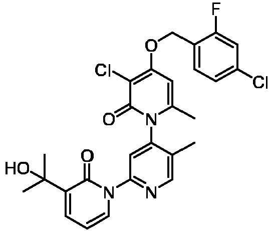 | 10 | 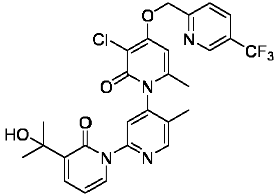 |
| 11 | 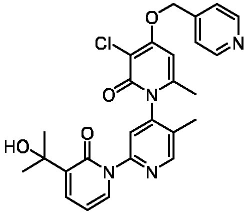 | 12 | 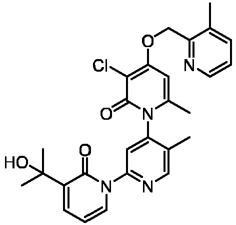 |
| 13 | 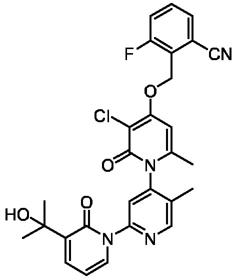 | 14 | 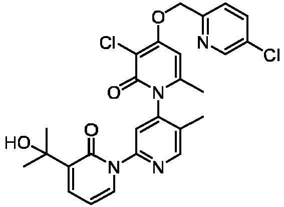 |
| 15 | 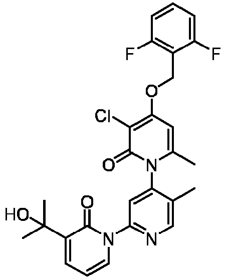 | 16 | 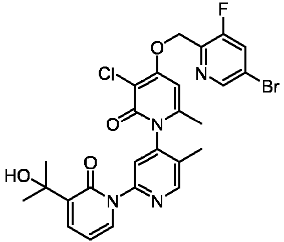 |
| 17 | 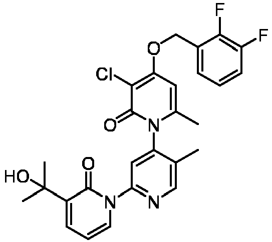 | 18 | 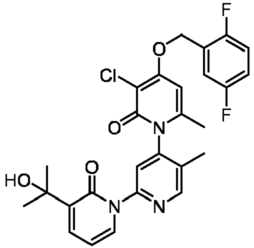 |
| 19 | 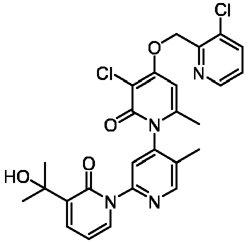 | 20 | 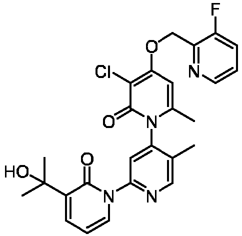 |
| 21 | 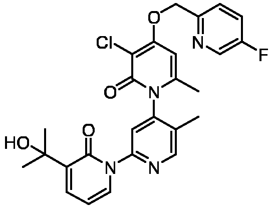 | 22 | 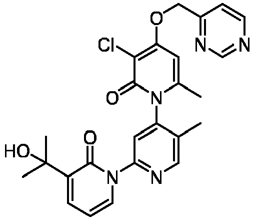 |
| 23 | 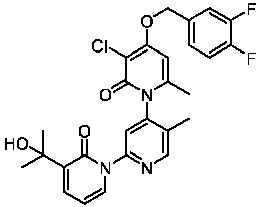 | 24 | 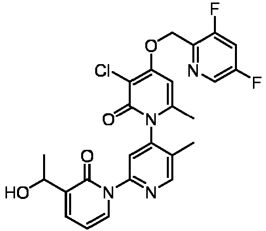 |
| 25 | 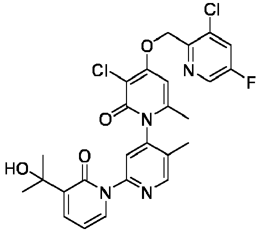 | 26 | 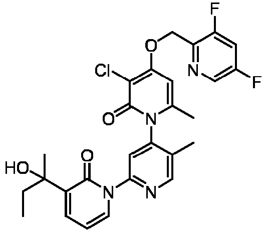 |
| 27 | 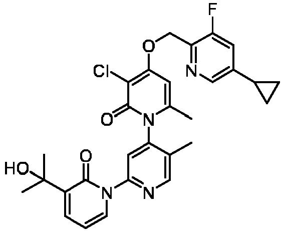 | 28 | 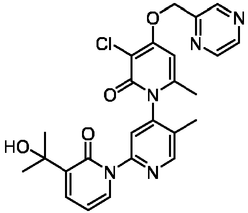 |
| 29 | 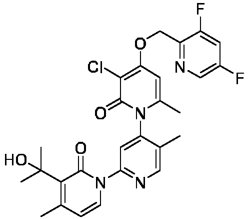 | 30 | 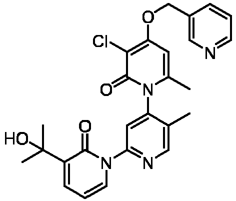 |
| 31 | 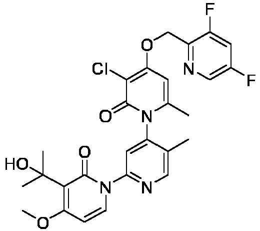 | 32 | 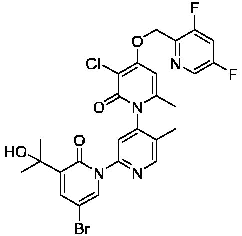 |
| 33 | 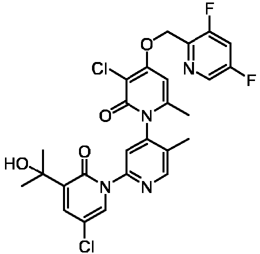 | 34 | 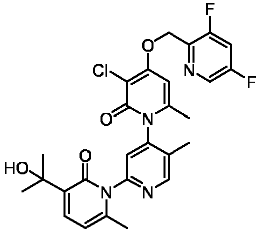 |
| 35 | 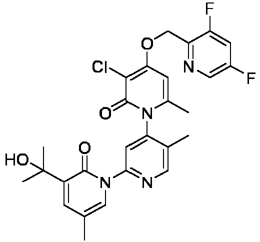 | 36 | 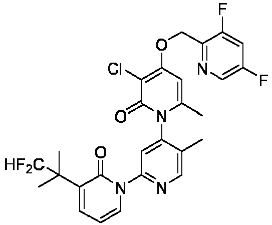 |
| 37 | 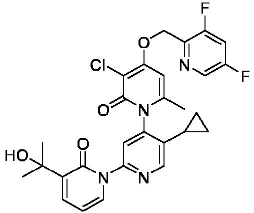 | 38 | 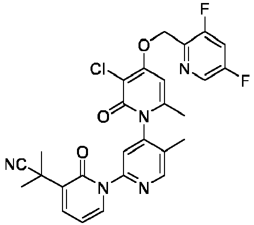 |
| 39 | 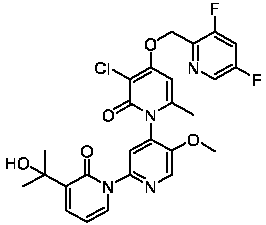 | 40 | 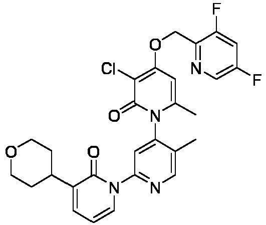 |
| 41 | 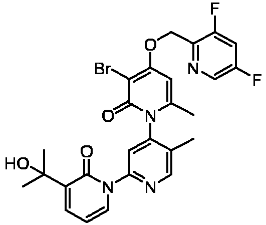 | 42 | 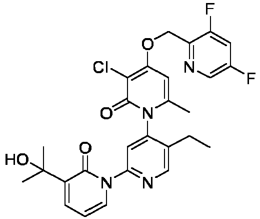 |
| 43 | 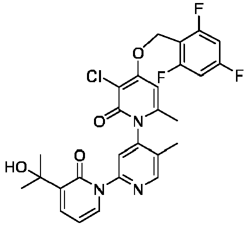 | 44 | 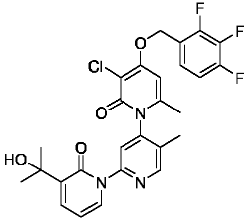 |
| 45 | 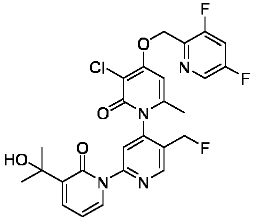 | 46 | 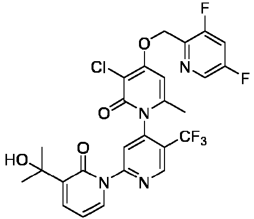 |

As a preferred technical solution of the present invention, the pharmaceutically acceptable salt refers to a salt prepared by the compound, or a racemate or an isomer thereof, and a pharmaceutically acceptable acid or base.

As a preferred technical solution of the present invention, more than one hydrogen atom of the compound, or a racemate, an isomer, or a pharmaceutically acceptable salt thereof is substituted by isotope deuterium.

The present invention further provides a pharmaceutical composition. The pharmaceutical composition includes the therapeutically effective amount of the compound, or a racemate, a geometric or stereoisomeric isomer, or a pharmaceutically acceptable salt thereof and a pharmaceutically acceptable carrier.

The present invention further provides an application of the compound, or a racemate, a geometric or stereoisomeric isomer, or a pharmaceutically acceptable salt thereof in the preparation of drugs for treating diseases. The diseases are p38/MK2 related diseases and specifically selected from a chronic inflammatory disease and an acute inflammatory disease. Preferably, the chronic inflammatory disease is rheumatoid arthritis.

Compared with compounds in the prior art, the compound of the present invention has improved TNFo activity, solubility, and in vivo PK effects.

For clarity, generic terms used in the description of compound are defined herein.

Unless otherwise specified, the following terms and phrases used herein are intended to have the following meanings. A specific term or phrase should not be considered indeterminate or unclear without specific definitions, but should be understood in its ordinary meaning. When a trade name appears herein, the trade name is intended to refer to a corresponding commodity or an active ingredient. As used herein, the term "pharmaceutically acceptable" aims at compounds, materials, compositions and/or dosage forms which, within the scope of sound medical judgment, are suitable for use in contact with human and animal tissue without excessive toxicity, irritation, allergic reactions or other problems or complications, and is commensurate with a reasonable benefit/risk ratio.

The term "pharmaceutically acceptable salt" is a salt of the compound of the present invention, and is prepared from the compound of the present invention having a specified substituent and a pharmaceutically acceptable acid or base.

Certain compounds of the present invention may exist in non-solvated or solvated forms, including hydrate forms. Generally, the solvated forms and the non-solvated forms are equivalent and are intended to be included within the scope of the present invention.

The compound of the present invention may exist in a specific geometric or stereoisomeric form. All of such compounds assumed in the present invention include cis and trans isomers, (-)- and (+)-enantiomers, (R)- and (S)-enantiomers, diastereomers isomers, (D)-isomers, (L)-isomers, atropisomers, and racemic mixtures thereof and other mixtures. The mixtures are, for example, mixtures enriched by the enantiomers or diastereomers. All of these mixtures are within the scope of the present invention. Substituents such as alkyl may have additional asymmetric carbon atoms. All of these isomers and mixtures thereof are included within the scope of the present invention.

Optically active (R)- and (S)-isomers, as well as D and L isomers, and atropisomers may be prepared by chiral synthesis or chiral reagents or other conventional technologies. An enantiomer of a certain compound in the present invention may be prepared by asymmetric synthesis or derivatization with chiral auxiliaries. The obtained diastereomeric mixtures are separated, and an auxiliary group is cleaved to provide the pure required enantiomer. Alternatively, when a molecule contains a basic functional group (such as amino) or an acidic functional group (such as carboxyl), a diastereomeric salt is formed with appropriate optically active acids or bases. Then, the diastereoisomers are split by means of conventional methods known in the art, and the pure enantiomers are recycled. In addition, the separation of the enantiomers and the diastereoisomers is generally completed by using chromatography. The chromatography adopts a chiral stationary phase, and is optionally combined with chemical derivatization (for example, generating carbamate from amines).

Atoms of the compound molecule of the present invention are isotopes, and effects of prolonging half-life, reducing a clearance rate, stabilizing metabolism and improving in vivo activities may be generally achieved through isotope derivatization. In addition, an implementation solution is included. At least one atom is substituted by an atom with a same atomic number (number of protons) and different mass numbers (sum of protons and neutrons). Examples of the isotopes included in the compound of the present invention include hydrogen atoms, carbon atoms, nitrogen atoms, oxygen atoms, phosphorus atoms, sulfur atoms, fluorine atoms, and chlorine atoms, which respectively include ²H, ³H, ¹³C, ¹⁴C, ¹⁵N, ¹⁷O, ¹⁸O, ³¹P, ³²P, ³⁵S, ¹⁸F, ³⁶Cl. In particular, radioisotopes that emit radiation with the decaying of the radioisotopes decay, such as ³H or ¹⁴C, may be used in the topographic anatomy examination of pharmaceutical preparations or in vivo compounds. Stable isotopes neither decaynor change with the amount of isotopes nor are the isotopes radioactive, so the isotopes are safe to use. When the atoms constituting the compound molecule of the present invention are isotopes, the isotopes can be converted according to general methods by substituting a reagent used in synthesis with a reagent containing the corresponding isotopes.

The compound of the present invention may include unnatural proportions of atomic isotopes at one or more atoms that constitute the compound. For example, the compound may be labeled with the radioisotopes, such as deuterium (²H), iodine-125 (¹²⁵ I) or C-14 (¹⁴C). The transformations of all of the isotopic compositions of the compound of the present invention, whether radioactive or not, are included within the scope of the present invention.

Further, one or more hydrogen atoms of the compound of the present invention are substituted with the isotope deuterium (²H). After deuterization, the compound of the present invention has the effects of prolonging the half-life, reducing the clearance rate, stabilizing the metabolism and improving the in vivo activity.

A method for preparing the isotopic derivatives generally includes a phase-transfer catalysis method. For example, a preferred deuterization method adopts the phase-transfer catalysis method (for example, tetraalkylammonium salt or NBu₄HSO₄). The methylene protons of a diphenylmethane compound are exchanged by using a phase-transfer catalyst, resulting in higher deuterium introduced through reduction with sodium deuterated borate than with deuterated silane (such as triethyldeuteratedsilane) or lewis acid such as aluminium chloride in the presence of an acid (such as methanesulfonic acid).

The term "pharmaceutically acceptable carrier" refers to any preparation carrier or medium that can deliver an effective amount of the active substance of the present invention without interfering with the biological activity of the active substance, and have no toxic side effects to hosts or patients. Representative carriers include water, oil, vegetables, minerals, cream base, lotion base, ointment base, and the like. These bases include suspending agents, thickening agents, penetration enhancers, and the like. Preparations of the bases are well known to those skilled in the field of cosmetics or regional medicines. For other information of the carrier, refer to Remington: The Science and Practice of Pharmacy, 21st Ed., Lippincott, Williams & Wilkins (2005).

The term "excipient" generally refers to a carrier, diluent and/or medium required to prepare an effective pharmaceutical composition.

The term "effective amount" or "therapeutically effective amount" with respect to a drug or pharmacologically active agent refers to a nontoxic but sufficient amount of the drug or agent to achieve desired effects. For oral dosage forms in the present invention, an "effective amount" of one active substance in a composition refers to the amount required to achieve the desired effect when the active substance is used in combination with another active substance in the composition. The determination of the effective amount varies from person to person, which depends on the age and general condition of a recipient and also depends on the specific active substance. The appropriate effective amount in individual cases may be determined by those skilled in the art based on routine experiments.

The term "active ingredient", "therapeutic agent", "active substance" or "active agent" refers to a chemical entity that is effective in treating target disorder, diseases or conditions.

The term "optional" or "optionally" means that the subsequently described event or condition may, but are not necessary to occur, and the description includes situations that the event or condition occurs and situations that the event or condition does not occur.

" 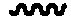 " indicates a linker bond.

The compound of the present invention may be prepared by various synthetic methods well known to those skilled in the art, including specific implementations enumerated below, implementations formed in combination with other chemical synthesis methods, and equivalent replacement methods well known to those skilled in the art, and preferred implementations include, but are not limited to, the embodiments of the present invention.

### Brief description of drawings

FIG.1 is a schematic representation of the crystal structure of a single molecule of Compound 1A.
FIG.2 is the crystal parameters of the crystal structure of Compound 1A.
FIG.3 is the atomic coordinates and isotropic displacement parameters of the crystal structure of Compound 1A.
FIG.4 is thebond lengths in the crystal structure of Compound 1A.
FIG.5 is the bond angles in the crystal structure of Compound 1A.
FIG.6 is the dihedral angles in the crystal structure of Compound 1A.

### Detailed Description of the Embodiments

This application is further described in detail below with reference to embodiments, but implementations of this application are not limited thereto.

### Embodiment 1

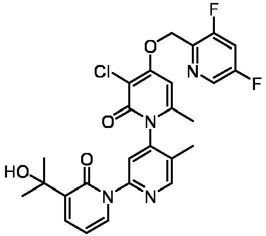

A synthetic route of a compound with a serial number being 1 is shown as follows.

At step A, 2-(bromomethyl)-3,5-difluoropyridine is synthesized.

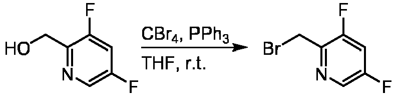

At zero degrees Celsius, triphenylphosphine (PPh₃, 813.5 mg, 3.11 mmol) and carbontetrabromide (CBr₄, 823.8 mg, 2.48 mmol) are successively added to tetrahydrofuran (THF, 5.0 ml) containing (3,5-difluoropyridin-2-yl)methanol (300.0 mg, 2.07 mmol) for reaction for 1h at room temperature (r.t.).

After reaction is completed, vacuum concentration is directly performed. Obtained residues are purified by silica gel column chromatography (eluent: n-hexane/ethyl acetate = 10/1). 372.4 mg of colorless oil 2-(bromomethyl)-3,5-difluoropyridine is obtained (yield: 86.5%). LC-MS: RT = 1.89 min, [M+H]⁺ = 208.01.

At step B, 3"-chloro-4"-((3,5-difluoropyridine-2-yl)methoxy)-3-(2-hydroxypropane-2-yl)-5',6"-dimethyl-2H,2"H-[1,2':4',1"-terpyridine]-2,2"-diketone is synthesized.

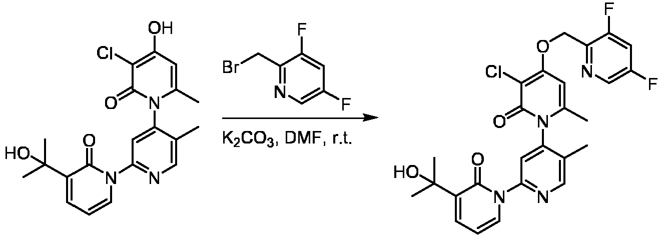

At room temperature, 2-(bromomethyl)-3,5-difluoropyridine (37.4 mg, 0.18 mmol) and potassium carbonate (K₂CO₃, 33.12 mg, 0.24 mmol) are added to N,N-dimethylformamide (DMF, 2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 28.0 mg of a white solid 3"-chloro-4"-((3,5-difluoropyridine-2-yl)methoxy)-3-(2-hydroxypropane-2-yl)-5',6"-dimethyl-2H,2"H-[1,2':4',1"-terpyridine]-2,2"-diketone is obtained (yield: 53.7%).

LC-MS: RT = 1.86 min, [M+H]⁺ = 529.22. ¹H NMR (400 MHz, DMSO) δ 8.68 (s, 1H), 8.60 (d, J = 2.3 Hz, 1H), 8.13-8.05 (m, 1H), 7.85 (dd, J = 6.9, 2.1 Hz, 1H), 7.78 (s, 1H), 7.69 (dd, J = 7.0, 2.1 Hz, 1H), 6.79 (s, 1H), 6.42 (t, J = 7.0 Hz, 1H), 5.47 (d, J = 1.6 Hz, 2H), 5.22 (s, 1H), 2.07 (s, 3H), 2.00 (s, 3H), 1.47 (s, 3H), 1.46 (s, 3H).

Supercritical fluid chromatography (AS-H column) is used to separate a racemic Embodiment 1 compound by means of a mobile phase of carbon dioxide and isopropanol, and atropisomer Embodiment 1A and 1B compounds are eluted successively.

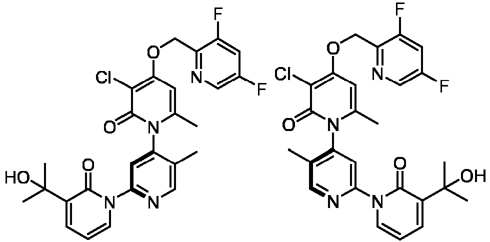

### (-)-Embodiment 1A(+)-Embodiment 1B

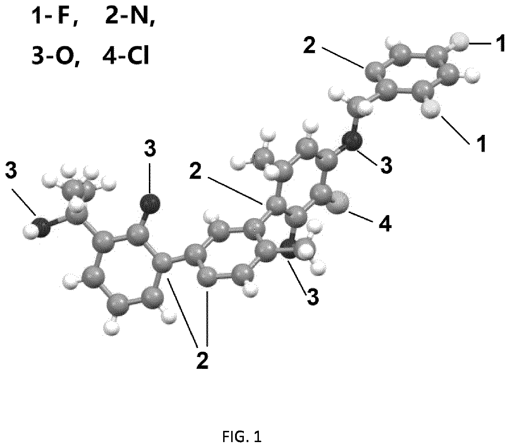

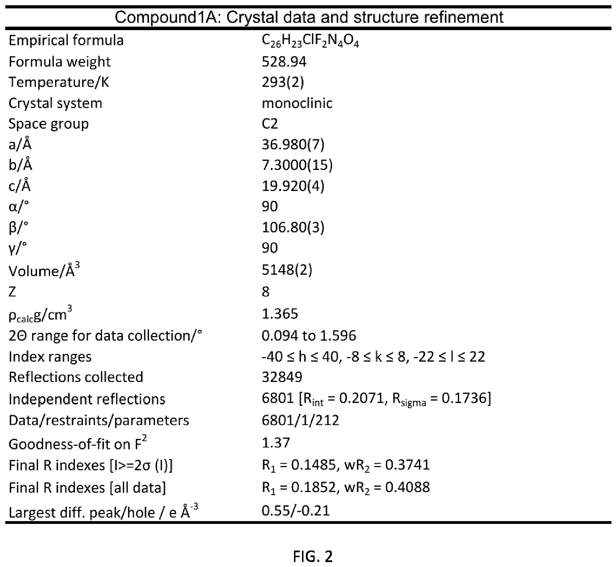

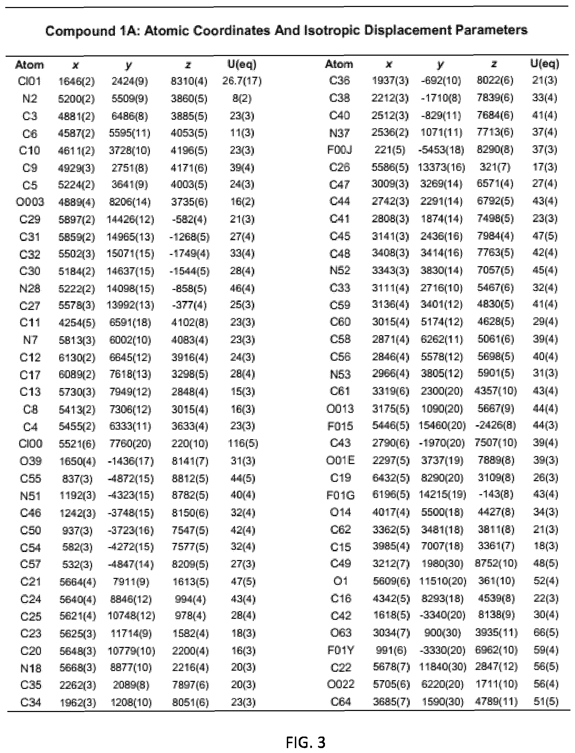

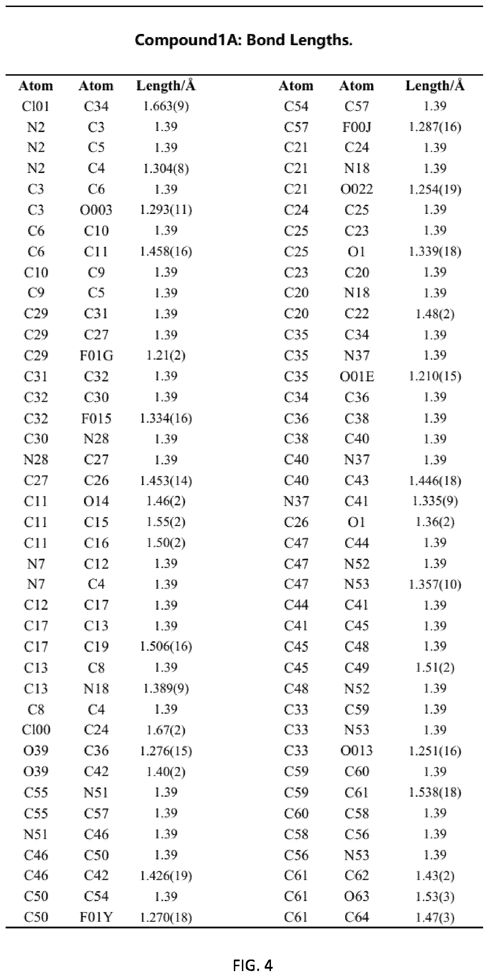

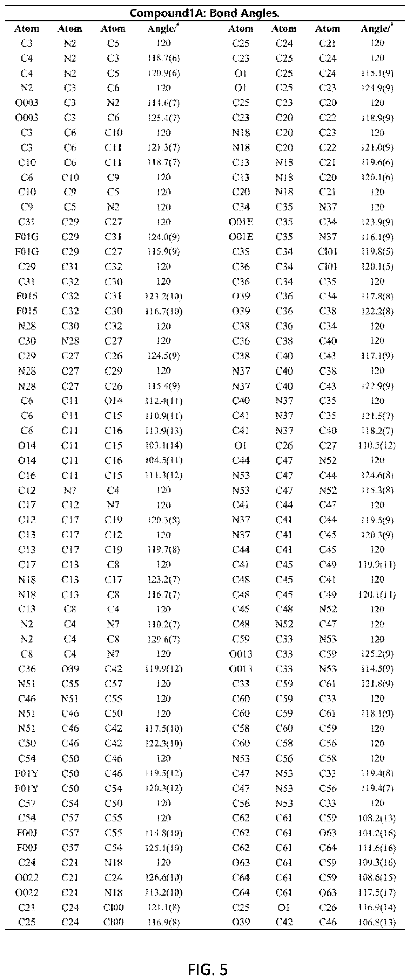

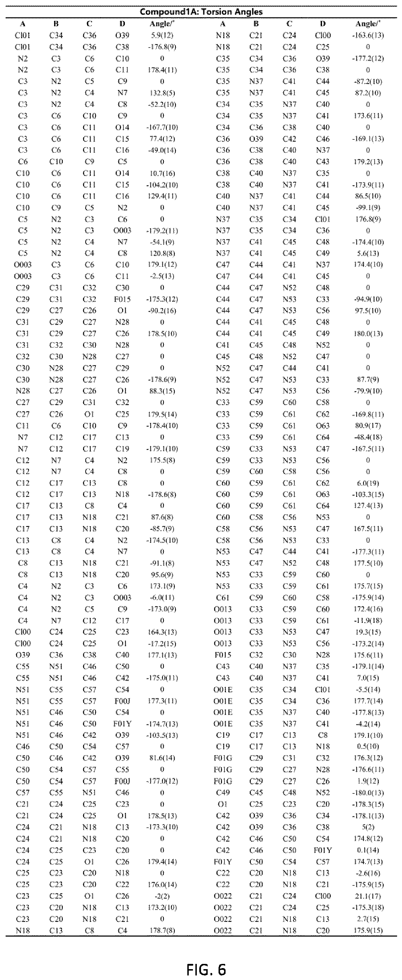

Embodiment 1A compound: RT = 4.21 min (SFC, AS-H, 0.46 cm I.D. × 15 cm L column, isogradient method with 30% isopropanol, flow rate being 2.5 mL/min, and cycling time being 10 min). [a]_{D}²⁵=-1.68° (MeOH, Rudolph Autopol I specific rotation instrument); and ¹H NMR (400 MHz, DMSO)δ 8.68 (s, 1H), 8.59 (d, J = 2.3 Hz, 1H), 8.11-8.05 (m, 1H), 7.85 (dd, J = 6.9, 2.0 Hz, 1H), 7.79 (s, 1H), 7.69 (dd, J = 7.0, 2.0 Hz, 1H), 6.80 (s, 1H), 6.42 (t, J = 6.9 Hz, 1H), 5.48 (d, J = 1.2 Hz, 2H), 5.23 (s, 1H), 2.07 (s, 3H), 2.00 (s, 3H), 1.47 (s, 3H), 1.46 (s, 3H).The crystal structure of a single molecule is shown in FIG.1. The main crystal parameters are shown in FIG.2. The atomic coordinates and isotropic displacement parameters of the crystal structure are shown in FIG.3. Thebond lengths in the crystal structure are shown in FIG.4. The bond angles in the crystal structure are shown in FIG.5. The dihedral angles in the crystal structure are shown in FIG.6.

Embodiment 1B compound: RT = 4.68 min (SFC, AS-H, 0.46 cm I.D. × 15 cm L column, isogradient method with 30% isopropanol, flow rate being 2.5 mL/min, and cycling time being 10 min). [a] _{D}²⁵+1.64° (MeOH, Rudolph Autopol I specific rotation instrument); and ¹H NMR (500 MHz, DMSO) δ 8.68 (s, 1H), 8.59 (d, J= 2.3 Hz, 1H), 8.11-8.06 (m, 1H), 7.85 (dd, J = 6.9, 2.1 Hz, 1H), 7.78 (s, 1H), 7.69 (dd, J = 7.0, 2.1 Hz, 1H), 6.80 (s, 1H), 6.42 (t, J = 6.9 Hz, 1H), 5.47 (d, J = 1.3 Hz, 2H), 5.24 (s, 1H), 2.07 (s, 3H), 2.00 (s, 3H), 1.47 (s, 3H), 1.46 (s, 3H).

### Embodiment 2

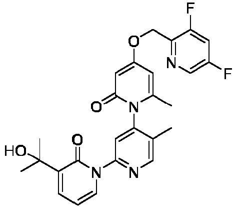

A synthetic route of a compound with a serial number being 2 is shown as follows.

At step A, 2'-chloro-4-hydroxyl-5',6-dimethyl-2H-[1,4'-bipyridyl]-2-one is synthesized.

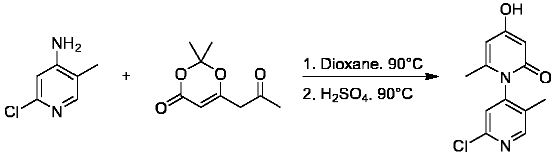

At room temperature, 2-chloro-5-methylpyridin-4-amine (2.3 g, 1.61 mmol) is dissolved into dioxane (40.0 ml), 2,2-dimethyl-6-(2-oxopropyl)-4H-1,3-dioxin-4-one (2.0 g, 1.07 mmol) is added, a temperature is warmed up to 90 degrees Celsius for reaction for 5h, 10N of a sulfuric acid aqueous solution is added dropwise, no solid is produced, and the reaction is continued for 2h.

After the reaction is complete, a solvent is spun out; a yellowish precipitate is precipitated by adding an appropriate amount of water; filtering is performed after 0.5h of stirring, and washing is performed with water for two times; and a product of 1.25 g of an earth-yellow solid 2'-chloro-4-hydroxyl-5',6-dimethyl-2H-[1,4'-bipyridyl]-2-one is collected (yield: 30.9%). LC-MS: RT = 1.65 min, [M+H]+ = 251.09.

At step B, 2'-chloro-4-((3,5-difluoropyridine-2-yl)methoxy)-5',6-dimethyl-2H-[1,4'-bipyridyl]-2-one is synthesized.

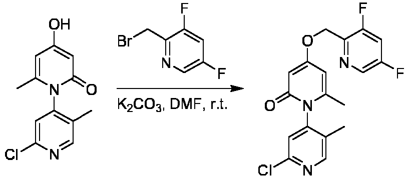

At room temperature, 2-(bromomethyl)-3,5-difluoropyridine (1.55 g, 7.47 mmol) and K₂CO₃ (1.37 g, 9.96 mmol) are added to N,N-DMF (15.0 ml) containing 2'-chloro-4-hydroxyl-5',6-dimethyl-2H-[1,4'-bipyridyl]-2-one (1.25 g, 4.98 mmol) for reaction for 2h at room temperature.

After the reaction is completed, the reaction is quenched with water; extraction is performed with ethyl acetate (30 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 1.08 g of a yellowish solid 2'-chloro-4-((3,5-difluoropyridine-2-yl)methoxy)-5',6-dimethyl-2H-[1,4'-bipyridyl]-2-one is obtained (yield: 57.4%). LC-MS: RT = 1.91 min, [M+H]+ = 378.24.

At step C, 4"-((3,5-difluoropyridine-2-yl)methoxy)-3-(2-hydroxypropane-2-yl)-5',6"-dimethyl-2H,2"H-[1,2':4',1"-terpyridine]-2,2"-diketone is synthesized.

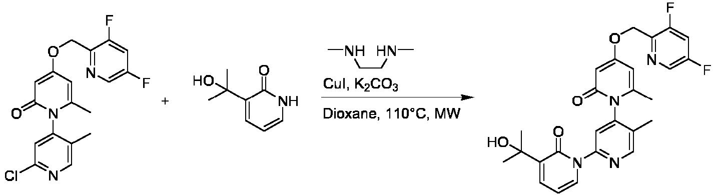

At room temperature, the 2'-chloro-4-((3,5-difluoropyridine-2-yl)methoxy)-5',6-dimethyl-2H-[1,4'-bipyridyl]-2-one (100.0 mg, 0.26 mmol), 3-(2-hydroxypropane-2-yl)pyridine-2(1H)-one (60.1 mg, 0.39 mmol), Cuprous Iodide (Cul, 9.5 mg, 0.05 mmol), and K₂CO₃ (71.8 mg, 0.52 mmol) are dissolved in the dioxane (2.0 ml), a drop of N,N-dimethylethylenediamine is added, and then the temperature is warmed up to 110 degrees Celsius for microwave reaction for 1h.

After the reaction is completed, suction filtration is performed, drip washing is performed with dichloromethane, and then filtrate is concentrated. Obtained residues are purified by silica gel column chromatography (eluent: ethyl acetate/n-hexane = 1/0). 26.0 mg of a white solid 4"-((3,5-difluoropyridine-2-yl)methoxy)-3-(2-hydroxypropane-2-yl)-5',6"-dimethyl-2H,2"H-[1,2':4',1"-terpyridine]-2,2"-diketone is obtained (yield: 20.2%).

LC-MS: RT = 1.82 min, [M+H]⁺ = 495.24. ¹H NMR (400 MHz, DMSO) δ 8.64 (s, 1H), 8.59 (d, J = 2.4 Hz, 1H), 8.07 (ddd, J = 10.0, 9.3, 2.4 Hz, 1H), 7.84 (dd, J = 6.9, 2.1 Hz, 1H), 7.70 (s, 1H), 7.70-7.66 (m, 1H), 6.41 (t, J = 6.9 Hz, 1H), 6.13 (d, J = 1.7 Hz, 1H), 6.04 (d, J = 2.5 Hz, 1H), 5.25 (s, 2H), 5.22 (s, 1H), 2.08 (s, 3H), 1.90 (s, 3H), 1.47 (s, 3H), 1.46 (s, 3H).

### Embodiment 3

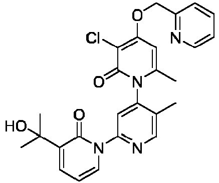

A synthetic route of a compound with a serial number being 3 is shown as follows.

At step A, 2-(bromomethyl)pyridine is synthesized.

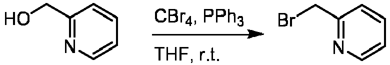

At zero degrees Celsius, PPh3 (813.5 mg, 3.11 mmol) and CBr4 (823.8 mg, 2.48 mmol) are added to THF (5.0 ml) containing the 2-(bromomethyl)pyridine (225.6 mg, 2.07 mmol) for reaction for 1h at room temperature.

After the reaction is completed, concentration is performed. Obtained residues are purified by silica gel column chromatography (eluent: n-hexane/ethyl acetate = 10/1). 322.3 mg of colorless oil 2-(bromomethyl)pyridine is obtained (yield: 90.5%).

At step B, 3"-chloro-3-(2-hydroxypropane-2-yl)-5',6"-dimethyl-4"-((pyridin-2-yl)methoxy-2H,2"H-[1,2':4',1"-terpyridine]-2,2"-diketone is synthesized.

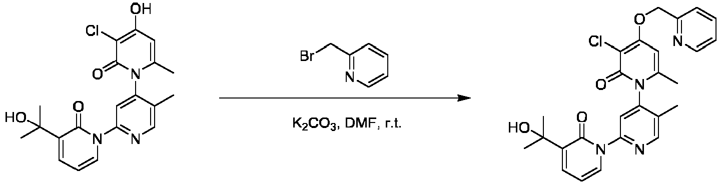

At room temperature, 2-(bromomethyl)pyridine (31.0 mg, 0.18 mmol) and K₂CO₃ (33.12 mg, 0.24 mmol) are added to N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 29.0 mg of a white solid 3"-chloro-3-(2-hydroxypropane-2-yl)-5',6"-dimethyl-4"-((pyridin-2-yl)methoxy -2H,2"H-[1 ,2':4',1"-terpyridine]-2,2"-diketone is obtained (yield: 49.0%).

LC-MS: RT = 1.77 min, [M+H]⁺ = 493.34. Nuclear magnetic resonance data: ¹H NMR (400 MHz, DMSO-d₆) δ 8.67 (s, 1H), 8.61-8.58 (m, 1H), 7.91 (td, J = 7.7, 1.8 Hz, 1H), 7.84 (dt, J = 5.5, 2.8 Hz, 1H), 7.77 (s, 1H), 7.68 (dd, J = 7.0, 2.1 Hz, 1H), 7.58 (d, J = 7.8 Hz, 1H), 7.39 (dd, J = 7.0, 5.3 Hz, 1H), 6.76 (s, 1H), 6.41 (t, J = 6.9 Hz, 1H), 5.42 (s, 2H), 5.21 (s, 1H), 2.06 (s, 3H), 1.99 (s, 3H), 1.45 (d, J = 4.1 Hz, 6H).

### Embodiment 4

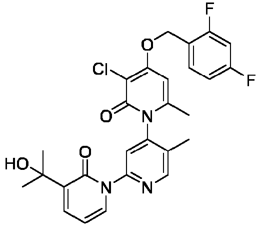

A synthetic route of a compound with a serial number being 4 is shown as follows.

At step A, 2,2-dimethyl-6-(2-oxopropyl)-4H-1,3-dioxin-4-one is synthesized.

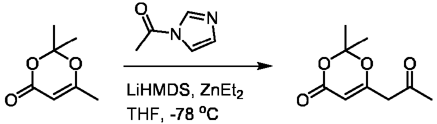

At room temperature, 2,2,6-trimethyl-4H-1,3-dioxin-4-one (30 g, 211.27 mmol) is dissolved into THF (100 ml), and the temperature is dropped to minus 70 degrees Celsius under nitrogen protection; lithium bis(trimethylsilyl)amide (LiHMDS, 290 ml, 1M) is added dropwise for reaction for 2h; diethylzinc (ZnEt₂, 290 ml, 1M) is added for reaction for 0.5h; and the temperature is slowly warmed up to minus 20 degrees Celsius within 1.5h, and 1-acetylimidazole (28 g, 253.52 mmol) is added for reaction for 3h.

After the reaction is completed, the reaction is quenched by being poured into ice water; pH=3-4 is adjusted with 6N of hydrochloric acid; extraction is performed with the ethyl acetate (50 ml × 3 times), and an organic phase is combined to perform drying; purification is performed through column chromatography (eluent: ethyl acetate/n-hexane = 4/1) to obtain 19 g of a yellow solid 2,2-dimethyl-6-(2-oxopropyl)-4H-1,3-dioxin-4-one (yield: 49%). LC-MS: RT = 1.65 min, [M+H]⁺ = 185.13.

At step B, 2'-bromo-4-hydroxyl-5',6-dimethyl-2H-[1,4'-bipyridyl]-2-one is synthesized.

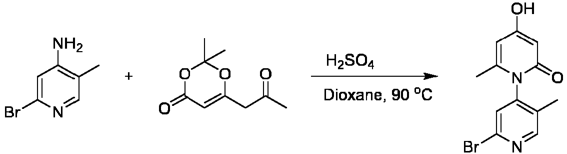

At room temperature, 2-bromo-5-methylpyridin-4-amine (5 g, 26.74 mmol) is dissolved into the dioxane (100 ml), 2,2-dimethyl-6-(2-oxopropyl)-4H-1,3-dioxin-4-one (5.9 g, 32.09 mmol) is added, the temperature is warmed up to 90 degrees Celsius for reaction for 5h, 10N of the sulfuric acid aqueous solution is added dropwise, until no solid is produced, and the reaction is continued for 2h.

After the reaction is complete, concentration is performed to remove the solvent; a yellowish precipitate is precipitated by adding water; filtering is performed after 0.5h of stirring, and washing is performed with water for two times; and a product of 6 g of an earth-yellow solid 2'-bromo-4-hydroxyl-5',6-dimethyl-2H-[1,4'-bipyridyl]-2-one is collected (yield: 76%). LC-MS: RT = 1.66 min, [M+H]⁺ = 295.11.

At step C, 2'-bromo-3-chloro-4-hydroxyl-5',6-dimethyl-2H-[1,4'-bipyridyl]-2-one is synthesized.

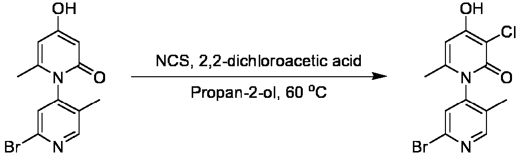

At room temperature, the 2'-bromo-4-hydroxyl-5',6-dimethyl-2H-[1,4'-bipyridyl]-2-one (200 mg, 0.68 mmol) is dissolved into isopropanol (Propan-2-ol, 5 ml); N-chlorosuccinimide (NCS, 62 mg, 0.37 mmol) and a drop of 2,2-dichloroacetic acid are added; and the temperature is warmed up to 60 degrees Celsius for reaction for 6h.

After the reaction is completed, the reaction is quenched with water; extraction is performed with ethyl acetate; an organic phase is combined; spin drying is performed; and purification (ethyl acetate) is performed through column chromatography to obtain 100 mg of a white solid 2'-bromo-3-chloro-4-hydroxyl-5',6-dimethyl-2H-[1,4'-bipyridyl]-2-one (yield: 83%). LC-MS: RT = 1.68 min, [M+H]⁺ = 329.06.

At step D, 3-(2-hydroxypropane-2-yl)pyridine-2(1H)-one is synthesized.

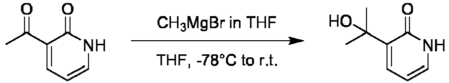

At minus 78 degrees Celsius, a THF solution (5.47 ml, 1 mol/ml) of methylmagnesium bromide (CH₃MgBr) is added dropwise into the THF (5.0 ml) containing 3-acetyl-2(1H)-pyridone (500.0 mg, 3.65 mmol); and the temperature is slowly warmed up to room temperature for reaction for 3h.

After the reaction is completed, the reaction is quenched with water; extraction is performed with a mixed solution of dichloromethane and isopropanol (dichloromethane/isopropanol=3/1, 30 ml × 5 times); an organic phase is combined; washing is performed with saturated salt water (30 ml × 2 times); and drying is performed with anhydrous sodium sulfate, and then concentration is performed. Obtained residues are purified by silica gel column chromatography (eluent: dichloromethane/methanol = 10/1). 496.0 mg of a white solid 3-(2-hydroxypropane-2-yl)pyridine-2(1H)-one is obtained (yield: 19.0%). LC-MS: RT = 1.33 min, [M+H]⁺ = 154.14.

At step E, 3"-chloro-4"-hydroxyl-3-(2-hydroxypropane-2-yl)-5',6"-dimethyl-2H,2"H-[1,2':4',1"-terpyridine]-2,2"-diketone is synthesized.

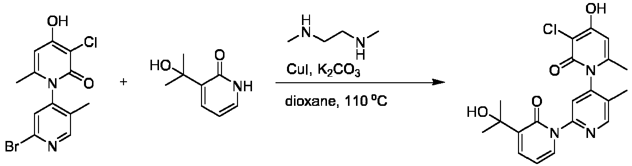

At room temperature, the 2'-bromo-3-chloro-4-hydroxyl-5',6-dimethyl-2H-[1,4'-bipyridyl]-2-one (100 mg, 0.30 mmol), 3-(2-hydroxypropane-2-yl)pyridine-2(1H)-one (93 mg, 0.61 mmol), Cul (12 mg, 0.06 mmol), and K₂CO₃ (84 mg, 0.61 mmol) are dissolved in the dioxane (4 ml), a drop of dimethylethylenediamine is added, and then the temperature is warmed up to 100 degrees Celsius for microwave reaction for 1h.

After the reaction is completed, filter is performed; washing is performed with the ethyl acetate; and spin drying is performed on filtrate, and the mixed sample is purified (ethyl acetate) by columns, to obtain 67 mg of a white solid 3"-chloro-4"-hydroxyl-3-(2-hydroxypropane-2-yl)-5',6"-dimethyl-2H,2"H-[1,2':4',1"-terpyridine]-2,2"-diketone (yield: 55%). LC-MS: RT = 1.70 min, [M+H]⁺ = 402.20.

At step F, 3"-chloro-4"-((2,4-difluorobenzyl)oxy)-3-(2-hydroxypropane-2-yl)-5',6"-dimethyl-2H,2"H-[1,2':4',1"-terpyridine]-2,2"-diketone is synthesized.

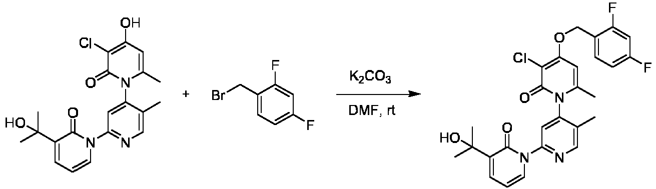

At room temperature, 2-fluoro-4-chlorobenzyl bromide (36 mg, 0.17 mmol) and K₂CO₃ (24 mg, 0.17 mmol) are added to the N,N-DMF (2.0 ml) containing the 3"-chloro-4"-hydroxyl-3-(2-hydroxypropane-2-yl)-5',6"-dimethyl-2H,2"H-[1,2':4',1"-terpyridine]-2,2"-diketone (35 mg, 0.09 mmol) for reaction for 1h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 11.0 mg of a white solid 3"-chloro-4"-((2,4-difluorobenzyl)oxy)-3-(2-hydroxypropane-2-yl)-5',6"-dimethyl-2H,2"H-[1,2':4',1"-terpyridine]-2,2"-diketone is obtained (yield: 24%). LC-MS: RT = 1.95 min, [M+H]⁺ = 528.13.

### Embodiment 5

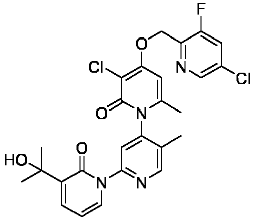

A synthetic route of a compound with a serial number being 5 is shown as follows.

At step A, 2-(bromomethyl)-3-fluoro-5-chloropyridine is synthesized.

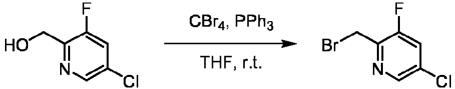

At zero degrees Celsius, PPh₃ (325 mg, 1.24 mmol) and CBr₄ (406 mg, 1.24 mmol) are added to THF (5.0 ml) containing (3-fluoro-5-chloropyridine-2-pyridine)methanol (200 mg, 1.24 mmol) for reaction for 1h at room temperature.

After the reaction is completed, concentration is performed. Obtained residues are purified by silica gel column chromatography (eluent: n-hexane/ethyl acetate = 10/1). 142 mg of colorless oil 2-(bromomethyl)-3-fluoro-5-chloropyridine is obtained (yield: 51%).

At step B, 3"-chloro-4"-((3-fluoro-5-chloropyridine-2-yl)methoxy)-3-(2-hydroxypropane-2-yl)-5',6"-dimethyl-2H,2"H-[1,2':4',1"-terpyridine]-2,2"-diketone is synthesized.

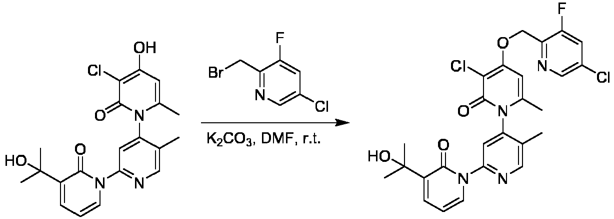

At room temperature, 2-(bromomethyl)-3-fluoro-5-chloropyridine (38 mg, 0.17 mmol) and K₂CO₃ (24 mg, 0.17 mmol) are added to the N,N-DMF (2.0 ml) containing the 3"-chloro-4"-hydroxyl-3-(2-hydroxypropane-2-yl)-5',6"-dimethyl-2H,2"H-[1,2':4',1"-terpyridine]-2,2"-diketone (35 mg, 0.09 mmol) for reaction for 1h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 14 mg of a white solid 3"-chloro-4"-((3-fluoro-5-chloropyridine-2-yl)methoxy)-3-(2-hydroxypropane-2-yl)-5',6"-dimethyl-2H,2"H-[1,2':4',1"-terpyridine]-2,2"-diketone is obtained (yield: 53.7%).

LC-MS: RT = 1.91 min, [M+H]⁺ = 545.17.

### Embodiment 6

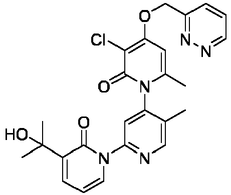

A synthetic route of a compound with a serial number being 6 is shown as follows.

At step A, 2-(bromomethyl)pyridazine is synthesized.

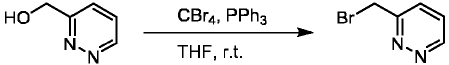

At zero degrees Celsius, PPh₃ (813.5 mg, 3.11 mmol) and CBr₄ (823.8 mg, 2.48 mmol) are added to THF (5.0 ml) containing the 2-pyridazinemethanol (227.7 mg, 2.07 mmol) for reaction for 1h at room temperature.

After the reaction is completed, concentration is performed. Obtained residues are purified by silica gel column chromatography (eluent: n-hexane/ethyl acetate = 10/1). 328.5 mg of colorless oil 2-(bromomethyl)pyridazine is obtained (yield: 91.7%).

At step B, 3"-chloro-3-(2-hydroxypropane-2-yl)-5',6"-dimethyl-4"-((pyridazin-3-yl)methoxy-2H,2"H-[1,2':4',1"-terpyridine]-2,2"-diketone is synthesized.

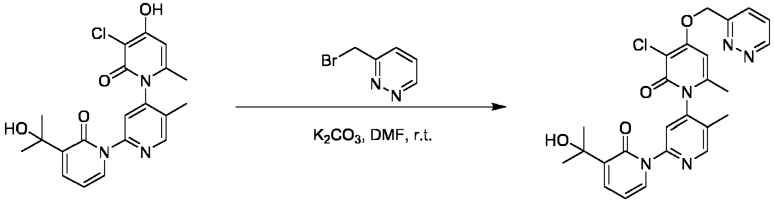

At room temperature, 2-(bromomethyl)pyridazine (31.1 mg, 0.18 mmol) and K₂CO₃ (33.1 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 30.5 mg of a white solid 3"-chloro-3-(2-hydroxypropane-2-yl)-5',6"-dimethyl-4"-((pyridazin-3-yl)methoxy-2H,2"H-[1,2':4',1"-terpyridine]-2,2"-diketone is obtained (yield: 51.4%).

LC-MS: RT = 1.68 min, [M+H]⁺ = 494.26. Nuclear magnetic resonance data: ¹H NMR (400 MHz, DMSO-d₆) δ 9.26 (dd, J = 4.7, 1.8 Hz, 1H), 8.68 (s, 1H), 7.88-7.82 (m, 3H), 7.77 (s, 1H), 7.68 (dd, J = 7.0, 2.0 Hz, 1H), 6.82 (s, 1H), 6.41 (t, J = 6.9 Hz, 1H), 5.65 (s, 2H), 5.21 (s, 1H), 2.07 (s, 3H), 2.00 (s, 3H), 1.45 (d, J = 4.0 Hz, 6H).

### Embodiment 7

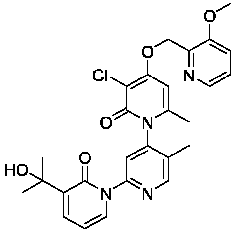

A synthetic route of a compound with a serial number being 7 is shown as follows.

At step A, 3"-chloro-3-(2-hydroxypropane-2-yl)-4"-((3-methoxypyridine-2-yl)methoxy)-5',6"-dimethyl-2H,2"H-[1,2':4',1"-terpyridine]-2,2"-diketone is synthesized.

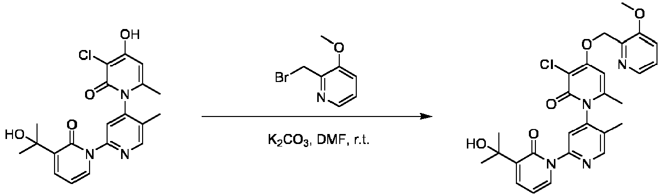

At room temperature, 2-(bromomethyl)-3-methoxypyridine (36.4 mg, 0.18 mmol) and K₂CO₃ (33.1 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 24 mg of a white solid 3"-chloro-4"-(pyridin-4-ylmethoxy)-3-(2-hydroxypropane-2-yl)-5',6"-dimethyl-2H,2"H-[1,2':4',1"-terpyridine]-2,2"-diketone is obtained (yield: 38.3%).

LC-MS: RT = 1.79min, [M+H]⁺ =523.28. Nuclear magnetic resonance data: ¹H NMR (400 MHz, DMSO-d₆) δ 8.67 (s, 1H), 8.20-8.16 (m, 1H), 7.84 (dd, J = 6.9, 2.0 Hz, 1H), 7.77 (s, 1H), 7.68 (dd, J = 7.0, 2.1 Hz, 1H), 7.56 (d, J = 8.4 Hz, 1H), 7.44 (dd, J = 8.4, 4.6 Hz, 1H), 6.76 (s, 1H), 6.41 (t, J = 6.9 Hz, 1H), 5.36 (s, 2H), 5.21 (s, 1H), 3.88 (s, 3H), 2.06 (s, 3H), 1.98 (s, 3H), 1.45 (d, J = 4.2 Hz, 6H).

### Embodiment 8

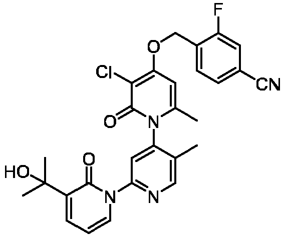

A synthetic route of a compound with a serial number being 8 is shown as follows.

At step A, 4-(((3"-chloro-3-(2-hydroxypropane-2-yl)-5',6"-dimethyl-2,2"-dioxo-2H,2"H-[1,2 2':4',1"-terpyridine]-4"-yl)oxy)methyl)-3-fluorobenzonitrile is synthesized.

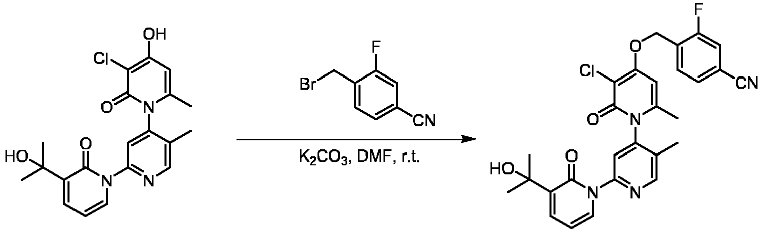

At room temperature, 4-(bromomethyl)-3-fluorobenzonitrile (38.16 mg, 0.18 mmol) and K₂CO₃ (33.12 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 26.5 mg of a white solid 4-(((3"-chloro-3-(2-hydroxypropane-2-yl)-5',6"-dimethyl-2,2"-dioxo-2H,2"H-[1,2 2':4',1"-terpyridine]-4"-yl)oxy)methyl)-3-fluorobenzonitrile is obtained (yield: 41.3%). LC-MS: RT = 1.79min, [M+H]⁺ = 535.23. Nuclear magnetic resonance data: ¹H NMR (400 MHz, DMSO-d₆) δ 8.68 (s, 1H), 7.97 (d, J = 9.6 Hz, 1H), 7.83 (dt, J = 10.8, 3.4 Hz, 3H), 7.77 (s, 1H), 7.68 (dd, J = 7.0, 2.0 Hz, 1H), 6.80 (s, 1H), 6.41 (t, J = 6.9 Hz, 1H), 5.49 (s, 2H), 5.21 (s, 1H), 2.07 (d, J = 8.7 Hz, 3H), 2.00 (s, 3H), 1.45 (d, J = 3.9 Hz, 6H).

### Embodiment 9

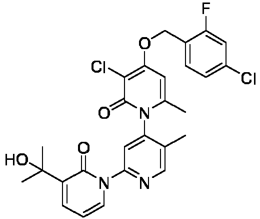

A synthetic route of a compound with a serial number being 9 is shown as follows.

At step A, 3"-chloro-4"-((4-chloro-2-fluorobenzyl)oxy)-3-(2-hydroxypropane-2-yl)-5',6"-dimethyl-2H,2"H-[1,2':4',1"-terpyridine]-2,2"-diketone is synthesized.

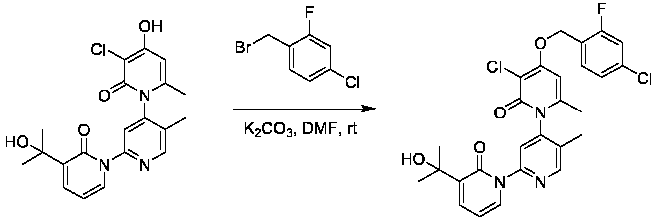

At room temperature, 2-fluoro-4-chlorobenzyl bromide (122 mg, 0.50 mmol) and K₂CO₃ (69 mg, 0.50 mmol) are added to the N,N-DMF (2.5 ml) containing 3"-chloro-4"-hydroxyl-3-(2-hydroxypropane-2-yl)-5',6"-dimethyl-2H,2"H-[1,2':4',1"-terpyridine]-2,2"-diketone (100 mg, 0.25 mmol) for reaction for 1h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 50 mg of a white solid 3"-chloro-4"-((4-chloro-2-fluorobenzyl)oxy)-3-(2-hydroxypropane-2-yl)-5',6"-dimethyl-2H,2"H-[1,2':4',1"-terpyridine]-2,2"-diketone is obtained (yield: 36%).

LC-MS: RT = 2.02 min, [M+H]⁺ = 544.18. ¹H NMR (400 MHz, DMSO-d₆) δ 8.67 (s, 1H), 7.84 (dd, J = 6.9, 2.1 Hz, 1H), 7.77 (s, 1H), 7.72-7.61 (m, 2H), 7.55 (dd, J = 10.0, 2.0 Hz, 1H), 7.40 (dd, J = 8.3, 1.8 Hz, 1H), 6.79 (s, 1H), 6.41 (t, J = 7.0 Hz, 1H), 5.37 (s, 2H), 5.21 (s, 1H), 2.06 (s, 3H), 2.00 (s, 3H), 1.45 (d, J = 3.9 Hz, 6H).

### Embodiment 10

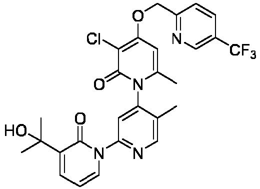

A synthetic route of a compound with a serial number being 10 is shown as follows.

At step A, 3"-chloro-4"-((5-(trifluoromethyl)pyridin-2-yl)methoxy)-3-(2-hydroxypropane-2-yl)-5',6"-dimethyl-2H,2"H-[1,2':4',1"-terpyridine]-2,2"-diketone is synthesized.

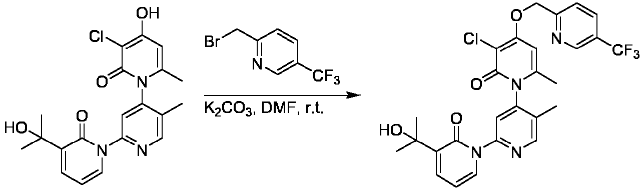

At room temperature, 2-(bromomethyl)-5-(trifluoromethyl)pyridine (43.2 mg, 0.18 mmol) and K₂CO₃ (33.12 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1 "-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 11.0 mg of a white solid 3"-chloro-4"-((5-(trifluoromethyl)pyridin-2-yl)methoxy)-3-(2-hydroxypropane-2-yl)-5',6"-dimethyl-2H,2"H-[1,2':4',1"-terpyridine]-2,2"-diketone is obtained (yield: 16.3%).

LC-MS: RT = 1.93 min, [M+H]⁺ = 561.23.

### Embodiment 11

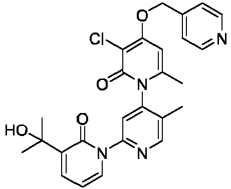

A synthetic route of a compound with a serial number being 11 is shown as follows.

At step A, 3"-chloro-4"-((pyridin-4-yl)methoxy)-3-(2-hydroxypropane-2-yl)-5',6"-dimethyl-2H,2"H-[1,2':4',1"-terpyridine]-2,2"-diketone is synthesized.

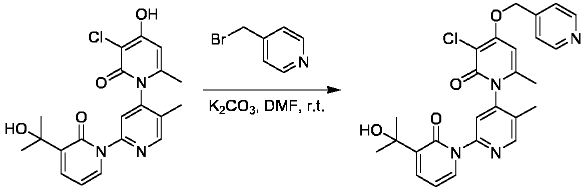

At room temperature, 4-(bromomethyl)pyridine (45.5 mg, 0.18 mmol) and K₂CO₃ (33.12 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 10.0 mg of a white solid 3"-chloro-4"-((pyridin-4-yl)methoxy)-3-(2-hydroxypropane-2-yl)-5',6"-dimethyl-2H,2"H-[1,2':4',1"-terpyridine]-2,2"-diketone is obtained (yield: 16.9%). LC-MS: RT = 1.61 min, [M+H]⁺ = 493.24.

### Embodiment 12

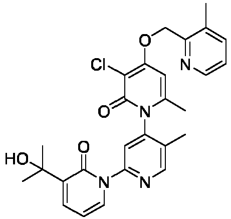

A synthetic route of a compound with a serial number being 12 is shown as follows.

At step A, 3"-chloro-4"-((3-methylpyrid in-2-yl)methoxy)-3-(2-hydroxypropane-2-yl)-5',6"-dimethyl-2H,2"H-[1,2':4',1"-terpyridine]-2,2"-diketone is synthesized.

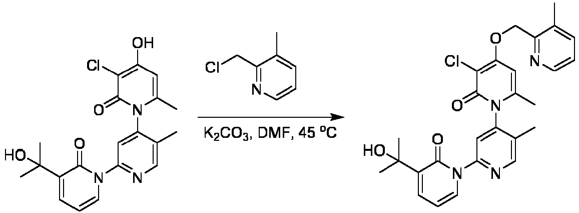

At room temperature, 2-(chloromethyl)-3-methylpyridine (71 mg, 0.50 mmol) and K₂CO₃ (69 mg, 0.50 mmol) are added to the N,N-DMF (2.5 ml) containing 3"-chloro-4"-hydroxyl-3-(2-hydroxypropane-2-yl)-5',6"-dimethyl-2H,2"H-[1,2':4',1"-terpyridine]-2,2"-diketone (100 mg, 0.25 mmol) for reaction for 4h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 21 mg of a white solid 3"-chloro-4"-((3-methylpyridin-2-yl)methoxy)-3-(2-hydroxypropane-2-yl)-5',6"-dimethyl-2H,2"H-[1,2':4',1"-terpyridine]-2,2"-diketone is obtained (yield: 16%).

LC-MS: RT = 1.75 min, [M+H]⁺ = 507.21.

### Embodiment 13

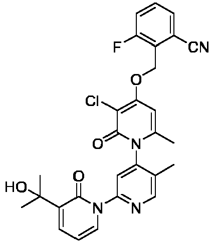

A synthetic route of a compound with a serial number being 13 is shown as follows.

At step A, 2-(((3"-chloro-3-(2-hydroxypropane-2-yl)-5',6"-dimethyl-2,2"-dione-2H,2"H-[1,2':4',1"-terpyridine]-4"-yl)oxy)methyl)-3-fluorobenzonitrile is synthesized.

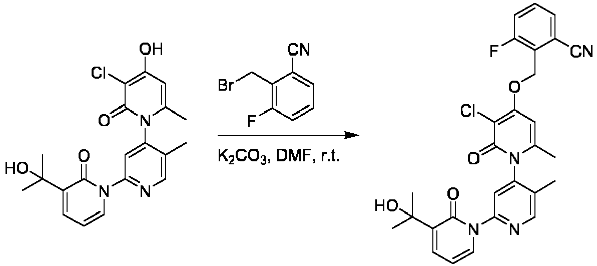

At room temperature, 2-(bromomethyl)-3-fluorobenzonitrile (38.16 mg, 0.18 mmol) and K₂CO₃ (33.12 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1 "-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 29.5 mg of a white solid 2-(((3"-chloro-3-(2-hydroxypropane-2-yl)-5',6"-dimethyl-2,2"-dione-2H,2"H-[1,2':4',1"-terpyridine]-4"-yl)oxy)methyl)-3-fluorobenzonitrile is obtained (yield: 45.9%).

LC-MS: RT = 1.88 min, [M+H]⁺ = 535.21. ¹H NMR (400 MHz, DMSO) δ 8.70 (s, 1H), 7.92-7.84 (m, 2H), 7.82 (s, 1H), 7.80-7.73 (m, 2H), 7.70 (dd, J = 7.0, 2.1 Hz, 1H), 6.88 (s, 1H), 6.43 (t, J = 6.9 Hz, 1H), 5.46 (s, 2H), 5.23 (s, 1H), 2.09 (s, 3H), 2.05 (s, 3H), 1.48 (s, 3H), 1.47 (s, 3H).

### Embodiment 14

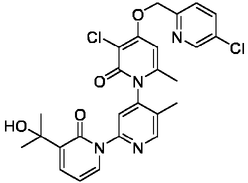

A synthetic route of a compound with a serial number being 14 is shown as follows.

At step A, 3"-chloro-4"-((5-chloropyridine-2-yl)methoxy)-3-(2-hydroxypropane-2-yl)-5',6"-dimethyl-2H,2"H-[1,2':4',1"-terpyridine]-2,2"-diketone is synthesized.

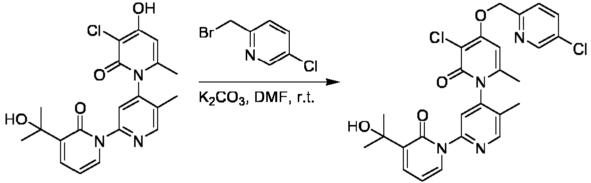

At room temperature, 2-(bromomethyl)-5-chloropyridine (36.9 mg, 0.18 mmol) and K₂CO₃ (33.12 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 14.6 mg of a white solid 3"-chloro-4"-((5-chloropyridine-2-yl)methoxy)-3-(2-hydroxypropane-2-yl)-5',6"-dimethyl-2H,2"H-[1,2':4',1"-terpyridine]-2,2"-diketone is obtained (yield: 23.1%).

LC-MS: RT = 1.89 min, [M+H]⁺ = 527.20.

### Embodiment 15

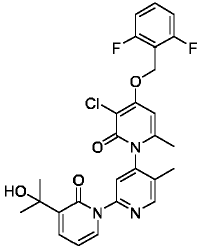

A synthetic route of a compound with a serial number being 15 is shown as follows.

At step A, 3"-chloro-4"-((2,6-difluorophenyl)methoxy)-3-(2-hydroxypropane-2-yl)-5',6"-dimethyl-2H,2"H-[1,2':4',1"-terpyridine]-2,2"-diketone is synthesized.

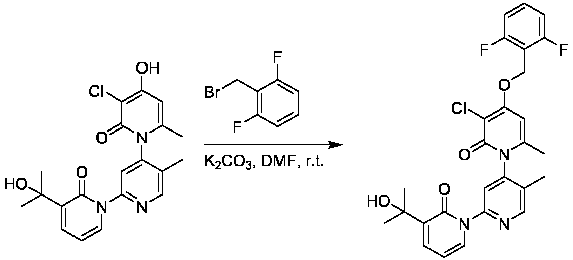

At room temperature, 2-(bromomethyl)-1,3-difluorobenzene (37.26 mg, 0.18 mmol) and K₂CO₃ (33.12 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1 "-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 15.31 mg of a white solid 3"-chloro-4"-((2,6-difluorophenyl)methoxy)-3-(2-hydroxypropane-2-yl)-5',6"-dimethyl-2H,2"H-[1,2':4',1"-terpyridine]-2,2"-diketone is obtained (yield: 24.1%).

LC-MS: RT = 1.93 min, [M+H]⁺ = 528.21.

### Embodiment 16

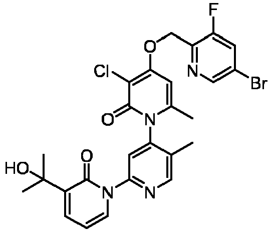

A synthetic route of a compound with a serial number being 16 is shown as follows.

At step A, 2-(bromomethyl)-3-fluoro-5-bromopyridine is synthesized.

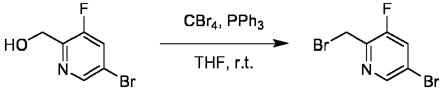

At zero degrees Celsius, PPh₃ (267 mg, 1.02 mmol) and CBr₄ (334 mg, 1.02 mmol) are successively added to THF (10 ml) containing (3-fluoro-5-bromopyridine-2-pyridine)methanol (210 mg, 1.02 mmol) for reaction for 1h at room temperature.

After the reaction is completed, concentration is performed. Obtained residues are purified by silica gel column chromatography (eluent: n-hexane/ethyl acetate = 10/1). 200 mg of colorless oil 2-(bromomethyl)-3-fluoro-5-bromopyridine is obtained (yield: 73%).

At step B, 3"-chloro-4"-((3-fluoro-5-bromopyridine-2-yl)methoxy)-3-(2-hydroxypropane-2-yl)-5',6"-dimethyl-2H,2"H-[1,2':4',1"-terpyridine]-2,2"-diketone is synthesized.

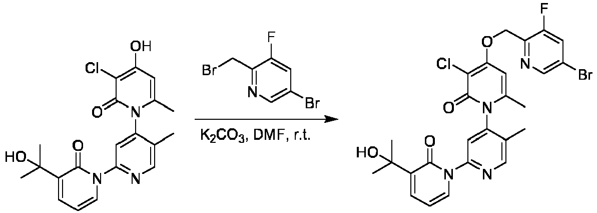

At room temperature, 2-(bromomethyl)-3-bromo-5-fluoropyridine (134 mg, 0.50 mmol) and K₂CO₃ (39 mg, 0.50 mmol) are added to the N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (100 mg, 0.25 mmol) for reaction for 1h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 14 mg of a white solid 3"-chloro-4"-((3-fluoro-5-bromopyridine-2-yl)methoxy)-3-(2-hydroxypropane-2-yl)-5',6"-dimethyl-2H,2"H-[1,2':4',1"-terpyridine]-2,2"-diketone is obtained (yield: 53.7%).

LC-MS: RT = 1.91 min, [M+H]⁺ = 589.13.

### Embodiment 17

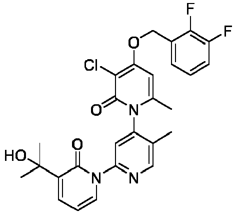

A synthetic route of a compound with a serial number being 17 is shown as follows.

At step A, 3"-chloro-4"-((2,3-difluorobenzyl)oxy)-3-(2-hydroxypropane-2-yl)-5',6"-dimethyl-2H,2"H-[1,2':4',1"-terpyridine]-2,2"-diketone is synthesized.

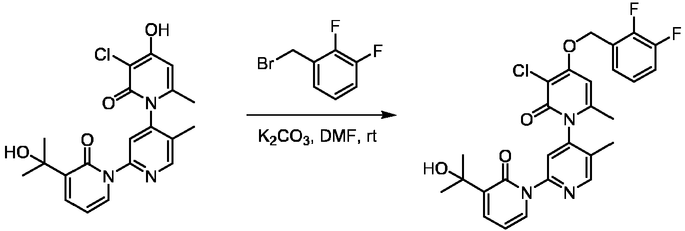

At room temperature, 2,3-difluorobenzyl bromide (31 mg, 0.149 mmol) and K₂CO₃ (35 mg, 0.50 mmol) are added to the N,N-DMF (2.5 ml) containing 3"-chloro-4"-hydroxyl-3-(2-hydroxypropane-2-yl)-5',6"-dimethyl-2H,2"H-[1,2':4',1"-terpyridine]-2,2"-diketone (50 mg, 0.124 mmol) for reaction for 1h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 6.8 mg of a white solid 3"-chloro-4"-((2,3-difluorobenzyl)oxy)-3-(2-hydroxypropane-2-yl)-5',6"-dimethyl-2H,2"H-[1,2':4',1"-terpyridine]-2,2"-diketone is obtained (yield: 10%).

LC-MS: RT = 1.97 min, [M+H]⁺ = 528.18.

### Embodiment 18

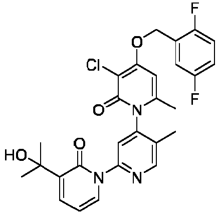

A synthetic route of a compound with a serial number being 18 is shown as follows.

At step A, 3"-chloro-4"-((2,5-difluorobenzyl)oxy)-3-(2-hydroxypropane-2-yl)-5',6"-dimethyl-2H,2"H-[1,2':4',1"-terpyridine]-2,2"-diketone is synthesized.

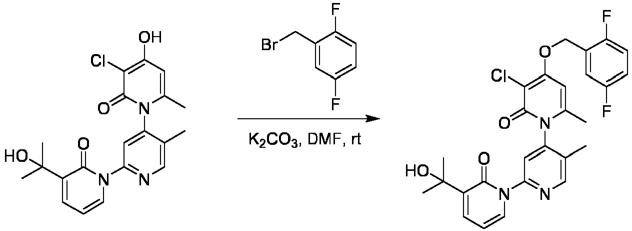

At room temperature, 2,5-difluorobenzyl bromide (31 mg, 0.149 mmol) and K₂CO₃ (35 mg, 0.50 mmol) are added to the N,N-DMF (2.5 ml) containing 3"-chloro-4"-hydroxyl-3-(2-hydroxypropane-2-yl)-5',6"-dimethyl-2H,2"H-[1,2':4',1"-terpyridine]-2,2"-diketone (50 mg, 0.124 mmol) for reaction for 1h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 7.8 mg of a white solid 3"-chloro-4"-((2,5-difluorobenzyl)oxy)-3-(2-hydroxypropane-2-yl)-5',6"-dimethyl-2H,2"H-[1,2':4',1"-terpyridine]-2,2"-diketone is obtained (yield: 12%).

LC-MS: RT = 1.93 min, [M+H]⁺ = 528.21.

### Embodiment 19

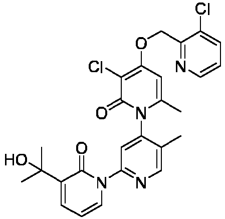

A synthetic route of a compound with a serial number being 19 is shown as follows.

At step A, 3"-chloro-4"-((3-chloropyridine-2-yl)methoxy)-3-(2-hydroxypropane-2-yl)-5',6"-dimethyl-2H,2"H-[1,2':4',1"-terpyridine]-2,2"-diketone is synthesized.

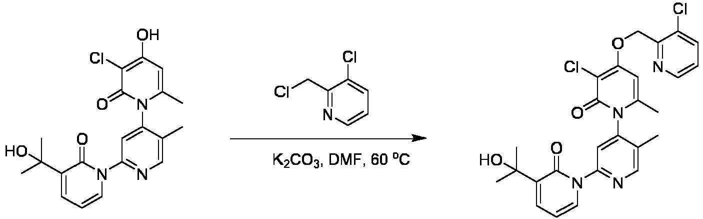

At room temperature, 3-chloro-2-(chloromethyl)pyridine (29.2 mg, 0.18 mmol) and K₂CO₃ (33.12 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 4h at 60 degrees Celsius.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then vacuum concentration is performed. Obtained residues are purified by silica gel column chromatography (eluent: ethyl acetate/n-hexane = 1/0). 19.8 mg of a white solid 3"-chloro-4"-((3-chloropyridine-2-yl)methoxy)-3-(2-hydroxypropane-2-yl)-5',6"-dimethyl-2H,2"H-[1,2':4',1"-terpyridine]-2,2"-diketone is obtained (yield: 31.3%).

LC-MS: RT = 1.84 min, [M+H]⁺ = 527.20. Nuclear magnetic resonance data: ¹H NMR (400 MHz, DMSO-d₆) δ 8.67 (s, 1H), 8.59 (dd, J = 4.7, 1.4 Hz, 1H), 8.05 (dd, J = 8.1, 1.4 Hz, 1H), 7.84 (dd, J = 6.9, 2.1 Hz, 1H), 7.78 (s, 1H), 7.68 (dt, J = 9.0, 2.0 Hz, 1H), 7.51 (dd, J = 8.1, 4.7 Hz, 1H), 6.76 (s, 1H), 6.41 (t, J = 7.0 Hz, 1H), 5.48 (d, J = 12.4 Hz, 2H), 5.21 (s, 1H), 2.07 (s, 3H), 1.98 (s, 3H), 1.46 (d, J = 4.1 Hz, 6H).

### Embodiment 20

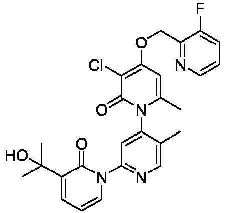

A synthetic route of a compound with a serial number being 20 is shown as follows.

At step A, 3"-chloro-4"-((3-fluoropyridine-2-yl)methoxy)-3-(2-hydroxypropane-2-yl)-5',6"-dimethyl-2H,2"H-[1,2':4',1"-terpyridine]-2,2"-diketone is synthesized.

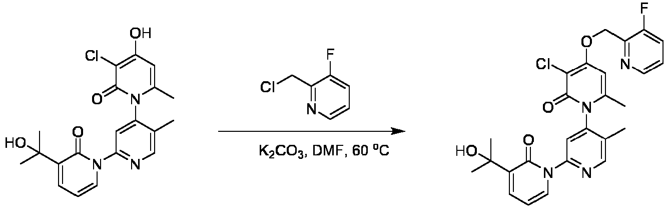

At room temperature, 3-fluoro-2-(chloromethyl)pyridine (26.2 mg, 0.18 mmol) and K₂CO₃ (33.12 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 4h at 60 degrees Celsius.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 23.0 mg of a white solid 3"-chloro-4"-((3-fluoropyridine-2-yl)methoxy)-3-(2-hydroxypropane-2-yl)-5',6"-dimethyl-2H,2"H-[1,2':4',1"-terpyridine]-2,2"-diketone is obtained (yield: 37.5%).

LC-MS: RT = 1.79 min, [M+H]⁺ = 511.20. Nuclear magnetic resonance data: ¹H NMR (400 MHz, DMSO-d₆) δ 8.67 (s, 1H), 8.51-8.47 (m, 1H), 7.86-7.82 (m, 2H), 7.78 (s, 1H), 7.68 (dd, J = 7.0, 2.1 Hz, 1H), 7.57 (dt, J = 8.6, 4.4 Hz, 1H), 6.80 (s, 1H), 6.41 (t, J = 6.9 Hz, 1H), 5.48 (d, J = 1.6 Hz, 2H), 5.21 (s, 1H), 2.07 (s, 3H), 1.99 (s, 3H), 1.46 (d, J = 4.1 Hz, 6H).

### Embodiment 21

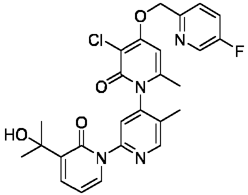

A synthetic route of a compound with a serial number being 21 is shown as follows.

At step A, 3"-chloro-4"-((5-fluoropyridine-2-yl)methoxy)-3-(2-hydroxypropane-2-yl)-5',6"-dimethyl-2H,2"H-[1,2':4',1"-terpyridine]-2,2"-diketone is synthesized.

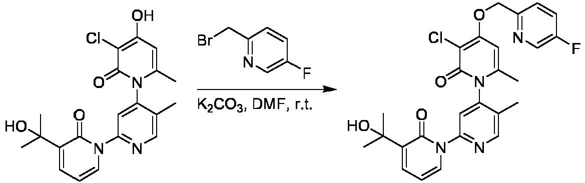

At room temperature, 2-(bromomethyl)-5-fluoropyridine (34.38 mg, 0.18 mmol) and K₂CO₃ (33.12 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 11.0 mg of a white solid 3"-chloro-4"-((5-fluoropyridine-2-yl)methoxy)-3-(2-hydroxypropane-2-yl)-5',6"-dimethyl-2H,2"H-[1,2':4',1"-terpyridine]-2,2"-diketone is obtained (yield: 18.0%).

LC-MS: RT = 1.82 min, [M+H]⁺ = 511.23.

### Embodiment 22

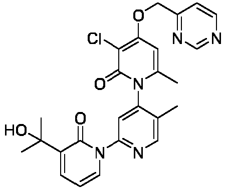

A synthetic route of a compound with a serial number being 22 is shown as follows.

At step A, 3"-chloro-3-(2-hydroxypropane-2-yl)-5',6"-dimethyl-4"-((pyrimidin-4-yl)methoxy)-2H,2"H-[1,2':4',1"-terpyridine]-2,2"-diketone is synthesized.

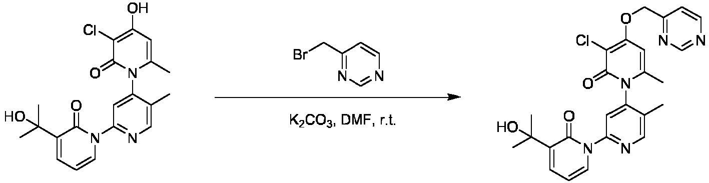

At room temperature, 4-(bromomethyl)pyridine (31.1 mg, 0.18 mmol) and K₂CO₃ (33.1 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 31.5 mg of a white solid 3"-chloro-3-(2-hydroxypropane-2-yl)-5',6"-dimethyl-4"-((pyrimidin-4-yl)methoxy-2H,2"H-[1,2':4',1"-terpyridine]-2,2"-diketone is obtained (yield: 53.2%).

LC-MS: RT = 1.70 min, [M+H]⁺ = 494.19. Nuclear magnetic resonance data: ¹H NMR (400 MHz, DMSO-d₆) δ 9.22 (s, 1H), 8.92 (s, 1H), 8.68 (s, 1H), 7.85 (dd, J = 7.0, 2.1 Hz, 1H), 7.77 (s, 1H), 7.71-7.63 (m, 2H), 6.73 (s, 1H), 6.41 (t, J = 7.0 Hz, 1H), 5.48 (s, 2H), 5.21 (s, 1H), 2.06 (s, 2H), 1.98 (s, 3H), 1.45 (d, J = 4.1 Hz, 6H).

### Embodiment 23

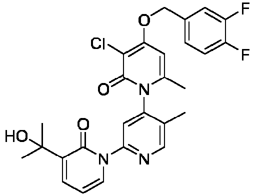

A synthetic route of a compound with a serial number being 23 is shown as follows.

At step A, 3"-chloro-4"-((3,4-difluorobenzyl)oxy)-3-(2-hydroxypropane-2-yl)-5',6"-dimethyl-2H,2"H-[1,2':4',1"-terpyridine]-2,2"-diketone is synthesized.

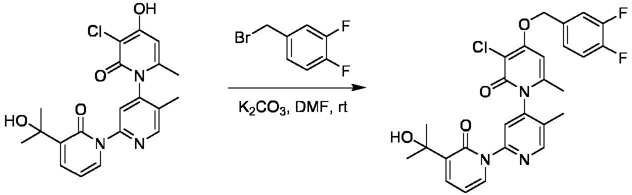

At room temperature, 3,4-difluorobenzyl bromide (31 mg, 0.149 mmol) and K₂CO₃ (35 mg, 0.50 mmol) are added to the N,N-DMF (2.5 ml) containing 3"-chloro-4"-hydroxyl-3-(2-hydroxypropane-2-yl)-5',6"-dimethyl-2H,2"H-[1,2':4',1"-terpyridine]-2,2"-diketone (50 mg, 0.124 mmol) for reaction for 1h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 6.5 mg of a white solid 3"-chloro-4"-((3,4-difluorobenzyl)oxy)-3-(2-hydroxypropane-2-yl)-5',6"-dimethyl-2H,2"H-[1,2':4',1"-terpyridine]-2,2"-diketone is obtained (yield: 10%).

LC-MS: RT = 1.95 min, [M+H]⁺ = 528.22.

### Embodiment 24

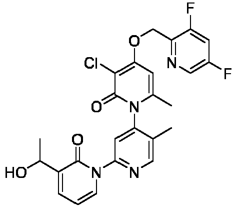

A compound with a serial number being 24 is prepared by means of the following method.

At step A, 2'-bromo-3-chloro-4-((3,5-difluoropyridine-2-yl)methoxy)-5',6-dimethyl-2H-[1,4'-bipyridyl]-2-one is synthesized.

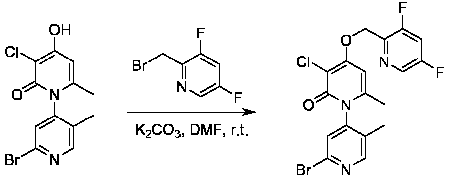

At room temperature, 2-(bromomethyl)-3,5-difluoropyridine (160 mg, 0.77 mmol) and K₂CO₃ (177 mg, 1.28 mmol) are added to an N,N-DMF solution (2.0 ml) containing the 2'-bromo-3-chloro-4-hydroxyl-5',6-dimethyl-2H-[1,4'-bipyridyl]-2-one (211 mg, 0.64 mmol) for reaction for 1h at room temperature.

After the reaction is completed, water (10.0 ml) is added, and then a solid is precipitated and filtered; the obtained filter cake is washed with water for two times; and decompression drying is performed, to obtain 290 mg of a yellowish solid 2'-bromo-3-chloro-4-((3,5-difluoropyridine-2-yl)methoxy)-5',6-dimethyl-2H-[1,4'-bipyridyl]-2-one is obtained (yield: 99%). LC-MS: RT = 1.91 min, [M+H]⁺ = 458.03.

At step B, 3-acetyl-3"-chloro-4"-((3,5-difluoropyridine-2-yl)methoxy)-5',6"-dimethyl-2H,2"H-[1,2':4',1"-terpyridine]-2,2"-diketone is synthesized.

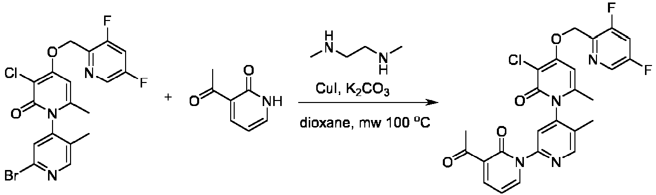

At room temperature, a 1,4-dioxane solution (5.0 ml) containing 2'-bromo-3-chloro-4-((3,5-difluoropyridine-2-yl)methoxy)-5',6-dimethyl-2H-[1,4'-bipyridyl]-2-one (100 mg, 0.22 mmol), 3-acetylpyridine-2(1H)-one (36 mg, 0.26 mmol), K₂CO₃ (60 mg, 0.44 mmol), Cul (8 mg, 0.044 mmol) and dimethylethylenediamine (8 mg, 0.087 mmol) is degassed, for microwave reaction for 1h by warming up the temperature to 100 degrees Celsius under nitrogen protection.

After the reaction is completed, filter is performed; filtrate is concentrated; and obtained residues are purified by silica gel column chromatography (eluent: ethyl acetate/n-hexane = 1/0). 105 mg of a white solid 3-acetyl-3"-chloro-4"-((3,5-difluoropyridine-2-yl)methoxy)-5',6"-dimethyl-2H,2"H-[1,2':4',1"-terpyridine]-2,2"-diketone is obtained (yield: 93.7%). LC-MS: RT = 1.84 min, [M+H]⁺ = 513.16.

At step C, 3"-chloro-4"-((3,5-difluoropyridine-2-yl)methoxy)-3-(1-hydroxyethyl)-5',6"-dimethyl-2H,2"H-[1,2':4',1"-terpyridine]-2,2"-diketone is synthesized.

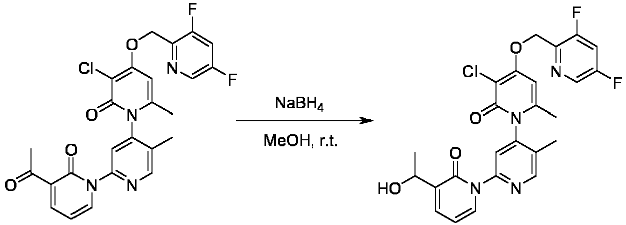

At zero degrees Celsius, sodium borohydride (6 mg, 0.15 mmol) is added to a methanol solution (MeOH, 2.0 ml) containing 3-acetyl-3"-chloro-4"-((3,5-difluoropyridine-2-yl)methoxy)-5',6"-dimethyl-2H,2"H-[1,2':4',1"-terpyridine]-2,2"-diketone (50 mg, 0.097 mmol), for reaction for 0.5h by warming up the temperature to room temperature.

After the reaction is completed, the reaction is quenched with water; extraction is performed with ethyl acetate (3 ml × 3 times); an organic phase is combined; washing is performed with saturated salt water (5 ml × 2 times); and drying is performed with anhydrous sodium sulfate, and then concentration is performed. Obtained residues are purified by silica gel column chromatography (eluent: ethyl acetate/n-hexane = 1/0), and then 36 mg of a white solid 3"-chloro-4"-((3,5-difluoropyridine-2-yl)methoxy)-3-(1-hydroxyethyl)-5',6"-dimethyl-2H,2"H-[1,2':4',1"-terpyridine]-2,2"-diketone is obtained (yield: 72%).

LC-MS: RT = 1.84 min, [M+H]⁺ = 513.16. ¹H NMR (400 MHz, DMSO) δ 8.67 (s, 1H), 8.59 (d, J = 2.3 Hz, 1H), 8.12-8.04 (m, 1H), 7.89-7.83 (m, 1H), 7.79 (d, J = 11.5 Hz, 1H), 7.60-7.53 (m, 1H), 6.79 (s, 1H), 6.43 (t, J = 6.9 Hz, 1H), 5.49 (t, J = 9.4 Hz, 2H), 5.15-5.08 (m, 1H), 4.73 (dd, J = 10.7, 5.6 Hz, 1H), 2.06 (s, 3H), 2.00 (s, 3H), 1.26 (dd, J = 8.8, 6.3 Hz, 3H).

### Embodiment 25

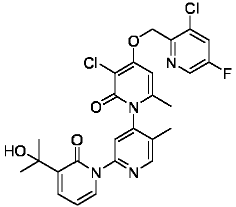

A synthetic route of a compound with a serial number being 25 is shown as follows.

At step A, 2-(bromomethyl)-3-chloro-5-fluoropyridine is synthesized.

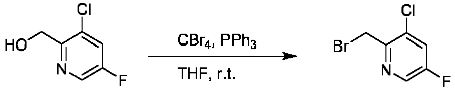

At zero degrees Celsius, PPh₃ (813.5 mg, 3.11 mmol) and CBr₄ (823.8 mg, 2.48 mmol) are added to THF (5.0 ml) containing the (3-chloro-5-fluoropyridine-2-yl)methanol (334.4 mg, 2.07 mmol), for reaction for 1h by warming up the temperature to room temperature.

After the reaction is completed, concentration is performed. Obtained residues are purified by silica gel column chromatography (eluent: n-hexane/ethyl acetate = 10/1). 380.6 mg of colorless oil 2-(bromomethyl)-3-chloro-5-fluoropyridine is obtained (yield: 81.9%).

At step B, 3"-chloro-4"-((3-chloro-5-fluoropyridine-2-yl)methoxy)-3-(2-hydroxypropane-2-yl)-5',6"-dimethyl-2H,2"H-[1,2':4',1"-terpyridine]-2,2"-diketone is synthesized.

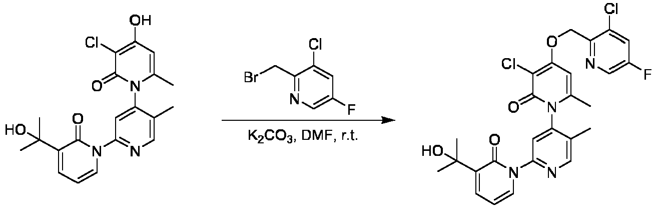

At room temperature, 2-(bromomethyl)-3-chloro-5-fluoropyridine (40.4 mg, 0.18 mmol) and K₂CO₃ (33.12 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1 "-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 15.31 mg of a white solid 3"-chloro-4"-((3-chloro-5-fluoropyridine-2-yl)methoxy)-3-(2-hydroxypropane-2-yl)-5',6"-dimethyl-2H,2"H-[1,2':4',1"-terpyridine]-2,2"-diketone is obtained (yield: 25.7%).

LC-MS: RT = 1.89min, [M+H]⁺ = 545.17.

### Embodiment 26

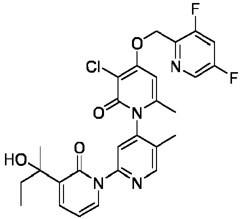

A compound with a serial number being 26 is prepared by means of the following method.

At step A, 3-(2-hydroxybutane-2-yl)pyridine-2-(1H)-one is synthesized.

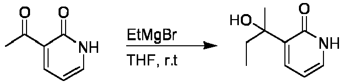

At room temperature, 3-acetylpyridine-2(1H)-one (300.0 mg, 2.2 mmol) is dissolved in THF (10.0 ml), and nitrogen replacement is performed for 3 times; the temperature is cooled to minus 20 degrees Celsius; and ethylmagnesium bromide (EtMgBr, 6.6 ml, 1.0 mol/l) is slowly added dropwise, and after the EtMgBr is added, reaction is performed for 1h by slowly warming up the temperature to room temperature.

After the reaction is completed, the reaction is quenched by adding a saturated ammonium chloride solution (25.0 ml) at zero degrees Celsius; extraction is performed with ethyl acetate (15 ml × 5 times); an organic phase is combined; washing is performed with saturated salt water (10 ml × 1 time); and drying is performed with anhydrous sodium sulfate, and then concentration is performed. Obtained residues are purified by silica gel column chromatography (eluent: n-hexane/ethyl acetate = 1/3). 200.0 mg of colorless oil 3-(2-hydroxybutane-2-yl)pyridine-2(1H)-one is obtained (yield: 54.1%). LC-MS: RT = 1.57 min, [M+H]⁺ = 168.17.

At step B, 3"-chloro-4"-((3,5-difluoropyridine-2-yl)methoxy)-3-(2-hydroxybutane-2-yl)-5',6"-dimethyl-2H,2"H-[1,2':4',1"-terpyridine]-2,2"-diketone is synthesized.

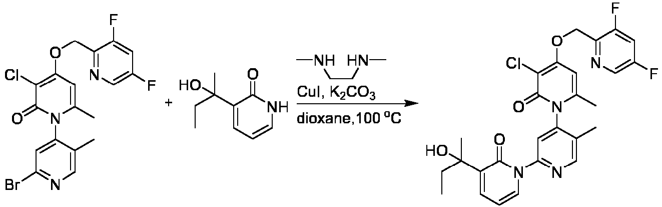

At room temperature, 2'-bromo-3-chloro-4-((3,5-difluoropyridine-2-yl)methoxy)-5',6-dimethyl-2H-[1,4'-bipyridyl]-2-one (200.0 mg, 0.5 mmol), 3-(2-hydroxybutane-2-yl)pyridine-2-(1H)-one (109.7 mg, 0.6 mmol) and K₂CO₃ (138.0 mg, 1.0 mmol) are dissolved in 1,4-dioxane (5.0 ml); Cul (18.2 mg, 1.0 mmol) and dimethylethylenediamine (0.01 ml) are added; and nitrogen replacement is performed for 3 times, for microwave reaction for 1h by warming up the temperature to 100 degrees Celsius.

After the reaction is completed, the reaction is quenched with water; extraction is performed with ethyl acetate (20 ml × 4 times); an organic phase is combined; washing is performed with saturated salt water (10 ml × 1 time); and drying is performed with anhydrous sodium sulfate, and then concentration is performed. Obtained residues are purified by silica gel column chromatography (eluent: dichloromethane/methanol = 1/20). 90.0 mg of a white solid 3"-chloro-4"-((3,5-difluoropyridine-2-yl)methoxy)-3-(2-hydroxybutane-2-yl)-5',6"-dimethyl-2H,2"H-[1,2':4',1"-terpyridine]-2,2"-diketone is obtained (yield: 34.6%).

LC-MS: RT = 1.89 min, [M+H]⁺ = 543.27.

### Embodiment 27

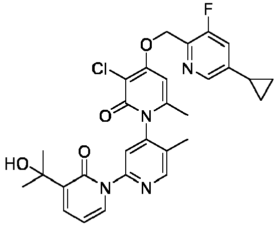

A synthetic route of a compound with a serial number being 27 is shown as follows.

At step A, (3-fluoro-5-cyclopropylpyridine-2-yl)methanol is synthesized.

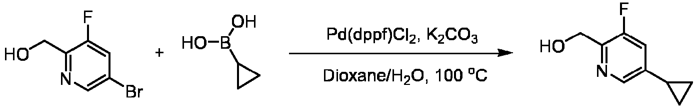

At room temperature, cyclopropylboronic acid (177 mg, 2.24 mmol), a palladium catalyst (Pd(dppf)Cl₂, 82 mg, 0.11 mmol) and K₂CO₃ (309 mg, 2.24 mmol) are added to a mixed solvent (dioxane/water=5/1, 18 ml) containing (3-fluoro-5-bromopyridine-2-yl)methanol (230 mg, 1.12 mmol), for reaction for 8h by warming up the temperature to 100 degrees Celsius.

After the reaction is completed, filter is performed; filtrate is concentrated, and diluted by adding water; extraction is performed with ethyl acetate (10 ml × 3 times); an organic phase is combined; and drying is performed with anhydrous sodium sulfate, and then concentration is performed. Obtained residues are purified by silica gel column chromatography (eluent: ethyl acetate/n-hexane = 1/10), to obtain 80 mg of colourless liquid (3-fluoro-5-cyclopropylpyridine-2-yl)methanol (yield: 43%).

At step B, 2-(bromomethyl)-3-fluoro-5-cyclopropylpyridine is synthesized.

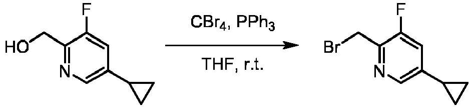

At zero degrees Celsius, PPh₃ (126 mg, 0.48 mmol) and CBr₄ (157 mg, 0.48 mmol) are added to THF (5 ml) containing (3-fluoro-5-cyclopropylpyridine-2-yl)methanol (80 mg, 0.48 mmol), for reaction for 1h at room temperature.

After the reaction is completed, concentration is performed. Obtained residues are purified by silica gel column chromatography (eluent: n-hexane/ethyl acetate = 10/1), to obtain 40 mg of colorless oil 2-(bromomethyl)-3-fluoro-5-cyclopropylpyridine (yield: 36%).

At step C, 3"-chloro-4"-((3-fluoro-5-cyclopropylpyridine-2-yl)methoxy)-3-(2-hydroxypropane-2-yl)-5',6"-dimethyl-2H,2"H-[1,2':4',1"-terpyridine]-2,2"-diketone is synthesized.

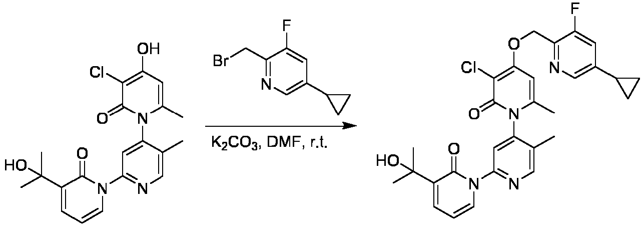

At room temperature, 2-(bromomethyl)-3-fluoro-5-cyclopropylpyridine (40 mg, 0.17 mmol) and K₂CO₃ (48 mg, 0.35 mmol) are added to the N,N-DMF (2.0 ml) containing 3"-chloro-4"-hydroxyl-3-(2-hydroxypropane-2-yl)-5',6"-dimethyl-2H,2"H-[1,2':4',1 "-terpyridine]-2,2"-diketone (68 mg, 0.17 mmol) for reaction for 1h at room temperature.

After the reaction is completed, the reaction is quenched with water; extraction is performed with ethyl acetate (10 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0), to obtain 1.07 mg of a white solid 3"-chloro-4"-((3-fluoro-5-cyclopropylpyridine-2-yl)methoxy)-3-(2-hydroxypropane-2-yl)-5',6"-dimethyl-2H,2"H-[1,2':4',1"-terpyridine]-2,2"-diketone (yield: 1%).

LC-MS: RT = 1.91 min, [M+H]⁺ = 551.21.

### Embodiment 28

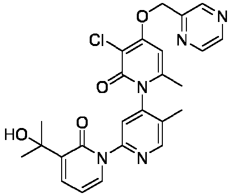

A synthetic route of a compound with a serial number being 28 is shown as follows.

At step A, 3"-chloro-4"-((pyrazin-2-yl)methoxy)-3-(2-hydroxypropane-2-yl)-5',6"-dimethyl-2H,2"H-[1,2':4',1"-terpyridine]-2,2"-diketone is synthesized.

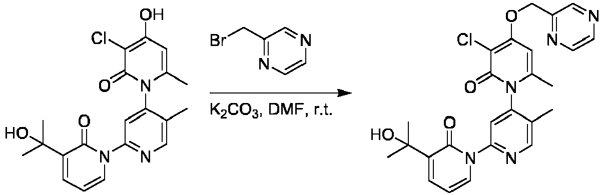

At room temperature, 2-(bromomethyl)pyrazine (31.14 mg, 0.18 mmol) and K₂CO₃ (33.12 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 12.0 mg of a white solid 3"-chloro-4"-((pyrazin-2-yl)methoxy)-3-(2-hydroxypropane-2-yl)-5',6"-dimethyl-2H,2"H-[1,2':4',1"-terpyridine]-2,2"-diketone is obtained (yield: 20.3%).

LC-MS: RT = 1.72 min, [M+H]⁺ = 494.23.

### Embodiment 29

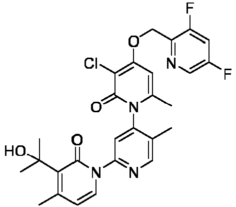

A compound with a serial number being 29 is prepared by means of the following method.

At step A, 3-acetyl-4-methylpyridine-2(1H)-one is synthesized.

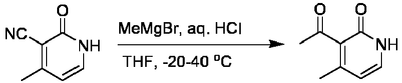

At room temperature, 4-methyl-2-oxo-1,2-dihydropyridine-3-carbonitrile (350.0 mg, 2.6 mmol) is dissolved in THF (10.0 ml), and nitrogen replacement is performed for 3 times; the temperature is cooled to minus 20 degrees Celsius; MeMgBr (2.2 ml, 3.0 mol/l) is added dropwise, and after the MeMgBr is added, reaction is performed for 2h by slowly warming up the temperature to room temperature; reaction is performed for 1h by warming up the temperature to 40 degrees Celsius; and 10 mg, 1 mol/l of hydrochloric acid is added, and reaction is performed for 1h by cooling the temperature to room temperature.

After the reaction is completed, the reaction is quenched by adding 25.0 ml of a saturated sodium bicarbonate solution; extraction is performed with ethyl acetate (15 ml × 3 times); an organic phase is combined; washing is performed with saturated salt water (10 ml × 1 time); and drying is performed with anhydrous sodium sulfate, and then concentration is performed, to obtain 350.0 mg of a yellowish solid 3-acetyl-4-methylpyridine-2(1H)-one (yield: 89.1%). LC-MS: RT = 1.21 min, [M+H]⁺ = 152.10.

At step B, 3-(2-hydroxypropane-2-yl)-4-methylpyridine-2(1H)-one is synthesized.

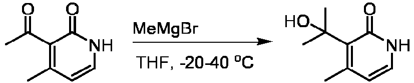

At room temperature, 3-acetyl-4-methylpyridine-2(1H)-one (350.0 mg, 2.3 mmol) is dissolved in THF (10.0 ml), and nitrogen replacement is performed for 3 times; the temperature is cooled to minus 20 degrees Celsius; and MeMgBr (2.3 ml, 3.0 mol/l) is added dropwise, and after the MeMgBr is added, reaction is performed for 2h by slowly warming up the temperature to room temperature.

After the reaction is completed, the reaction is quenched by adding the saturated ammonium chloride solution (30.0 ml); extraction is performed with ethyl acetate (15 ml × 5 times); an organic phase is combined; washing is performed with saturated salt water (10 ml × 1 time); drying is performed with anhydrous sodium sulfate, and then concentration is performed; and obtained residues are purified by silica gel column chromatography (eluent: dichloromethane/methanol = 1/20), to obtain 106.0 mg of a yellowish solid 3-(2-hydroxypropane-2-yl)-4-methylpyridine-2(1H)-one (yield: 27.5%). LC-MS: RT = 1.57 min, [M+H]⁺ = 168.17.

At step C, 3"-chloro-4"-((3,5-difluoropyridine-2-yl)methoxy)-3-(2-hydroxypropane-2-yl)-4,5',6"-trimethyl-2H,2"H-[1,2':4',1"-terpyridine]-2,2"-diketone is synthesized.

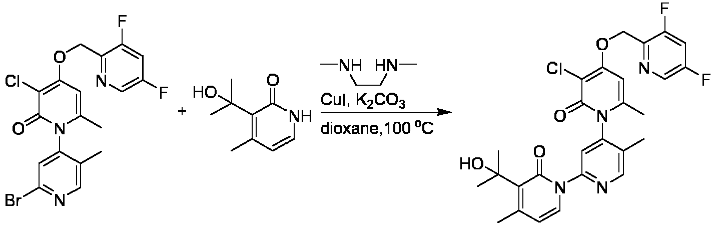

At room temperature, 2'-bromo-3-chloro-4-((3,5-difluoropyridine-2-yl)methoxy)-5',6-dimethyl-2H-[1,4'-bipyridyl]-2-one (100.0 mg, 0.2 mmol), 3-(2-hydroxybutane-2-yl)-4-methylpyridine-2-(1H)-one (54.8 mg, 0.3 mmol) and K₂CO₃ (58.0 mg, 0.4 mmol) are dissolved in 1,4-dioxane (5.0 ml); Cul (8.0 mg, 0.4 mmol) and dimethylethylenediamine (0.01 ml) are added; and nitrogen replacement is performed for 3 times, for microwave reaction for 1h by warming up the temperature to 100 degrees Celsius.

After the reaction is completed, the reaction is quenched with water; extraction is performed with ethyl acetate (20 ml × 4 times); an organic phase is combined; washing is performed with saturated salt water (10 ml × 1 time); and drying is performed with anhydrous sodium sulfate, and then concentration is performed. Obtained residues are purified by silica gel column chromatography (eluent: dichloromethane/methanol = 1/20). 50.0 mg of a yellowish solid 3"-chloro-4"-((3,5-difluoropyridine-2-yl)methoxy)-3-(2-hydroxypropane-2-yl)-4,5',6"-trimethyl-2H,2"H-[1,2':4',1"-terpyridine]-2,2"-diketone is obtained (yield: 43.9%).

LC-MS: RT = 1.89 min, [M+H]⁺ = 543.24. ¹H NMR (400 MHz, DMSO): δ = 8.66 (s, 1H), 8.59 (d, J = 2.4 Hz, 1H), 8.10 - 8.05 (m, 1H), 7.74 (d, J = 1.7 Hz, 1H), 6.79 (s, 1H), 6.21 (d, J = 7.3 Hz, 1H), 5.90 (s, 1H), 5.47 (d, J = 1.5 Hz, 2H), 3.57 (s, 1H), 2.44 (s, 3H), 2.06 (s, 3H), 1.99 (s, 3H), 1.53 (d, J = 3.3 Hz, 6H).

### Embodiment 30

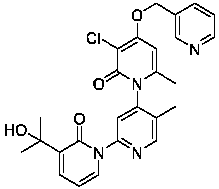

A specific synthetic route of a compound with a serial number being 30 is shown as follows.

At step A, 3"-chloro-3-(2-hydroxypropane-2-yl)-5',6"-dimethyl-4"-((pyridin-3-yl)methoxy)-2H,2"H-[1,2':4',1"-terpyridine]-2,2"-diketone is synthesized.

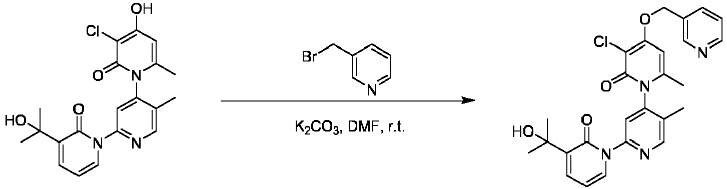

At room temperature, 3-(bromomethyl)pyridine (31.0 mg, 0.18 mmol) and K₂CO₃ (33.12 mg, 0.24 mmol) are added to the N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol) for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 29.6 mg of a white solid 3"-chloro-3-(2-hydroxypropane-2-yl)-5',6"-dimethyl-4"-((pyridin-3-yl)methoxy-2H,2"H-[1,2':4',1"-terpyridine]-2,2"-diketone is obtained (yield: 40.1%).

LC-MS: RT = 1.64 min, [M+H]⁺ = 493.22. Nuclear magnetic resonance data: ¹H NMR (400 MHz, DMSO-d₆) δ 8.71 (d, J = 1.7 Hz, 1H), 8.67 (s, 1H), 8.59 (dd, J = 4.8, 1.5 Hz, 1H), 7.91 (d, J = 7.9 Hz, 1H), 7.84 (dd, J = 6.9, 2.0 Hz, 1H), 7.77 (s, 1H), 7.68 (dd, J = 7.0, 2.1 Hz, 1H), 7.48 (dd, J = 7.8, 4.8 Hz, 1H), 6.77 (s, 1H), 6.41 (t, J = 7.0 Hz, 1H), 5.40 (s, 2H), 5.21 (s, 1H), 2.06 (s, 3H), 2.00 (s, 3H), 1.45 (d, J = 3.8 Hz, 6H).

### Embodiment 31

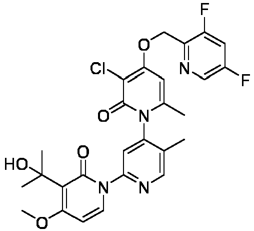

A compound with a serial number being 31 is prepared by means of the following method.

At step A, 3-acetyl-4-methoxypyridine-2(1H)-one is synthesized.

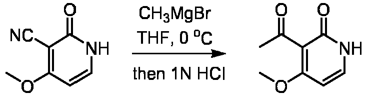

At zero degrees Celsius, MeMgBr (1.33 ml, 4.00 mmol, 3 mol/ml) is added to a THF solution (4.0 ml) containing 4-methoxy-2-oxo-1,2-dihydropyridine-3-carbonitrile (200 mg, 1.33 mmol), for reaction for 1h by warming up the temperature to room temperature; and an aqueous hydrochloric acid solution (1 ml, 1 mol/ml) is added, and the reaction is continued for 1h at room temperature.

After the reaction is completed, the reaction is quenched with water; extraction is performed with ethyl acetate (5ml × 3times); an organic phase is combined; washing is performed with saturated salt water (5 ml × 2 times); and drying is performed with anhydrous sodium sulfate, and then concentration is performed. Obtained residues are purified by silica gel column chromatography (eluent: ethyl acetate/n-hexane = 1/0), to obtain 150 mg of a yellowish solid 3-acetyl-4-methoxypyridine-2(1H)-one (yield: 67.6%). LC-MS: RT = 1.93 min, [M+H]⁺= 168.01.

At step B, 3-(2-hydroxypropane-2-yl)-4-methoxypyridine-2(1H)-one is synthesized.

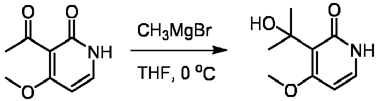

At zero degrees Celsius, MeMgBr (0.90 ml, 2.69 mmol, 3 mol/ml) is added to the THF solution (4.0 ml) containing 3-acetyl-4-methoxypyridine-2(1H)-one (150 mg, 0.90 mmol), for reaction for 1h by warming up the temperature to room temperature.

After the reaction is completed, the reaction is quenched with water; extraction is performed with ethyl acetate (5ml × 3times); an organic phase is combined; washing is performed with saturated salt water (5 ml × 2 times); and drying is performed with anhydrous sodium sulfate, and then concentration is performed.

Obtained residues are purified by silica gel column chromatography (eluent: ethyl acetate/n-hexane = 1/0), to obtain 65 mg of a yellowish solid 3-(2-hydroxypropane-2-yl)-4-methoxypyridine-2(1H)-one (yield: 39.6%).LC-MS: RT = 1.95 min, [M+H]+ = 184.12.

At step C, 3"-chloro-4"-((3,5-difluoropyridine-2-yl)methoxy)-3-(2-hydroxypropane-2-yl)-4-methoxy-5',6"-dimethyl-2H,2"H-[1,2':4',1"-terpyridine]-2,2"-diketone is synthesized.

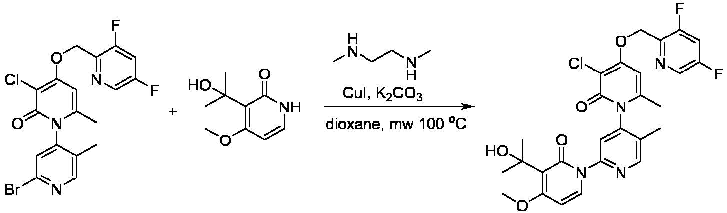

At room temperature, a 1,4-dioxane solution (4.0 ml) containing 2'-bromo-3-chloro-4-((3,5-difluoropyridine-2-yl)methoxy)-5',6-dimethyl-2H-[1,4'-bipyridyl]-2-one (82 mg, 0.18 mmol), 3-(2-hydroxypropane-2-yl)-4-methoxypyridine-2(1H)-one (58 mg, 0.32 mmol), K₂CO₃ (50 mg, 0.36 mmol), Cul (7 mg, 0.036 mmol) and dimethylethylenediamine (6 mg, 0.072 mmol) is degassed, for microwave reaction for 1h by warming up the temperature to 100 degrees Celsius under nitrogen protection.

After the reaction is completed, filter is performed; filtrate is concentrated. Residues are purified by silica gel column chromatography (eluent: ethyl acetate/n-hexane = 1/0). 75 mg of a yellowish solid 3"-chloro-4"-((3,5-difluoropyridine-2-yl)methoxy)-3-(2-hydroxypropane-2-yl)-4-methoxy-5',6"-dimethyl-2H,2"H-[1,2':4',1"-terpyridine]-2,2"-diketone is obtained (yield: 75%).

LC-MS: RT = 1.88 min, [M+H]⁺ = 559.19. ¹H NMR (400 MHz, DMSO) δ8.67 (s, 1H), 8.59 (d, J = 2.4 Hz, 1H),8.10-8.06 (m, 1H),8.00 (d, J = 7.9 Hz, 1H),7.75 (s, 1H),7.59 (s, 1H), 6.78 (s, 1H), 6.60 (d, J = 8.0 Hz, 1H), 5.47 (d, J = 2.0 Hz, 2H), 3.91 (s, 3H), 2.07 (s, 3H), 1.99 (s, 3H), 1.46 (s, 3H), 1.43 (s, 3H).

### Embodiment 32

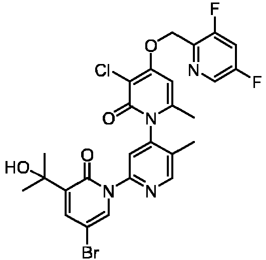

A compound with a serial number being 32 is prepared by means of the following method.

At step A, 3-acetyl-5-bromopyridine-2(1H)-one is synthesized.

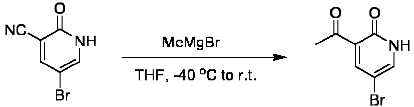

At minus 40 degrees Celsius, a THF solution (1.0 ml, 2.0 mol/l) of MeMgBr is slowly added dropwise into the THF (4.0 ml) containing 5-bromo-2-oxo-1,2-dihydropyridine-3-carbonitrile (199.0 mg, 1.0 mmol), for reaction for 2h by warming up the temperature to room temperature.

After the reaction is completed, the reaction is quenched by adding a diluted hydrochloric acid aqueous solution (4.0 ml, 1.0 mol/l); pH is adjusted to be neutral by using a saturated sodium bicarbonate solution; extraction is performed with dichloromethane (20 ml × 3 times); an organic phase is combined; washing is performed with saturated salt water (20 ml × 2 times); drying is performed with anhydrous sodium sulfate, and then concentration is performed; and obtained residues are directly used for next reaction. LC-MS: RT = 1.57min, [M+H]⁺ =216.02.

At step B, 5-bromo-3-(2-hydroxypropane-2-yl)pyridine-2-(1H)-one is synthesized.

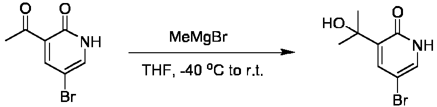

At minus 40 degrees Celsius, the THF solution (1.0 ml, 2.0 mol/l) of MeMgBr is slowly added dropwise into the THF (4.0 ml) containing 3-acetyl-5-bromopyridine-2(1H)-one (216.0 mg, 1.0 mmol), for reaction for 2h by warming up the temperature to room temperature.

After the reaction is completed, the reaction is quenched by adding a diluted hydrochloric acid aqueous solution (4.0 ml, 1.0 mol/l); pH is adjusted to be neutral by using a saturated sodium bicarbonate solution; extraction is performed with dichloromethane (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 2/1). 150 mg of a white solid 5-bromo-3-(2-hydroxypropane-2-yl)pyridine-2-(1H)-one is obtained (yield: 64.7%). LC-MS: RT = 1.63min, [M+H]⁺ =234.08.

At step C, 5-bromo-3"-chloro-4"-((3,5-difluoropyridine-2-yl)methoxy)-3-(2-hydroxypropane-2-yl)-5',6"-dimethyl-2H,2"H-[1,2':4',1"-terpyridine]-2,2"-diketone is synthesized.

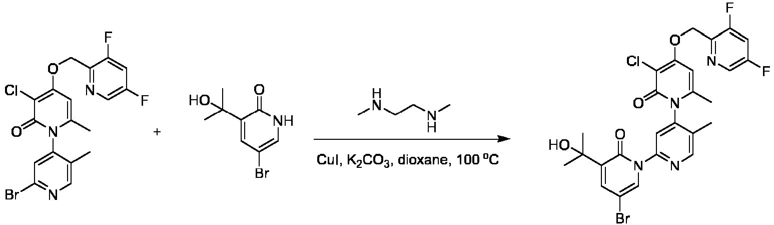

At room temperature, the 2'-bromo-3-chloro-4-((3,5-difluoropyridine-2-yl)methoxy)-5',6-dimethyl-2H-[1,4'-bipyridyl]-2-one (137 mg, 0.30 mmol), 5-bromo-3-(2-hydroxypropane-2-yl)pyridine-2(1H)-one (141.5 mg, 0.61 mmol), CuI (12 mg, 0.06 mmol), and K₂CO₃ (84 mg, 0.61 mmol) are dissolved in the dioxane (4 ml), a drop of dimethylethylenediamine is added, and then the temperature is warmed up to 100 degrees Celsius for microwave reaction for 1h under nitrogen protection.

After the reaction is completed, filter is performed; filtrate is concentrated; and obtained residues are purified by silica gel column chromatography (eluent: ethyl acetate/n-hexane = 1/0). 129.6 mg of a white solid 5-bromo-3"-chloro-4"-((3,5-difluoropyridine-2-yl)methoxy)-3-(2-hydroxypropane-2-yl)-5',6"-dimethyl-2H,2"H-[1,2':4',1"-terpyridine]-2,2"-diketone is obtained (yield: 71.1%).

LC-MS: RT = 1.97 min, [M+H]⁺ = 609.14. Nuclear magnetic resonance data: ¹H NMR (400 MHz, DMSO-d6) δ 8.67 (s, 1H), 8.59 (d, J = 2.3 Hz, 1H), 8.14-8.04 (m, 2H), 7.77 (s, 1H), 7.72 (d, J = 2.8 Hz, 1H), 6.78 (s, 1H), 5.47 (d, J = 1.5 Hz, 2H), 5.28 (s, 1H), 2.07 (s, 3H), 1.98 (s, 3H), 1.45 (d, J = 4.0 Hz, 6H).

### Embodiment 33

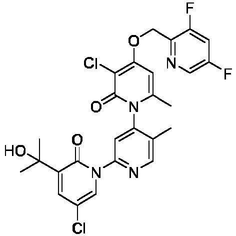

A compound with a serial number being 33 is prepared by means of the following method.

At step A, 3-acetyl-5-chloropyridine-2(1H)-one is synthesized.

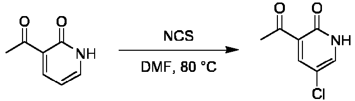

At room temperature, NCS (500 mg, 3.67 mmol) is added to N,N-DMF (5 ml) containing 3-acetylpyridine-2(1H)-one (500 mg, 3.67 mmol), for reaction for 3h by warming up the temperature to 80 degrees Celsius.

After the reaction is completed, the reaction is quenched with water; extraction is performed with ethyl acetate (30 ml × 3 times); an organic phase is combined; washing is performed with saturated salt water (30 ml × 2 times); and drying is performed with anhydrous sodium sulfate, and then concentration is performed. Obtained residues are purified by silica gel column chromatography (eluent: dichloromethane/methanol = 10/1). 300 mg of a yellowish solid 3-acetyl-5-chloropyridine-2(1H)-one is obtained (yield: 54%). LC-MS: RT = 1.68 min, [M+H]⁺ = 172.07.

At step B, 3-(2-hydroxypropane-2-yl)-5-chloropyridine-2(1H)-one is synthesized.

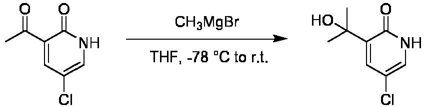

At minus 78 degrees Celsius, a THF solution (3 ml, 1 mol/ml) of MeMgBr is added dropwiseinto the THF (20 ml) containing 3-acetyl-5-chloropyridine-2(1H)-one (300 mg, 1.75 mmol), for reaction for 3h by warming up the temperature to room temperature.

After the reaction is completed, the reaction is quenched with water; extraction is performed with a mixed solution of dichloromethane and isopropanol (dichloromethane/isopropanol=3/1, 30 ml × 5 times); an organic phase is combined; washing is performed with saturated salt water (30 ml × 2 times); and drying is performed with anhydrous sodium sulfate, and then concentration is performed. Obtained residues are purified by silica gel column chromatography (eluent: dichloromethane/methanol = 10/1). 150 mg of a white solid 3-(2-hydroxypropane-2-yl)-5-chloropyridine-2(1H)-one is obtained (yield: 46%). LC-MS: RT = 1.59 min, [M+H]⁺ = 188.10.

At step C, 3",5-dichloro-4"-((3,5-difluoropyridine-2-yl)methoxy)-3-(2-hydroxypropane-2-yl)-5',6"-dimethyl-2H,2"H-[1,2':4',1"-terpyridine]-2,2"-diketone is synthesized.

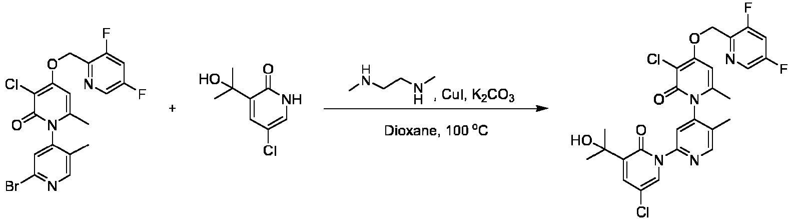

At room temperature, 2'-bromo-3-chloro-4-((3,5-difluoropyridine-2-yl)methoxy)-5',6-dimethyl-2H-[1,4'-bipyridyl]-2-one (80 mg, 0.18 mmol), 3-(2-hydroxypropane-2-yl)-5-chloropyridine-2(1H)-one (33 mg, 0.18 mmol), CuI (7 mg, 0.04 mmol), and K₂CO₃ (48 mg, 0.35 mmol) are dissolved in the dioxane (4 ml), and a drop of dimethylethylenediamine is added, for microwave reaction for 1h by warming up the temperature to 100 degrees Celsius.

After the reaction is completed, filter is performed; drip washing is performed with ethyl acetate, and filtrate is concentrated; and the obtained crude product is prepared with high performance liquid phase to obtain 50 mg of a white solid 3",5-dichloro-4"-((3,5-difluoropyridine-2-yl)methoxy)-3-(2-hydroxypropane-2-yl)-5',6"-dimethyl-2H,2"H-[1,2':4',1"-terpyridine]-2,2"-diketone (yield: 51%).

LC-MS: RT = 1.93 min, [M+H]⁺ = 563.18. ¹H NMR (400 MHz, DMSO-d6) δ 8.67 (s, 1H), 8.59 (d, J = 2.3 Hz, 1H), 8.14-8.01 (m, 2H), 7.78 (s, 1H), 7.65 (d, J = 3.0 Hz, 1H), 6.78 (s, 1H), 5.47 (d, J = 1.4 Hz, 2H), 5.29 (s, 1H), 2.07 (s, 3H), 1.98 (s, 3H), 1.50-1.42 (m, 6H).

### Embodiment 34

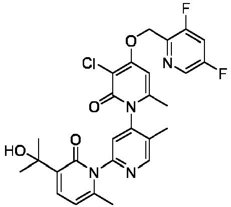

A synthetic route of a compound with a serial number being 34 is shown as follows.

At step A, 3-acetyl-6-methylpyridine-2(1H)-one is synthesized.

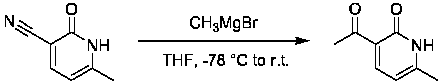

At minus 78 degrees Celsius, a THF solution (5.47 ml, 1 mol/ml) of MeMgBr is added dropwise into the THF (20 ml) containing 6-methyl-2-oxo-1,2-dihydropyridine-3-carbonitrile (500 mg, 3.73 mmol), for reaction for 3h by slowly warming up the temperature to room temperature.

After the reaction is completed, the reaction is quenched with water; extraction is performed with a mixed solution of dichloromethane and isopropanol (dichloromethane/isopropanol=3/1, 30 ml × 5 times); an organic phase is combined; washing is performed with saturated salt water (30 ml × 2 times); and drying is performed with anhydrous sodium sulfate, and then concentration is performed. Obtained residues are purified by silica gel column chromatography (eluent: dichloromethane/methanol = 10/1), to obtain 300 mg of a yellowish solid 3-acetyl-6-methylpyridine-2(1H)-one (yield: 53%). LC-MS: RT = 1.29 min, [M+H]⁺ = 152.15.

At step B, 3-(2-hydroxypropane-2-yl)-6-methylpyridine-2(1H)-one is synthesized.

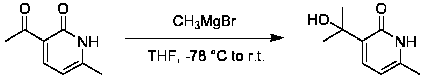

At minus 78 degrees Celsius, a THF solution (3 ml, 1 mol/ml) of MeMgBr is added dropwise into the THF (20 ml) containing 3-acetyl-6-methylpyridine-2(1H)-one (300 mg, 1.98 mmol), for reaction for 3h by warming up the temperature to room temperature.

After the reaction is completed, the reaction is quenched with water; extraction is performed with a mixed solution of dichloromethane and isopropanol (dichloromethane/isopropanol=3/1, 30 ml × 5 times); an organic phase is combined; washing is performed with saturated salt water (30 ml × 2 times); and drying is performed with anhydrous sodium sulfate, and then concentration is performed. Obtained residues are purified by silica gel column chromatography (eluent: dichloromethane/methanol = 10/1). 200 mg of a white solid 3-(2-hydroxypropane-2-yl)-6-methylpyridine-2(1H)-one is obtained (yield: 60%). LC-MS: RT = 1.52 min, [M+H]⁺ = 168.20.

At step C, 3"-chloro-4"-((3,5-difluoropyridine-2-yl)methoxy)-3-(2-hydroxypropane-2-yl)-5',6,6"-trimethyl-2H,2"H-[1,2':4',1"-terpyridine]-2,2"-diketone is synthesized.

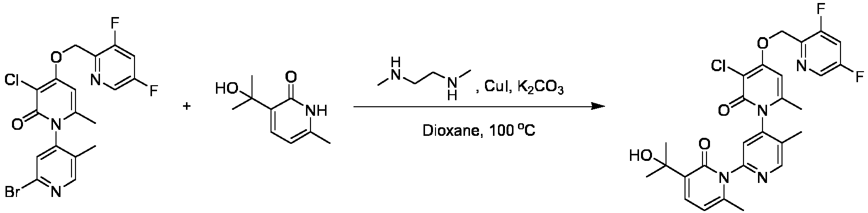

At room temperature, 2'-bromo-3-chloro-4-((3,5-difluoropyridine-2-yl)methoxy)-5',6-dimethyl-2H-[1,4'-bipyridyl]-2-one (80 mg, 0.18 mmol), 3-(2-hydroxypropane-2-yl)-6-methylpyridine-2(1H)-one (30 mg, 0.18 mmol), Cul (7 mg, 0.04 mmol), and K₂CO₃ (48 mg, 0.35 mmol) are dissolved in the dioxane (4 ml), and a drop of dimethylethylenediamine is added, for microwave reaction for 1h by warming up the temperature to 100 degrees Celsius.

After the reaction is completed, filter is performed; drip washing is performed with ethyl acetate, and filtrate is concentrated; and the obtained crude product is prepared with high performance liquid phase to obtain 2.49 mg of a white solid 3"-chloro-4"-((3,5-difluoropyridine-2-yl)methoxy)-3-(2-hydroxypropane-2-yl)-5',6,6"-trimethyl-2H,2"H-[1,2':4',1"-terpyridine]-2,2"-diketone (yield: 3%).

LC-MS: RT = 1.88 min, [M+H]⁺ = 543.18.

### Embodiment 35

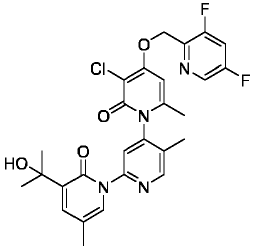

A synthetic route of a compound with a serial number being 35 is shown as follows.

At step A, 3"-chloro-4"-((3,5-difluoropyridine-2-yl)methoxy)-3-(2-hydroxypropane-2-yl)-5,5',6"-trimethyl-2H,2"H-[1,2':4',1"-terpyridine]-2,2"-diketone is synthesized.

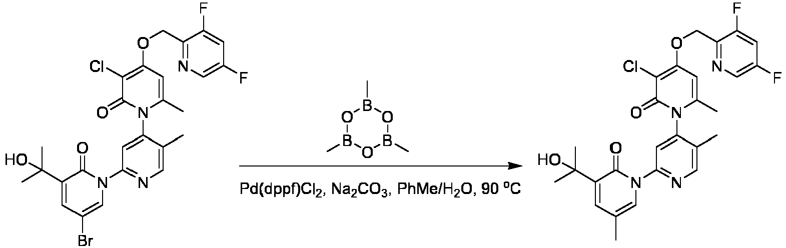

At room temperature, trimethylboroxine (0.02 ml, 0.16 mmol), [1,1'-Bis(diphenylphosphino)ferrocene]dichloropalladium (1.17 mg, 0.0016 mmol) and Na₂CO₃ (33.9 mg, 0.32 mmol) are added to a mixed solvent (2.0 ml, toluene/water=3/1) containing 5-bromo-3"-chloro-4"-((3,5-difluoropyridine-2-yl)methoxy)-3-(2-hydroxypropane-2-yl)-5',6"-dimethyl-2H,2"H-[1,2':4',1"-terpyridine]-2,2"-diketone (100 mg, 0.16 mmol), for reaction for 2h by warming up the temperature to 90 degrees Celsius under nitrogen protection.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then concentration is performed. Obtained residues are purified by silica gel column chromatography (eluent: ethyl acetate/n-hexane = 1/0). 15.4 mg of a white solid 3"-chloro-4"-((3,5-difluoropyridine-2-yl)methoxy)-3-(2-hydroxypropane-2-yl)-5,5',6"-trimethyl-2H,2"H-[1,2':4',1"-terpyridine]-2,2"-diketone is obtained (yield: 17.8%).

LC-MS: RT = 1.89 min, [M+H]⁺ = 543.25.

### Embodiment 36

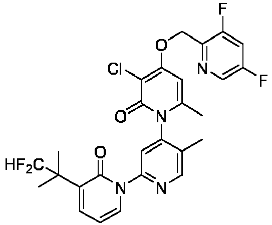

A synthetic route of a compound with a serial number being 36 is shown as follows.

At step A, 3-(bromomethyl)-2-methoxypyridine is synthesized.

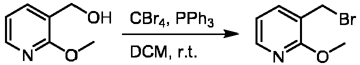

At zero degrees Celsius, PPh₃ (5.65 g, 21.56 mmol) and CBr₄ (5.72 g, 17.24 mmol) are added to dichloromethane (30.0 ml) containing (2-methoxypyridine-3-yl)methanol (2.0 g, 14.37 mmol) for reaction for 1h at room temperature.

After reaction is completed, vacuum concentration is performed. Obtained residues are purified by silica gel column chromatography (eluent: n-hexane). 2.95 g of yellow colorless oil 3-(bromomethyl)-2-methoxypyridine is obtained (yield: 97.7%). LCMS: RT = 1.97 min, [M+H]⁺ = 202.03.

At step B, 2-(2-methoxypyridine-3-yl)acetonitrile is synthesized.

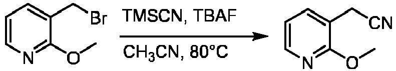

At room temperature, trimethylsilyl cyanide (3.60 ml, 28.68 mmol) and a THF solution (28.7 ml, 1.0 mol/l) of tetrabutylammonium fluoride are added to acetonitrile (30.0 ml) containing 3-(bromomethyl)-2-methoxypyridine (2.9 g, 14.34 mmol), for reaction for 1h at 80 degrees Celsius.

After the reaction is completed, the reaction is quenched with water; extraction is performed with ethyl acetate (30 ml × 3 times); an organic phase is combined; washing is performed with saturated salt water (20 ml × 2 times); and drying is performed with anhydrous sodium sulfate, and then vacuum concentration is performed. Obtained residues are purified by silica gel column chromatography (eluent: n-hexane). 1.90 g of a white solid 2-(2-methoxypyridine-3-yl)acetonitrile is obtained (yield: 88.82%). LCMS: RT = 1.73 min, [M+H]⁺ = 149.17.

At step C, 2-(2-methoxypyridine-3-yl)-2-methylpropionitrile is synthesized.

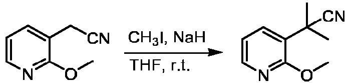

At room temperature, THF (5.0 ml) containing 2-(2-methoxypyridine-3-yl)acetonitrile (1.90 g, 0.013 mmol) is added to THF (15.0 ml) containing sodium hydride (2.0 g, 0.05 mmol, 60% of which being dispersed in paraffin oil), for reaction for 2h at room temperature; and CH₃I is added, to continue the reaction overnight.

After the reaction is completed, the reaction is quenched with water; extraction is performed with ethyl acetate (30 ml × 3 times); an organic phase is combined; washing is performed with saturated salt water (20 ml × 2 times); and drying is performed with anhydrous sodium sulfate, and then vacuum concentration is performed. Obtained residues are purified by silica gel column chromatography (eluent: n-hexane). 2.17 g of colourless liquid 2-(2-methoxypyridine-3-yl)-2-methylpropionitrile is obtained (yield: 94.2%). LCMS: RT = 1.89 min, [M+H]⁺ = 177.11.

At step D, 2-(2-methoxypyridine-3-yl)-2-methylpropanal is synthesized.

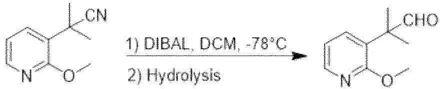

At minus 78 degrees Celsius, a toluene solution (12.15 ml, 1.5 mol/l) of diisobutylaluminium hydride is added to dichloromethane (200.0 ml) containing 2-(2-methoxypyridine-3-yl)-2-methylpropionitrile (2.17 g, 12.23 mmol), for reaction by warm up the temperature to room temperature.

After the reaction is completed, the reaction is quenched with water; dichloromethane is evaporated; diluted hydrochloric acid is added to stir for 30 min; extraction is performed with ethyl acetate (30 ml × 3 times); an organic phase is combined; washing is performed with saturated salt water (20 ml × 2 times); and drying is performed with anhydrous sodium sulfate, and then vacuum concentration is performed. Obtained residues are purified by silica gel column chromatography (eluent: n-hexane). 1.56 g of colourless liquid 2-(2-methoxypyridine-3-yl)-2-methylpropanal is obtained (yield: 70.9%). LCMS: RT = 1.91 min, [M+H]⁺ = 180.16.

At step E, 3-(1,1-difluoro-2-methylpropane-2-yl)-2-methoxypyridine is synthesized.

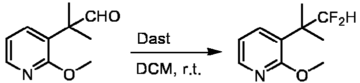

At zero degrees Celsius, diethylaminosulfurtrifluoride (2.13 ml, 17.34 mol/l) is added to dichloromethane (20.0 ml) containing 2-(2-methoxypyridine-3-yl)-2-methylpropanal (1.56 g, 8.67 mmol), for reaction at room temperature.

After the reaction is completed, the reaction is quenched with water; extraction is performed with dichloromethane (30 ml × 3 times); an organic phase is combined; washing is performed with saturated salt water (20 ml × 2 times); and drying is performed with anhydrous sodium sulfate, and then vacuum concentration is performed. Obtained residues are purified by silica gel column chromatography (eluent: n-hexane). 700.0 mg of colourless liquid 3-(1,1-difluoro-2-methylpropane-2-yl)-2-methoxypyridine is obtained (yield: 40.0%). LCMS: RT = 2.05 min, [M+H]⁺ = 202.15.

At step F, 3-(1,1-difluoro-2-methylpropane-2-yl)pyridine-2(1H)-one is synthesized.

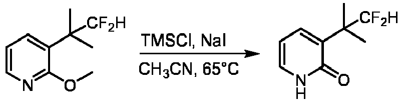

At room temperature, sodium iodide (780.7 mg, 5.2 mmol) and chlorotrimethylsilane (660.0 µL, 5.2 mmol) are added to acetonitrile (7.0 ml) containing 3-(1,1-difluoro-2-methylpropane-2-yl)-2-methoxypyridine (700.0 mg, 3.47 mmol), for reaction at 65 degrees Celsius.

After the reaction is completed, the reaction is quenched with water; pH is adjusted to 8 with saturated sodium carbonate; extraction is performed with ethyl acetate (30 ml × 3 times); an organic phase is combined; washing is performed with saturated salt water (20 ml × 2 times); and drying is performed with anhydrous sodium sulfate, and then vacuum concentration is performed. Obtained residues are purified by silica gel column chromatography (eluent: methanol/dichloromethane = 1/20). 533.0 mg of yellow colorless oil 3-(1,1-difluoro-2-methylpropane-2-yl)pyridine-2(1H)-one is obtained (yield: 81.6%). LCMS: RT = 1.68 min, [M+H]⁺ = 188.16.

At step G, 3"-chloro-3-(1,1-difluoro-2-methylpropane-2-yl)-4"-((3,5-difluoropyridine-2-yl)methoxy)-5',6"-dimethyl-2H,2"H-[1,2':4',1"-terpyridine]-2,2"-diketone is synthesized.

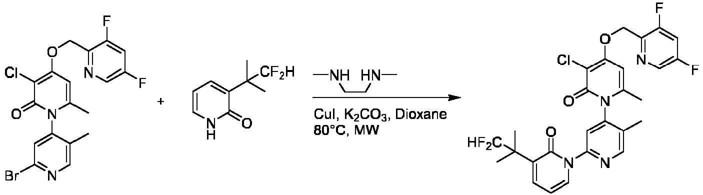

At room temperature, the 2'-bromo-3-chloro-4-((3,5-difluoropyridine-2-yl)methoxy)-5',6-dimethyl-2H-[1,4'-bipyridyl]-2-one (100.0 mg, 0.22 mmol), 3-(1,1-difluoro-2-methylpropane-2-yl)pyridine-2(1H)-one (49.6 mg, 0.26 mmol), Cul (9.5 mg, 0.05 mmol), and K₂CO₃ (60.7 mg, 0.44 mmol) are dissolved in the dioxane (2.0 ml), and a drop of N,N-dimethylethylenediamine is added, for microwave reaction for 1h at 80 degrees Celsius.

After the reaction is completed, suction filtration is performed; a filter cake is washed with dichloromethane; and vacuum concentration is performed on filtrate. Obtained residues are purified by silica gel column chromatography (eluent: ethyl acetate). 28.0 mg of a white solid 3"-chloro-3-(1,1-difluoro-2-methylpropane-2-yl)-4"-((3,5-difluoropyridine-2-yl)methoxy)-5',6"-dimethyl-2H,2"H-[1,2':4',1"-terpyridine]-2,2"-diketone is obtained (yield: 22.6%). LCMS: RT = 1.99 min, [M+H]⁺ = 563.17.

### Embodiment 37

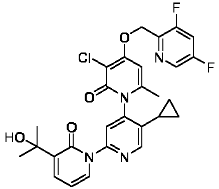

A synthetic route of a compound with a serial number being 37 is shown as follows.

At step A, 2-chloro-5-cyclopropylpyridine-4-amine is synthesized.

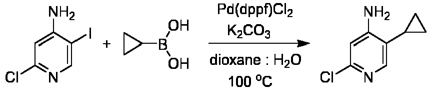

At room temperature, a 1,4-dioxane solution (20.0 ml) containing 2-chloro-5-iodo-4-pyridinamine (1.0 g, 3.93 mmol), cyclopropylboronic acid (0.68 g, 7.86 mmol), [1,1'-Bis(diphenylphosphino)ferrocene]dichloropalladium (290 mg, 0.39 mmol) and K₂CO₃ (1.09 g, 7.86 mmol) is warmed up to 100°C for reaction for 24h.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then vacuum concentration is performed. Obtained residues are purified by silica gel column chromatography (eluent: ethyl acetate/n-hexane = 1/5), to obtain 580 mg of 2-chloro-5-cyclopropylpyridine-4-amine (yield: 87.6%). LCMS: RT = 0.7 min, [M+H]⁺ = 169.09.

At step B, 2'-chloro-5'-cyclopropyl-4-hydroxyl-6-methyl-2H-[1,4'-bipyridyl]-2-one is synthesized.

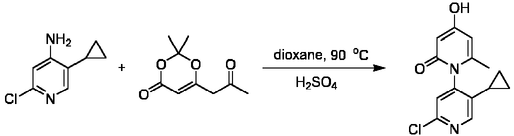

At room temperature, 2,2-dimethyl-6-(2-oxopropyl)-4H-1,3-dioxin-4-one (760 mg, 4.13 mmol) is added to 1,4-dioxane (10.0 ml) containing 2-chloro-5-cyclopropylpyridine-4-amine (580 mg, 3.44 mmol), for reaction for 2h by warming up the temperature to 90°C. Then, a sulfuric acid solution (1.5 ml, 10 mol/l) is added dropwise, to continue to react for 2h at 90°C.

After the reaction is completed, the temperature is cooled to room temperature; a solid is precipitated by adding water, and filter is performed; drip washing is performed with water for 2 times; and then decompression drying is performed, to obtain 550 mg of a yellowish solid 2'-chloro-5'-cyclopropyl-4-hydroxyl-6-methyl-2H-[1,4'-bipyridyl]-2-one is obtained (yield: 57.8%). LCMS: RT = 1.70 min, [M+H]⁺ = 277.12.

At step C, 2',3-dichloro-5'-cyclopropyl-4-hydroxyl-6-methyl-2H-[1,4'-bipyridyl]-2-one is synthesized.

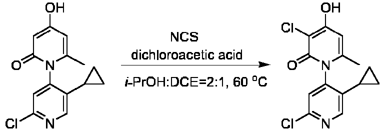

At room temperature, 1,2-dichloroethane (2.5 ml), NCS (159 mg, 1.19 mmol) and dichloroacetic acid (1 drop) are added to isopropanol (5.0 ml) containing 2'-chloro-5'-cyclopropyl-4-hydroxyl-6-methyl-2H-[1,4'-bipyridyl]-2-one (300 mg, 1.08 mmol); and the mixed solution is warmed up to 65 degrees Celsius for reaction for 1h.

After the reaction is completed, the reaction is quenched by adding water (1.0 ml); a solvent is removed under reduced pressure; ethyl acetate is added to the obtained residues for pulping, and then filter is performed; and decompression drying is performed, to obtain 230 mg of a yellowish solid 2',3-dichloro-5'-cyclopropyl-4-hydroxyl-6-methyl-2H-[1,4'-bipyridyl]-2-one is obtained (yield: 68.2%). LCMS: RT = 1.73 min, [M+H]⁺ = 311.10.

At step D, 2',3-dichloro-5'-cyclopropyl-4-((3,5-difluoropyridine-2-yl)methoxy)-6-methyl-2H-[1,4'-bipyridyl]-2-one is synthesized.

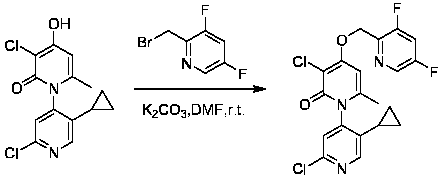

At room temperature, 2-(bromomethyl)-3,5-difluoropyridine (136 mg, 0.66 mmol) and K₂CO₃ (151 mg, 1.01 mmol) are added to an N,N-DMF solution (2.0 ml) containing 2',3-dichloro-5'-cyclopropyl-4-hydroxyl-6-methyl-2H-[1,4'-bipyridyl]-2-one (170 mg, 0.55 mmol) for reaction for 1h at room temperature.

After the reaction is completed, water (10.0 ml) is added, and then a solid is precipitated; filter is performed, and drip washing is performed with water for two times; and decompression drying is performed, to obtain 180 mg of a yellowish solid 2',3-dichloro-5'-cyclopropyl-4-((3,5-difluoropyridine-2-yl)methoxy)-6-methyl-2H-[1,4'-bipyridyl]-2-one is obtained (yield: 75%). LCMS: RT = 1.96 min, [M+H]⁺ = 438.13.

At step E, 3"-chloro-5'-cyclopropyl-4"-((3,5-difluoropyridine-2-yl)methoxy)-3-(2-hydroxypropane-2-yl)-6"-methyl-2H,2"H-[1,2':4',1"-terpyridine]-2,2"-diketone is synthesized.

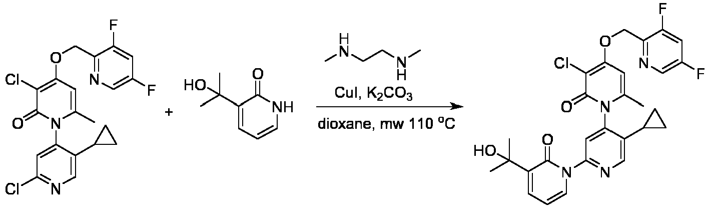

At room temperature, 1,4-dioxane (5.0 ml) containing 2',3-dichloro-5'-cyclopropyl-4-((3,5-difluoropyridine-2-yl)methoxy)-6-methyl-2H-[1,4'-bipyridyl]-2-one (180 mg, 0.41 mmol), 3-(2-hydroxypropane-2-yl)-pyridine-2(1H)-one (75 mg, 0.49 mmol), K₂CO₃ (114 mg, 0.82 mmol), CuI (16 mg, 0.082 mmol) and dimethylethylenediamine (15 mg, 0.16 mmol) is degassed, for microwave reaction for 2h at 110 degrees Celsius under nitrogen protection.

After the reaction is completed, filter is performed; and vacuum concentration is performed. Obtained residues are purified by silica gel column chromatography (eluent: ethyl acetate). 16 mg of a yellowish solid 3"-chloro-5'-cyclopropyl-4"-((3,5-difluoropyridine-2-yl)methoxy)-3-(2-hydroxypropane-2-yl)-6"-methyl-2H,2"H-[1,2':4',1"-terpyridine]-2,2"-diketone is obtained (yield: 7%). LCMS: RT = 1.89min, [M+H]⁺ = 555.22. ¹H NMR (400 MHz, DMSO-d₆) δ 8.59 (d, J = 2.4 Hz, 1H), 8.40 (s, 1H), 8.14-8.01 (m, 1H), 7.82 (dd, J = 6.9, 2.0 Hz, 1H), 7.75 (s, 1H), 7.68 (dd, J = 7.1, 2.1 Hz, 1H), 6.80 (s, 1H), 6.40 (t, J = 6.9 Hz, 1H), 5.47 (s, 2H), 5.22 (s, 1H), 2.02 (s, 3H), 1.54-1.47 (m, 1H), 1.46 (s, 3H), 1.44 (s, 3H), 1.06-1.00 (m, 1H), 0.89 (m, 2H), 0.72-0.67 (m, 1H).

### Embodiment 38

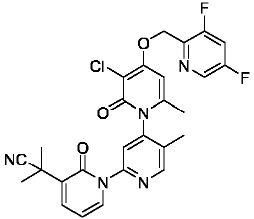

A synthetic route of a compound with a serial number being 38 is shown as follows.

At step A, 2-methyl-2-(2-oxo-1,2-dihydropyridine-3-yl)propionitrile is synthesized.

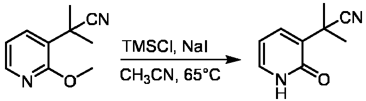

At room temperature, sodium iodide (255.0 mg, 1.7 mmol) and chlorotrimethylsilane (215.0 µL, 1.7 mmol) are added to acetonitrile (3.0 ml) containing 2-(2-methoxypyridine-3-yl)-2-methylpropionitrile (200.0 mg, 1.13 mmol), for reaction by warming up the temperature to 65 degrees Celsius.

After the reaction is completed, the reaction is quenched with water; pH is adjusted to 8 with saturated sodium carbonate; extraction is performed with ethyl acetate (30 ml × 3 times); an organic phase is combined; washing is performed with saturated salt water (20 ml × 2 times); and drying is performed with anhydrous sodium sulfate, and then vacuum concentration is performed. Obtained residues are purified by silica gel column chromatography (eluent: methanol/dichloromethane = 1/20). 170.0 mg of yellow colorless oil 2-methyl-2-(2-oxo-1,2-dihydropyridine-3-yl)propionitrile is obtained (92.3%). LCMS: RT = 1.56 min, [M+H]⁺ = 163.13.

At step B, 2-(3"-chloro-4"-((3,5-difluoropyridine-2-yl)methoxy)-5',6"-dimethyl-2,2"-diketone-2H,2"H-[1,2':4',1"-terpyridine]-3-yl)-2-methylpropionitrile is synthesized.

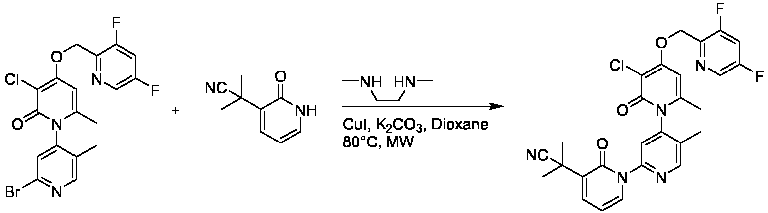

At room temperature, 2'-bromo-3-chloro-4-((3,5-difluoropyridine-2-yl)methoxy)-5',6-dimethyl-2H-[1,4'-bipyridyl]-2-one (100.0 mg, 0.22 mmol), 2-methyl-2-(2-oxo-1,2-dihydropyridine-3-yl)propionitrile (42.6 mg, 0.26 mmol), Cul (9.5 mg, 0.05 mmol), and K₂CO₃ (60.7 mg, 0.44 mmol) are dissolved in dioxane (2.0 ml), and a drop of N,N-dimethylethylenediamine is added, for microwave reaction for 1h at 80 degrees Celsius.

After the reaction is completed, suction filtration is performed; drip washing is performed with dichloromethane; and vacuum concentration is performed. Obtained residues are purified by silica gel column chromatography (eluent: ethyl acetate). 37.0 mg of a white solid 2-(3"-chloro-4"-((3,5-difluoropyridine-2-yl)methoxy)-5',6"-dimethyl-2,2"-diketone-2H,2"H-[1,2':4',1"-terpyridine]-3-yl)-2-methylpropionitrile is obtained (yield: 31.2%). LCMS: RT = 1.91 min, [M+H]⁺ = 538.20.

### Embodiment 39

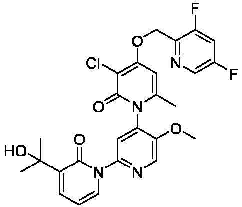

A synthetic route of a compound with a serial number being 39 is shown as follows.

At step A, 2'-chloro-4-hydroxyl-5'-methoxy-6-methyl-2H-[1,4'-bipyridyl]-2-one is synthesized.

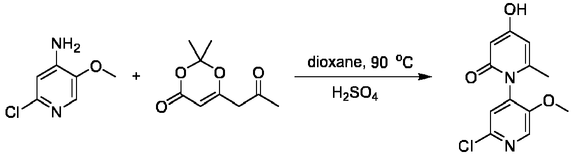

At room temperature, 2,2-dimethyl-6-(2-oxopropyl)-4H-1,3-dioxin-4-one (700 mg, 3.78 mmol) is added to 1,4-dioxane (10.0 ml) containing 2-chloro-4-amino-5-methoxypyridine (500 mg, 3.15 mmol), for reaction for 2h by warming up the temperature to 90degrees Celsius. Then, a sulfuric acid solution (1.2 ml, 10 mol/l) is added dropwise, to continue to react for 2h at 90degrees Celsius.

After the reaction is completed, the temperature is cooled to room temperature; a solid is precipitated by adding water, and filter is performed; drip washing is performed with water for 2 times; and then decompression drying is performed, to obtain 540 mg of a yellowish solid 2'-chloro-4-hydroxyl-5'-methoxy-6-methyl-2H-[1,4'-bipyridyl]-2-one is obtained (yield: 64.3%). LCMS: RT = 1.66 min, [M+H]⁺ = 267.04.

At step B, 2',3-dichloro-4-hydroxyl-5'-methoxy-6-methyl-2H-[1,4'-bipyridyl]-2-one is synthesized.

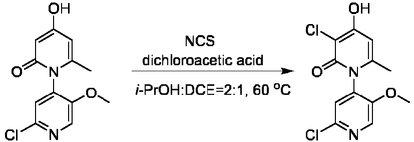

At room temperature, 1,2-dichloroethane (3.0 ml), NCS (297 mg, 2.23 mmol) and dichloroacetic acid (3 drops) are added to isopropanol (6.0 ml) containing 2'-chloro-4-hydroxyl-5'-methoxy-6-methyl-2H-[1,4'-bipyridyl]-2-one (540 mg, 2.02 mmol), for reaction for 1h by warming up the temperature to 65 degrees Celsius.

After the reaction is completed, the reaction is quenched by adding water (1.0 ml); a solvent is removed under reduced pressure; pulping is performed by adding ethyl acetate, and then filter is performed; and decompression drying is performed, to obtain 450 mg of a yellowish solid 2',3-dichloro-4-hydroxyl-5'-methoxy-6-methyl-2H-[1,4'-bipyridyl]-2-one is obtained (yield: 73.9%). LCMS: RT = 1.68 min, [M+H]⁺ = 301.09.

At step C, 2',3-dichloro-4-((3,5-difluoropyridine-2-yl)methoxy)-5'-methoxy-6-methyl-2H-[1,4'-bipyridyl]-2-one is synthesized.

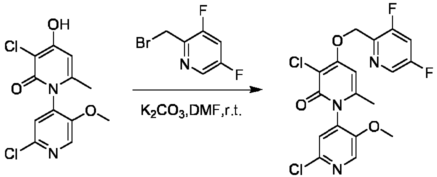

At room temperature, 2-(bromomethyl)-3,5-difluoropyridine (180 mg, 0.87 mmol) and K₂CO₃(200 mg, 1.44 mmol) are added to an N,N-DMF solution (3.0 ml) containing 2',3-dichloro-4-hydroxyl-5'-methoxy-6-methyl-2H-[1,4'-bipyridyl]-2-one (217 mg, 0.72 mmol), for reaction for 1h at room temperature.

After the reaction is completed, water (10.0 ml) is added, and then a solid is precipitated; filter is performed, and drip washing is performed with water for two times; and decompression drying is performed, to obtain 220 mg of a yellowish solid 2',3-dichloro-4-((3,5-difluoropyridine-2-yl)methoxy)-5'-methoxy-6-methyl-2H-[1,4'-bipyridyl]-2-one is obtained (yield: 71.4%). LCMS: RT = 1.89 min, [M+H]⁺ = 428.07.

At step D, 3"-chloro-4"-((3,5-difluoropyridine-2-yl)methoxy)-3-(2-hydroxypropane-2-yl)-5'-methoxy-6"-methyl-2H,2"H-[1,2':4',1"-terpyridine]-2,2"-diketone is synthesized.

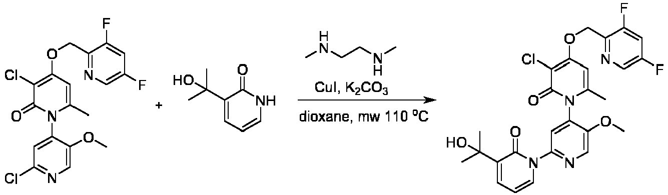

At room temperature, 1,4-dioxane (5.0 ml) containing 2',3-dichloro-4-((3,5-difluoropyridine-2-yl)methoxy)-5'-methoxy-6-methyl-2H-[1,4'-bipyridyl]-2-one (220 mg, 0.51 mmol), 3-(2-hydroxypropane-2-yl)-pyridine-2(1H)-one (118 mg, 0.77 mmol), K2CO3 (132 mg, 1.02 mmol), Cul (49 mg, 0.26 mmol) and dimethylethylenediamine (46 mg, 0.51 mmol) is degassed, for microwave reaction for 2h at 110 degrees Celsius under nitrogen protection.

After the reaction is completed, filter is performed; and vacuum concentration is performed. Obtained residues are purified by silica gel column chromatography (eluent: ethyl acetate). 22 mg of a yellowish solid 3"-chloro-4"-((3,5-difluoropyridine-2-yl)methoxy)-3-(2-hydroxypropane-2-yl)-5'-methoxy-6"-methyl-2H,2"H-[1,2':4',1"-terpyridine]-2,2"-diketone is obtained (yield: 7.8%). LCMS: RT = 1.86 min, [M+H]⁺ = 545.21.

### Embodiment 40

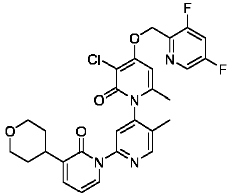

A synthetic route of a compound with a serial number being 40 is shown as follows.

At step A, 3-bromo-2-((4-methoxybenzyl)oxy)pyridine is synthesized.

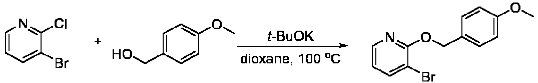

At room temperature, 4-methoxybenzyl alcohol (1.5 g, 11.0 mmol) and potassium tert-butoxide (1.34 g, 12.0 mmol) are added to 1,4-dioxane (30.0 ml) containing 3-bromo-2-chloropyridine (1.92 g, 10.0 mmol), for reaction for 2h by warming up the temperature to 100 degrees Celsius.

After the reaction is completed, the reaction is quenched with water; extraction is performed with ethyl acetate (50 ml × 3 times); an organic phase is combined; washing is performed with saturated salt water (50 ml × 2 times); and drying is performed with anhydrous sodium sulfate, and then vacuum concentration is performed. Obtained residues are purified by silica gel column chromatography (eluent: ethyl acetate/n-hexane = 1/8). 2.85 g of yellow colorless oil 3-bromo-2-((4-methoxybenzyl)oxy)pyridine is obtained (yield: 97.3%). LCMS: RT = 2.18 min, [M+H]⁺ = 294.05.

At step B, 3-(3,6-dihydro-2H-pyran-4-yl)-2-((4-methoxybenzyl)oxy)pyridine is synthesized.

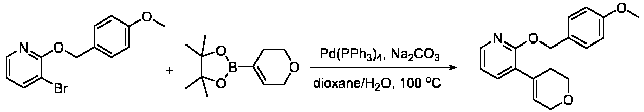

At room temperature, tetrakis(triphenylphosphine)palladium (115.6 mg, 0.1 mmol) and Na₂CO₃(270 mg, 2.5 mmol) are added to a mixed solvent solution (4.0 ml, 1,4-dioxane/water = 3/1) containing 3-bromo-2-((4-methoxybenzyl)oxy)pyridine (293.0 mg, 1.0 mmol) and 3,6-dihydro-2H-pyran-4-boronic acid pinacol ester (252.0 mg, 1.2 mmol), for reaction for 12h by warming up the temperature to 100 degrees Celsius under nitrogen protection.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then vacuum concentration is performed. Obtained residues are purified by silica gel column chromatography (eluent: ethyl acetate/n-hexane = 1/8). 194 mg of a yellow solid 3-(3,6-dihydro-2H-pyran-4-yl)-2-((4-methoxybenzyl)oxy)pyridine is obtained (yield: 65.3%). LCMS: RT = 2.14 min, [M+H]⁺ = 298.24.

At step C, 3-(tetrahydro-2H-pyran-4-yl)pyridine-2(1H)-one is synthesized.

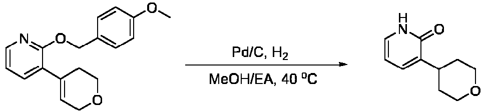

At room temperature, wet palladium carbon (125.8 mg, 0.065 mmol) is added to a mixed solvent solution (4.0 ml, methanol/ethyl acetate = 2/1) containing 3-(3,6-dihydro-2H-pyran-4-yl)-2-((4-methoxybenzyl)oxy)pyridine (194.0 mg, 0.65 mmol); and hydrogen replacement is performed for three times, for reaction for 12h by warming up the temperature to 40 degrees Celsius under hydrogen atmosphere.

After the reaction is completed, filter is performed, and then concentration is performed. The obtained yellowish solid 3-(tetrahydro-2H-pyran-4-yl)pyridine-2(1H)-one is directly used for a next step. LCMS: RT = 1.50 min, [M+H]⁺ = 180.15.

At step C, 3"-chloro-4"-((3,5-difluoropyridine-2-yl)methoxy)-5',6"-dimethyl-3-(tetrahydro-2H-pyran-4-yl)-2H,2"H-[1,2':4',1"-terpyridine]-2,2"-diketone is synthesized.

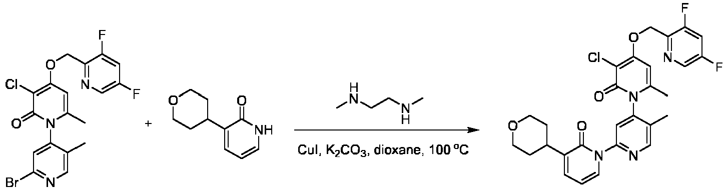

At room temperature, 2'-bromo-3-chloro-4-((3,5-difluoropyridine-2-yl)methoxy)-5',6-dimethyl-2H-[1,4'-bipyridyl]-2-one (137 mg, 0.30 mmol), 3-(tetrahydro-2H-pyran-4-yl)pyridine-2(1H)-one (109.2 mg, 0.61 mmol), Cul (12 mg, 0.06 mmol), and K₂CO₃ (84 mg, 0.61 mmol) are dissolved in the dioxane (4.0 ml); and dimethylethylenediamine (one drop) is added, for microwave reaction for 40 min at 80 degrees Celsius under nitrogen protection.

After the reaction is completed, filter is performed; concentration is performed; and purification is performed by silica gel column chromatography (eluent: ethyl acetate). 115.3 mg of a white solid 3"-chloro-4"-((3,5-difluoropyridine-2-yl)methoxy)-5',6"-dimethyl-3-(tetrahydro-2H-pyran-4-yl)-2H,2"H-[1,2':4',1"-terpyridine]-2,2"-diketone is obtained (yield: 69.4%). LCMS: RT = 1.86 min, [M+H]⁺ = 555.23.

### Embodiment 41

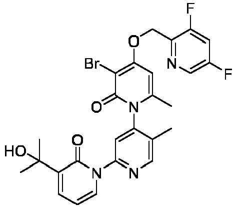

A synthetic route of a compound with a serial number being 41 is shown as follows.

At step A, 2',3-dibromo-4-((3,5-difluoropyridine-2-yl)methoxy)-5',6-dimethyl-2H-[1,4'-bipyridyl]-2-one is synthesized.

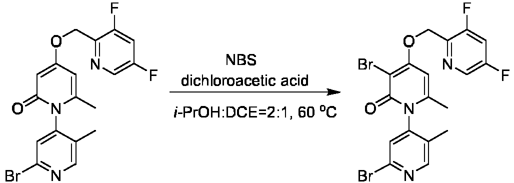

At room temperature, N-bromosuccinimide (77 mg, 0.65 mmol) and dichloroacetic acid (1 drop) are added to isopropanol (3.0 ml) containing 2'-bromo-4-((3,5-difluoropyridine-2-yl)methoxy)-5',6-dimethyl-2H-[1,4'-bipyridyl]-2-one (250 mg, 0.59 mmol), for reaction for 1h by warming up the temperature to 65 degrees Celsius.

After the reaction is completed, the temperature is cooled to room temperature; filter is performed; and decompression drying is performed, to obtain 230 mg of a white solid 2',3-dibromo-4-((3,5-difluoropyridine-2-yl)methoxy)-5',6-dimethyl-2H-[1,4'-bipyridyl]-2-one is obtained (yield: 77.2%). LCMS: RT = 1.93 min, [M+H]⁺ = 501.99.

At step B, 3"-bromo-4"-((3,5-difluoropyridine-2-yl)methoxy)-3-(2-hydroxypropane-2-yl)-5',6"-dimethyl-2H,2"H-[1,2':4',1"-terpyridine]-2,2"-diketone is synthesized.

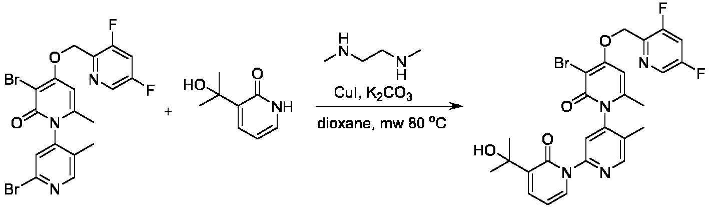

At room temperature, 1,4-dioxane (2.0 ml) containing 2',3-dibromo-4-((3,5-difluoropyridine-2-yl)methoxy)-5',6-dimethyl-2H-[1,4'-bipyridyl]-2-one (100 mg, 0.20 mmol), 3-(2-hydroxypropane-2-yl)-pyridine-2(1H)-one (37 mg, 0.24 mmol), K₂CO₃ (55 mg, 0.40 mmol), Cul (8 mg, 0.04 mmol) and dimethylethylenediamine (7 mg, 0.08 mmol) is degassed, for microwave reaction for 1h at 80 degrees Celsius under nitrogen protection.

After the reaction is completed, filter is performed; and concentration is performed. Purification is performed by silica gel column chromatography (eluent: ethyl acetate). 47 mg of a white solid 3"-bromo-4"-((3,5-difluoropyridine-2-yl)methoxy)-3-(2-hydroxypropane-2-yl)-5',6"-dimethyl-2H,2"H-[1,2':4',1"-terpyridine]-2,2"-diketone is obtained (yield: 41.2%). LCMS: RT = 1.87 min, [M+H]⁺ = 575.11.

### Embodiment 42

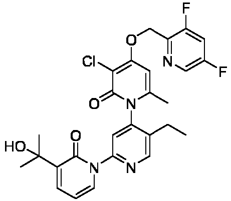

A synthetic route of a compound with a serial number being 42 is shown as follows.

At step A, 2-chloro-5-ethylpyridine-4-amine is synthesized.

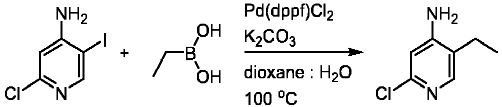

A mixed solution (20.0 ml, 1,4-dioxane:water =4:1) of 1,4-dioxane containing 2-chloro-5-iodo-4-pyridinamine (1.5 g, 5.9 mmol), ethylboronic acid (871.1 mg, 11.8 mmol), [1,1'-Bis(diphenylphosphino)ferrocene]dichloropalladium (431.2 mg, 0.6 mmol) and K₂CO₃ (1.6 g, 11.8 mmol) and water is warmed up to 100 degrees Celsius for reaction for 24h.

After the reaction is completed, drip washing is performed by adding water; extraction is performed with ethyl acetate (20 ml × 4 times); an organic phase is combined; washing is performed with saturated salt water (20 ml); and drying is performed with anhydrous sodium sulfate, and then vacuum concentration is performed. Obtained residues are purified by silica gel column chromatography (eluent: ethyl acetate/n-hexane = 1/5), to obtain 410.0 mg of a yellowish solid 2-chloro-5-ethylpyridine-4-amine (yield: 44.5%). LCMS: RT = 0.47 min, [M+H]⁺ = 157.09.

At step B, 2'-chloro-5'-ethyl-4-hydroxyl-6-methyl-2H-[1,4'-bipyridyl]-2-one is synthesized.

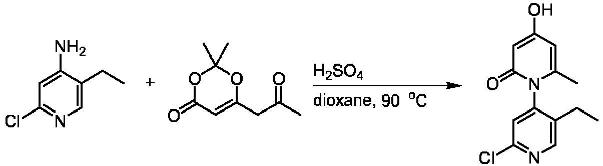

At room temperature, 2,2-dimethyl-6-(2-oxopropyl)-4H-1,3-dioxin-4-one (574.6 mg, 3.1 mmol) is added to 1,4-dioxane (10.0 ml) containing 2-chloro-5-ethylpyridine-4-amine (410.0 mg, 2.6 mmol), for reaction for 2h by warming up the temperature to 90degrees Celsius. Then, a sulfuric acid solution (1.5 ml, 10 mol/l) is added dropwise, to continue to react for 2h at 90degrees Celsius.

After the reaction is completed, the temperature is cooled to room temperature; a solid is precipitated by adding water, and filter is performed; drip washing is performed with water for 2 times; and then decompression drying is performed, to obtain 180.0 mg of a yellowish solid 2'-chloro-5'-ethyl-4-hydroxyl-6-methyl-2H-[1,4'-bipyridyl]-2-one is obtained (yield: 26.2%). LCMS: RT = 1.70 min, [M+H]⁺ = 265.13.

At step C, 2',3-dichloro-5'-ethyl-4-hydroxyl-6-methyl-2H-[1,4'-bipyridyl]-2-one is synthesized.

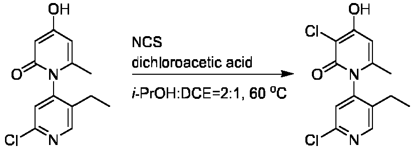

At room temperature, 1,2-dichloroethane (2.5 ml), NCS (100.1 mg, 0.7 mmol) and dichloroacetic acid (1 drop) are added to isopropanol (5.0 ml) containing 2'-chloro-5'-ethyl-4-hydroxyl-6-methyl-2H-[1,4'-bipyridyl]-2-one (180.0 mg, 0.7 mmol); and the mixed solution is warmed up to 65 degrees Celsius for reaction for 1h.

After the reaction is completed, the reaction is quenched with water; extraction is performed with ethyl acetate (20 ml × 4 times); an organic phase is combined; washing is performed with saturated salt water (10 ml); and drying is performed with anhydrous sodium sulfate, and then vacuum concentration is performed. Obtained residues are purified by silica gel column chromatography (eluent: ethyl acetate/n-hexane = 4/1), to obtain 230.0 mg of a yellowish solid (crude product) 2',3-dichloro-5'-ethyl-4-hydroxyl-6-methyl-2H-[1,4'-bipyridyl]-2-one. LCMS: RT = 1.74 min, [M+H]⁺ = 299.12.

At step D, 2',3-dichloro-5'-ethyl-4-((3,5-difluoropyridine-2-yl)methoxy)-6-methyl-2H-[1,4'-bipyridyl]-2-one is synthesized.

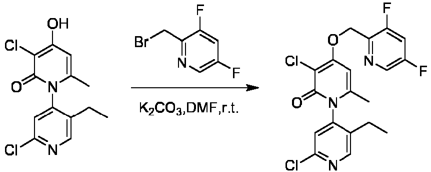

At room temperature, 2-(bromomethyl)-3,5-difluoropyridine (176.2 mg, 0.8 mmol) and K₂CO₃ (212.5 mg, 1.5 mmol) are added to an N,N-DMF solution (2.0 ml) containing 2',3-dichloro-5'-ethyl-4-hydroxyl-6-methyl-2H-[1,4'-bipyridyl]-2-one (230.0 mg, 0.8 mmol), for reaction for 1h at room temperature.

After the reaction is completed, the reaction is quenched with water; extraction is performed with ethyl acetate (20 ml × 3 times); an organic phase is combined; washing is performed with saturated salt water (10 ml); and drying is performed with anhydrous sodium sulfate, and then vacuum concentration is performed. Obtained residues are purified by silica gel column chromatography (eluent: ethyl acetate/n-hexane = 4/1), to obtain 91.0 mg of a yellowish solid 2',3-dichloro-5'-ethyl-4-((3,5-difluoropyridine-2-yl)methoxy)-6-methyl-2H-[1,4'-bipyridyl]-2-one (yield: 27.7%). LCMS: RT = 1.95 min, [M+H]⁺ = 426.12.

At step E, 3"-chloro-5'-ethyl-4"-((3,5-difluoropyridine-2-yl)methoxy)-3-(2-hydroxypropane-2-yl)-6"-methyl-2H,2"H-[1,2':4',1"-terpyridine]-2,2"-diketone is synthesized.

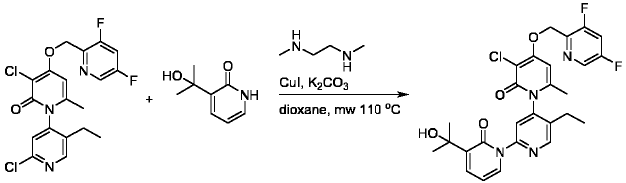

At room temperature, 1,4-dioxane (5.0 ml) containing 2',3-dichloro-5'-ethyl-4-((3,5-difluoropyridine-2-yl)methoxy)-6-methyl-2H-[1,4'-bipyridyl]-2-one (71.0 mg, 0.61 mmol), 3-(2-hydroxypropane-2-yl)-pyridine-2(1H)-one (38.3 mg, 0.25 mmol), K₂CO₃ (146.1 mg, 0.3 mmol), Cul (15.8 mg, 0.08 mmol) and dimethylethylenediamine (15 mg, 0.16 mmol) is degassed, for microwave reaction for 2h at 110 degrees Celsius under nitrogen protection.

After the reaction is completed, filter is performed; and concentration is performed. Preparation and separation are performed with a high performance liquid phase, to obtain 3.0 mg of a white solid 3"-chloro-5'-ethyl-4"-((3,5-difluoropyridine-2-yl)methoxy)-3-(2-hydroxypropane-2-yl)-6"-methyl-2H,2"H-[1,2':4',1"-terpyridine]-2,2"-diketone is obtained (yield: 3.3%). LCMS: RT = 1.91 min, [M+H]⁺ = 543.20.

### Embodiment 43

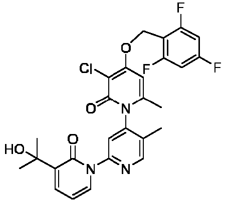

A synthetic route of a compound with a serial number being 43 is shown as follows.

At step A, 3"-chloro-4"-((2,4,6-trifluorophenyl)oxy)-3-(2-hydroxypropane-2-yl)-5',6"-dimethyl-2H,2"H-[1,2':4',1"-terpyridine]-2,2"-diketone is synthesized.

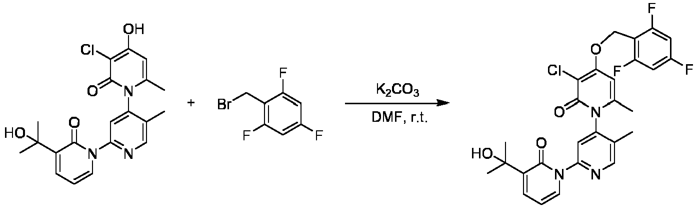

At room temperature, 2,4,6-trifluorobenzyl bromide (45 mg, 0.20 mmol) and K₂CO₃ (55 mg, 0.40 mmol) are added to N,N-DMF (3 ml) containing 3"-chloro-4"-((2,4,6-trifluorophenyl)oxy)-3-(2-hydroxypropane-2-yl)-5',6"-dimethyl-2H,2"H-[1,2':4',1"-terpyridine]-2,2"-diketone (80 mg, 0.20 mmol), for reaction for 1h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then vacuum concentration is performed. Obtained residues are purified by silica gel column chromatography (eluent: ethyl acetate). 21 mg of a white solid 3"-chloro-4"-((2,4,6-trifluorophenyl)oxy)-3-(2-hydroxypropane-2-yl)-5',6"-dimethyl-2H,2"H-[1,2':4',1"-terpyridine]-2,2"-diketone is obtained (yield: 25%). LCMS: RT = 1.97 min, [M+H]⁺ = 546.21.

### Embodiment 44

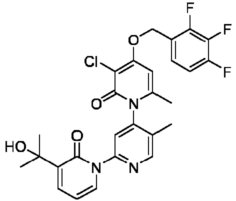

A synthetic route of a compound with a serial number being 44 is shown as follows.

At step A, 1-(bromomethyl)-2,3,4-trifluorobenzene is synthesized.

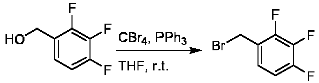

At zero degrees Celsius, PPh₃ (813.5 mg, 3.11 mmol) and CBr₄ (823.8 mg, 2.48 mmol) are added to THF (5.0 ml) containing the (2,3,4-trifluorophenyl)methanol (335.4 mg, 2.07 mmol), for reaction for 1h at room temperature.

After reaction is completed, vacuum concentration is performed. Obtained residues are purified by silica gel column chromatography (eluent: n-hexane/ethyl acetate = 10/1). 388.0 mg of colorless oil 1-(bromomethyl)-2,3,4-trifluorobenzene is obtained (yield: 73.2%). LCMS: RT = 2.11 min.

At step B, 3"-chloro-3-(2-hydroxypropane-2-yl)-5',6"-dimethyl-4"-((2,3,4-trifluorophenyl)oxy)-2H,2"H-[1,2':4',1"-terpyridine]-2,2"-diketone is synthesized.

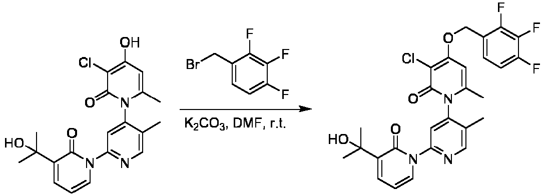

At room temperature, 1-(bromomethyl)-2,3,4-trifluorobenzene (40.2 mg, 0.18 mmol) and K₂CO₃ (33.12 mg, 0.24 mmol) are added to N,N-DMF (2.0 ml) containing 3"-chloro-4"-hydroxyl-3-(2-hydroxypropane-2-yl)-5',6"-dimethyl-2H,2"H-[1,2':4',1"-terpyridine]-2,2"-diketone (50.0 mg, 0.12 mmol), for reaction for 2h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then vacuum concentration is performed. Obtained residues are purified by silica gel column chromatography (eluent: ethyl acetate/n-hexane = 1/0). 35.2 mg of a white solid 3"-chloro-3-(2-hydroxypropane-2-yl)-5',6"-dimethyl-4"-((2,3,4-trifluorophenyl)oxy)-2H,2"H-[1,2':4',1"-terpyridine]-2,2"-diketone is obtained (yield: 53.7%). LCMS: RT = 1.98 min, [M+H]⁺ = 546.20.

### Embodiment 45

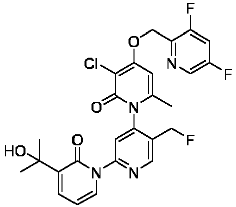

A synthetic route of a compound with a serial number being 45 is shown as follows.

At step A, 6-chloro-4-((4-methoxybenzyl)amino)methyl nicotinate is synthesized.

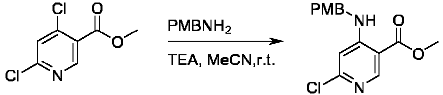

At room temperature, methyl 4,6-dichloronicotinate (7.0 g, 33.9 mmol), 4-methoxybenzylamine (5.6 g, 40.8 mmol) and triethylamine (4.1 g, 40.8 mmol) are dissolved in acetonitrile (60.0 ml), for reaction for 12h at room temperature.

After the reaction is completed, concentration is performed, and water is added; extraction is performed with ethyl acetate (20 ml × 4 times); an organic phase is combined; washing is performed with saturated salt water (20 ml); and drying is performed with anhydrous sodium sulfate, and then vacuum concentration is performed. Obtained residues are purified by silica gel column chromatography (eluent: ethyl acetate/n-hexane = 1/3), to obtain 9.3 g of a white solid 6-chloro-4-((4-methoxybenzyl)amino)methyl nicotinate (yield: 89.6%). LCMS: RT = 2.07 min, [M+H]⁺ = 307.13.

At step B, (6-chloro-4-((4-methoxybenzyl)amino)pyridin-3-yl)methanol is synthesized.

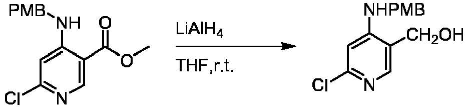

At room temperature, 6-chloro-4-((4-methoxybenzyl)amino)methyl nicotinate (9.3 g, 30.3 mmol) is dissolved in THF (90.0 ml); at zero degrees Celsius, lithium aluminum hydride (1.7 g, 45.5 mmol) is added in batches, for reaction for 30 min by warming up the temperature to room temperature.

After the reaction is completed, 1.7 ml of water and 1.7 ml of 15% sodium hydroxide aqueous solution are added under an ice bath; suction filtration; and concentration is performed to obtain 5.0 g of a white solid (6-chloro-4-((4-methoxybenzyl)amino)pyridin-3-yl)methanol (yield: 60.0%). LCMS: RT = 1.73 min, [M+H]⁺ = 1279.16.

At step C, 2-chloro-5-(fluoromethyl)-N-(4-methoxybenzyl)pyridin-4-amine is synthesized.

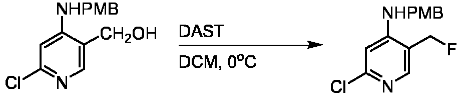

At room temperature, (6-chloro-4-((4-methoxybenzyl)amino)pyridin-3-yl)methanol (5.0 g, 18.0 mmol) is dissolved in dichloromethane (100.0 ml); the temperature is cooled below zero degrees Celsius; diethylaminosulfurtrifluoride (4.3 g, 27.0 mmol) is added dropwise; after the diethylaminosulfurtrifluoride is added, reaction is performed for 30 min at zero degrees Celsius.

After the reaction is completed, reactant is poured into a saturated sodium bicarbonate aqueous solution; extraction is performed with dichloromethane (30 ml × 3 times); an organic phase is combined; washing is performed with saturated salt water (20 ml); and drying is performed with anhydrous sodium sulfate, and then vacuum concentration is performed. Obtained residues are purified by silica gel column chromatography (eluent: dichloromethane), to obtain 930.0 mg of yellowish liquid 2-chloro-5-(fluoromethyl)-N-(4-methoxybenzyl)pyridin-4-amine (yield: 18.4%). LCMS: RT = 1.93 min, [M+H]⁺ = 281.17.

At step D, 2-chloro-5-(fluoromethyl)pyridin-4-amine is synthesized.

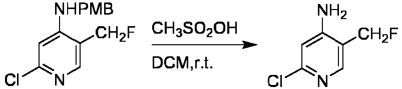

At room temperature, 2-chloro-5-(fluoromethyl)-N-(4-methoxybenzyl)pyridin-4-amine (930.0 mg, 3.3 mmol) is dissolved in dichloromethane (10.0 ml); the temperature is cooled below zero degrees Celsius; methanesulfonic acid (1.0 ml) is added dropwise; after the methanesulfonic acid is added, reaction is performed for 2h at room temperature.

After the reaction is completed, the reaction solution is slowly poured into the saturated sodium bicarbonate aqueous solution; extraction is performed with dichloromethane (10 ml × 3 times); an organic phase is combined; drying is performed with anhydrous sodium sulfate; and vacuum concentration is performed, to obtain 393.0 mg of a yellowish solid 2-chloro-5-(fluoromethyl)pyridin-4-amine (yield: 44.5%). LCMS: RT = 0.47 min, [M+H]⁺ = 161.08.

At step E, 2'-chloro-5'-(fluoromethyl)-4-hydroxyl-6-methyl-2H-[1,4'-bipyridyl]-2-one is synthesized.

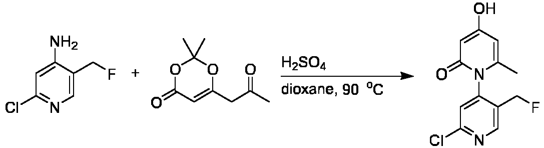

At room temperature, 2,2-dimethyl-6-(2-oxopropyl)-4H-1,3-dioxin-4-one (542.9 mg, 2.9 mmol) is added to 1,4-dioxane (10.0 ml) containing 2-chloro-5-(fluoromethyl)pyridin-4-amine (393.0 mg, 2.4 mmol), for reaction for 2h by warming up the temperature to 90degrees Celsius. Then, a sulfuric acid solution (1.0 ml, 10 mol/l) is added dropwise, to continue to react for 2h at 90degrees Celsius.

After the reaction is completed, the temperature is cooled to room temperature; a solid is precipitated by adding water, and filter is performed; drip washing is performed with water for 2 times; and then decompression drying is performed, to obtain 163.0 mg of a yellowish solid 2'-chloro-5'-(fluoromethyl)-4-hydroxyl-6-methyl-2H-[1,4'-bipyridyl]-2-one is obtained (yield: 25.3%). LCMS: RT = 1.68 min, [M+H]⁺ = 269.12.

At step F, 2',3-dichloro-5'-(fluoromethyl)-4-hydroxyl-6-methyl-2H-[1,4'-bipyridyl]-2-one is synthesized.

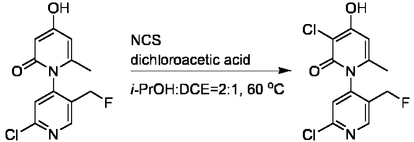

At room temperature, 1,2-dichloroethane (2.5 ml), NCS (78.4 mg, 0.6 mmol) and dichloroacetic acid (1 drop) are added to isopropanol (5.0 ml) containing 2'-chloro-5'-(fluoromethyl)-4-hydroxyl-6-methyl-2H-[1,4'-bipyridyl]-2-one (143.0 mg, 0.5 mmol), for reaction for 1h by warming up the temperature to 65 degrees Celsius.

After the reaction is completed, the reaction is quenched with water; extraction is performed with ethyl acetate (10 ml × 4 times); an organic phase is combined; washing is performed with saturated salt water (10 ml); and drying is performed with anhydrous sodium sulfate, and then vacuum concentration is performed. Obtained residues are purified by silica gel column chromatography (eluent: ethyl acetate/n-hexane = 4/1), to obtain 23.0 mg of a yellowish solid 2',3-dichloro-5'-(fluoromethyl)-4-hydroxyl-6-methyl-2H-[1,4'-bipyridyl]-2-one (yield: 15.2%). LCMS: RT = 1.68 min, [M+H]⁺ = 303.07.

At step G, 2',3-dichloro-4-((3,5-difluoropyridine-2-yl)methoxy)-5'-(fluoromethyl)-6-methyl-2H-[1,4'-bipyridyl]-2-one is synthesized.

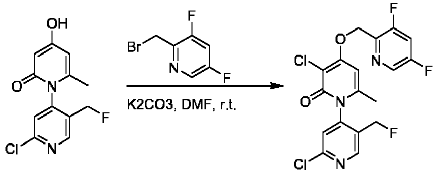

At room temperature, 2-(bromomethyl)-3,5-difluoropyridine (19.0 mg, 0.09 mmol) and K₂CO₃ (20.9 mg, 0.15 mmol) are added to an N,N-DMF solution (5.0 ml) containing 2',3-dichloro-5'-(fluoromethyl)-4-hydroxyl-6-methyl-2H-[1,4'-bipyridyl]-2-one (23.0 mg, 0.07 mmol), for reaction for 1h at room temperature.

After the reaction is completed, the reaction is quenched with water; extraction is performed with ethyl acetate (10 ml × 3 times); an organic phase is combined; washing is performed with saturated salt water (10 ml); and drying is performed with anhydrous sodium sulfate, and then vacuum concentration is performed. Obtained residues are purified by silica gel column chromatography (eluent: ethyl acetate/n-hexane = 6/1), to obtain 15.0 mg of a yellow solid 2',3-dichloro-4-((3,5-difluoropyridine-2-yl)methoxy)-5'-(fluoromethyl)-6-methyl-2H-[1,4'-bipyridyl]-2-one (yield: 49.9%). LCMS: RT = 1.91 min, [M+H]⁺ = 430.09.

At step H, 3"-chloro-4"-((3,5-difluoropyridine-2-yl)methoxy)-5'-(fluoromethyl)-3-(2-hydroxypropane-2-yl)-6"-methyl-2H,2"H-[1,2':4',1"-terpyridine]-2,2"-diketone is synthesized.

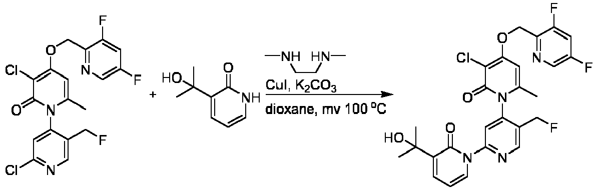

At room temperature, 1,4-dioxane (5.0 ml) containing 2',3-dichloro-4-((3,5-difluoropyridine-2-yl)methoxy)-5'-(fluoromethyl)-6-methyl-2H-[1,4'-bipyridyl]-2-one (15.0 mg, 0.035 mmol), 3-(2-hydroxypropane-2-yl)-pyridine-2(1H)-one (8.0 mg, 0.052 mmol), K₂CO₃ (9.6 mg, 0.07 mmol), Cul (3.3 mg, 0.0175 mmol) and dimethylethylenediamine (15 mg, 0.16 mmol) is degassed, for microwave reaction for 1h at 100 degrees Celsius under nitrogen protection.

After the reaction is completed, filter is performed; and concentration is performed. Preparation and separation are performed with a high performance liquid phase, to obtain 0.5 mg of a white solid 3"-chloro-4"-((3,5-difluoropyridine-2-yl)methoxy)-5'-(fluoromethyl)-3-(2-hydroxypropane-2-yl)-6"-methyl-2H,2"H-[1,2':4',1"-terpyridine]-2,2"-diketone is obtained (yield: 2.6%). LCMS: RT = 1.86 min, [M+H]⁺ = 547.20.

### Embodiment 46

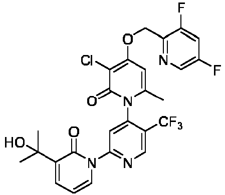

A synthetic route of a compound with a serial number being 46 is shown as follows.

At step A, 2'-chloro-4-hydroxyl-6-methyl-5'-(trifluoromethyl)-2H-[1,4'-bipyridyl]-2-one is synthesized.

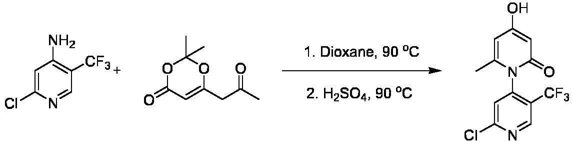

At room temperature, 2-chloro-5-trifluoromethylpyridine-4-amine (1.55 g, 7.6 mmol) is dissolved into dioxane (20 ml), 2,2-dimethyl-6-(2-oxopropyl)-4H-1,3-dioxin-4-one (1.4 g, 7.6 mmol) is further added, for reaction for 5h by warming up the temperature to 90 degrees Celsius; and then, 10N of a sulfuric acid aqueous solution is further added dropwise until no solid is produced, and the reaction is continued for 2h.

After the reaction is complete, concentration is performed; a yellowish precipitate is precipitated by adding water; filtering is performed after 0.5h of stirring, and drip washing is performed with water for two times; and 1 g of an earth-yellow solid 2'-chloro-4-hydroxyl-6-methyl-5'-(trifluoromethyl)-2H-[1,4'-bipyridyl]-2-one is obtained (yield: 43%). LCMS: RT = 1.75 min, [M+H]⁺ = 305.04.

At step C, 2',3-dichloro-4-hydroxyl-6-methyl-5'-(trifluoromethyl)-2H-[1,4'-bipyridyl]-2-one is synthesized.

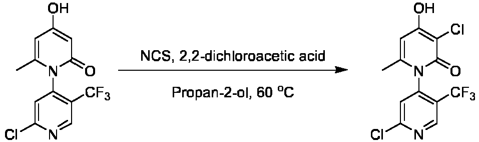

At room temperature, 2'-chloro-4-hydroxyl-6-methyl-5'-(trifluoromethyl)-2H-[1,4'-bipyridyl]-2-one (1 g, 3.28 mmol) is dissolved in isopropanol (30 ml); NCS (540 mg, 3.28 mmol) is further added; and then dichloroacetic acid (1 drop) is added, for reaction for 6h by warming up the temperature to 60 degrees Celsius.

After the reaction is completed, the reaction is quenched with water; extraction is then performed with ethyl acetate; an organic phase is combined; drying is performed with anhydrous sodium sulfate; filter is performed, and then concentration is performed; and purification (ethyl acetate) is performed through column chromatography to obtain 900 mg of a white solid 2',3-dichloro-4-hydroxyl-6-methyl-5'-(trifluoromethyl)-2H-[1,4'-bipyridyl]-2-one (yield: 82%). LCMS: RT = 1.78 min, [M+H]⁺ = 338.99.

At step B, 2',3-dichloro-4-((3,5-difluoropyridine-2-yl)methoxy)-6-methyl-5'-(trifluoromethyl)-2H-[1,4'-bipyridyl]-2-one is synthesized.

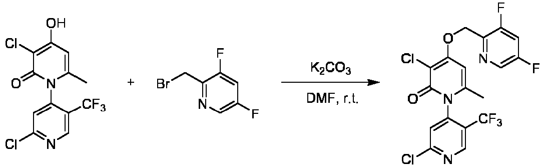

At room temperature, 2-(bromomethyl)-3,5-difluoropyridine (185 mg, 0.89 mmol) and K₂CO₃ (246 mg, 1.78 mmol) are added to N,N-DMF solution (3 ml) containing 2',3-dichloro-4-hydroxyl-6-methyl-5'-(trifluoromethyl)-2H-[1,4'-bipyridyl]-2-one (300 mg, 0.89 mmol), for reaction for 1h at room temperature.

After the reaction is completed, the reaction is quenched with water; extraction is performed with ethyl acetate (20 ml × 3 times); an organic phase is combined; washing is performed with saturated salt water (20 ml × 2 times); and drying is performed with anhydrous sodium sulfate, and then vacuum concentration is performed. Purification is performed by silica gel column chromatography (eluent: ethyl acetate). 150 mg of a white solid 2',3-dichloro-4-((3,5-difluoropyridine-2-yl)methoxy)-6-methyl-5'-(trifluoromethyl)-2H-[1,4'-bipyridyl]-2-one is obtained (yield: 36%). LCMS: RT = 1.98 min, [M+H]⁺ = 466.04.

At step E, 3"-chloro-4"-((3,5-difluoropyridine-2-yl)methoxy)-3-(2-hydroxypropane-2-yl)-6"-methyl-5'-(trifluoromethyl)-2H,2"H-[1,2':4',1"-terpyridine]-2,2"-diketone is synthesized.

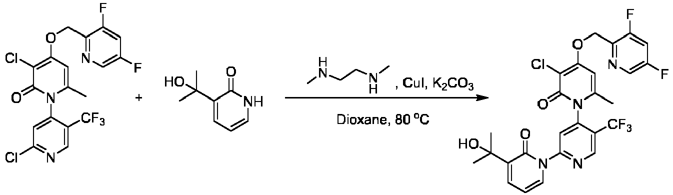

At room temperature, 2',3-dichloro-4-((3,5-difluoropyridine-2-yl)methoxy)-6-methyl-5'-(trifluoromethyl)-2H-[1,4'-bipyridyl]-2-one (50 mg, 0.11 mmol) Cul (6 mg, 0.02 mmol) and K₂CO₃ (61 mg, 0.22 mmol) are dissolved in dioxane (4 ml), and dimethylethylenediamine (one drop) is further added, for microwave reaction for 1h at 80 degrees Celsius.

After the reaction is completed, filter is performed; drip washing is performed with ethyl acetate; concentration is performed; and purification is performed through column chromatography, to obtain 1 mg of a white solid 3"-chloro-4"-((3,5-difluoropyridine-2-yl)methoxy)-3-(2-hydroxypropane-2-yl)-6"-methyl-5'-(trifluoromethyl)-2H,2"H-[1,2':4',1"-terpyridine]-2,2"-diketone. LCMS: RT = 1.97 min, [M+H]⁺ = 583.20.

### Comparative example 1

A structure of the compound 3-chloro-4-((3,5-difluoropyridine-2-yl)methoxy)-2'-(2-(2-hydroxypropane-2-yl)pyrimidin-4-yl-5',6-dimethyl-2H-[1,4'-bipyridyl]-2-one is shown as follows.

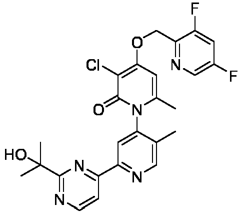

For the synthetic route of the compound in Comparative example 1, refer to a synthetic route of a compound of which Chinese Patent Application No. is CN201480032278.5 and specification serial number is 49.

Supercritical fluid chromatography (OD-H column) is used to separate a racemic Comparative example 1 compound by means of a mobile phase of carbon dioxide and ethanol, and atropisomer Comparative example 1A and 1B compounds are eluted successively.

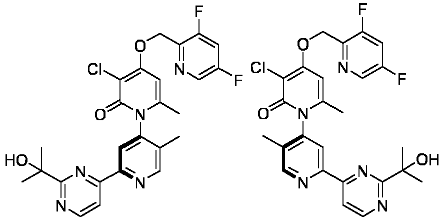

### (-) Comparative example 1A (+) Comparative example 1B

Comparative example 1A compound: RT = 4.47 min (SFC, OD-H, 0.46 cm I.D. × 15 cm L column, isogradient method with 40% ethanol, flow rate being 2.5 mL/min, and cycling time being 10 min); and [a]_{D}²⁵-0.66°(MeOH, Rudolph Autopol I specific rotation instrument).

Comparative example 1B compound: RT = 4.68 min (SFC, OD-H, 0.46 cm I.D. × 15 cm L column, isogradient method with 40% ethanol, flow rate being 2.5 mL/min, and cycling time being 10 min). [a]_{D}²⁵+0.68°(MeOH, Rudolph Autopol I specific rotation instrument).

### Embodiment 47: an experiment of releasing TNFα from U937 induced by LPS

Cytokine regulation in human monocytes has shown that the p38 pathway is the key to the biosynthesis of a plurality of pro-inflammatory cytokines including TNFα, IL-1β and IL-6. Therefore, the inhibition of the p38 MAPK pathway is to reduce inflammatory response by reducing the biosynthesis of the pro-inflammatory cytokines. This research shows half amount of the compound in the present invention required to inhibit the biosynthesis of TNFα (pro-inflammatory cytokine). This is a reflection of the effect of the compound in the present invention to facilitate reduction of inflammation, which is useful in the treatment of various diseases, including chronic inflammatory diseases, acute inflammatory diseases, or self-inflammatory diseases. The potency and efficacy of the p38 inhibitor in blocking cytokine production are evaluated using a human U937 cell line.

### Reagents and instruments:

A 1640 medium with an article number being A10491-01, Gibco; penicillin-streptomycin with an article number being 15140-122, Gibco; fetal bovine serum with an article number being 10099-141C, Gibco; PBS with an article number being 10010-031, Gibco; LPS with an article number being L2880, Sigma; PMA with an article number being P1585, Sigma; dimethyl sulfoxide with an article number being D8418-1L, Sigma; and a TNFα kit with an article number being K151QWD-4, MSD.

A 96-well plate with an article number being 3599, Corning; a plate-vibrating device with an article number being QB-9002, QILINBEIER; a centrifuge with an article number being 5810R, Eppendorf; a carbon dioxide incubator with an article number being 371, Thermo; a counter with an article number being C10281, Gibco; a microscope with an article number being CKX41, OLYMPUS; and an MSD plate reader with an article number being 1201MESO SECTOR 600, MSD.

### Experimental cells:

U937, ATCC with an article number being CRL-1593.2.

### Drug preparation:

About 2 mg of a drug is weighted; a mother solution is prepared to 10 mM (which is calculated by free alkali) by means of DMSO; and the mother solution is diluted 10-fold to 1 mM, and then is successively diluted 4-fold to 250 µM, 62.5 µM, 15.6 µM, 3.9 µM, 0.97 µM, 0.24 µM, and 0.061 µM. The above series of diluted DMSO drugs is diluted 20-fold as working solutions by using media.

### Experimental method:

At Day 0, 10000/well inoculation is performed, stimulation is performed for 48h with 20 ng/ml of PMA, and culture is performed at 37°C, 5%CO₂.

At Day 2, 1. Supernatant is removed from differentiated U937, cleaning is performed once with PBS, and 96 µl of the 1640 medium is added.

2. 2 µl of the compound containing (DMSO with a final concentration being 0.1%), and culture is performed for 30 min at 37°C, 5%CO₂.

3. 2µl of LPS (a final concentration being 100ng/ml) is added, cells are stimulated, and culture is performed for 4h at 37°C, 5%CO₂.

4. Centrifugation is performed, supernatant is taken, and content of TNFα in the supernatant is determined through ELISA.

### Statistical method:

The content of TNFα corresponding to each well is calculated by using a standard curve in the kit.

The compound IC₅₀ is calculated by using a GraphPad nonlinear fitting formula, and for an experimental result, refer to Table 1.

**Table 1 IC₅₀ value that the compound of the present invention inhibits the production of TNFα**

| Embodiment compound serial number | IC₅₀ (nM) |
|---|---|
| 1 | 1.63 |
| 3 | 14.7 |
| 4 | 2.05 |
| 5 | 4.05 |
| 8 | 14.43 |
| 9 | 2.72 |
| 12 | 5.09 |
| 13 | 4.26 |
| 14 | 8.21 |
| 15 | 1.60 |
| 16 | 3.31 |
| 17 | 1.73 |
| 18 | 2.39 |
| 19 | 8.11 |
| 20 | 6.86 |
| 21 | 7.11 |
| 23 | 5.53 |
| 24 | 11.15 |
| 25 | 6.06 |
| 26 | 2.84 |
| 27 | 16.58 |
| 29 | 15.11 |
| 30 | 11.86 |
| 35 | 24.32 |
| Comparative example 1 | 10.4 (n = 3) |

From the experimental results of Table 1, it may be learned that, the compound of the present invention has an obvious inhibitory activity on the production of TNFα, so that related diseases such as inflammatory response may be regulated.

### Embodiment 48: Determination of solubility.

### 1. Preparation of a control solution

About 0.5 mg of a to-be-tested sample in a centrifuge tube is weighted, an appropriate amount of DMSO is added to completely dissolve the sample, and then methanol is added to make up to 1 ml. The to-be-tested sample is the compound with the serial number being 1 and the compound in Comparative example 1.

### 2. Preparation of a test solution

About 1.0 mg of each of the compound in Comparative example 1 and the compound with the serial number being 1 is weighed into two centrifuge tubes, and 1 ml of buffer solutions of PBS=2.0 and 7.4 are added to the two tubes, respectively. (If there are more pH values to be investigated, the preparation method may be deduced by analogy).

3. The prepared control solution and test solution are simultaneously put into a 37°C water bath and heated for 1h, after 1h, the solutions are taken out and cooled to room temperature, and then the solutions are filtered with a 0.22 µm filter membrane for sample injection.

4. The concentration of the sample in the test solution is calculated according to C=(A*(ms/vs))/AS, and experiment results are shown in Table 2.

Note: ms, vs and AS are the weight, volume and peak area of the sample in the control solution, respectively.

A is the peak area of the test solution.

**Table 2 Solubility of the compound of the present invention**

| Embodiment compound serial number | pH = 2.0 | pH = 7.4 |
|---|---|---|
| 1 | 427.1 µg/mL | 595.1 µg/mL |
| 2 | 192 µg/mL | 234.5 µg/mL |
| 3 | 1285.5 µg/mL | 140.2 µg/mL |
| 20 | 768.9 µg/mL | 772.7 µg/mL |
| Comparative example 1 | 56.8 µg/mL | 26.8 µg/mL |

From the experiment results of Table 2, it may be learned that, compounds with serial numbers being 1, 2, 3 and 20 in this application have desirable solubility, and are superior to those of the compound in Comparative example 1.

### Embodiment 49: Pharmacokinetic experiment

### 1. Reagents and instruments

Polyethylene glycol 400 (batch number: GORKREUT, Saen Chemical Technology (Shanghai) Co., Ltd.); DMSO (batch number: 20200319, GHTECH (Guangdong) Co., Ltd.); and normal saline (batch number: C20052604, Jiangxi Kelun Pharmaceutical Co., Ltd.). An LC-MS instrument (Thermo Fisher Ultimate 3000 UPLC, TSQ QUANTUM ULTRA TSQ Quantum GC).

### 2. Experimental animals

5 male Beagle dogs with weights being 5 kg to 7 kg, purchased from Beijing Mars Biotechnology Co., Ltd.

### 3. Preparations

Test powder is accurately weighted, and is completely dissolved with DMSO, then PEG-400 is added; vortex and ultrasonic mixing are performed; then the normal saline is added for vortex and ultrasonic mixing to make the mixture to 0.5 mg/mL (DMSO:PEG-400:NS=5:60:35, V/V/V); and 5 mL/kg is given through intragastric administration, and 1 mL/kg is given through intravenous administration.

### 4. Blood sample collection

After the above mixture is given to the dogs through intragastric administration (n=3) or intravenous administration (n=2), about 1 mL of blood is collected from the forelimb or hindlimb vein with a scalp needle, respectively, at 5 min (intravenous administration only), 15 min, 30 min, 1 h, 2 h, 4 h, 6 h, 8 h, 24 h to put into blood collection tubes containing EDTA-K2 anticoagulant; centrifugation is performed for 10 min at 4000 rpm to separate plasma; and then the tubes are stored at -80°C for testing.

### 5. Bioanalysis

A certain amount of a test sample is accurately weighted and dissolved with DMSO to 2 mg/mL, as a stock solution. The stock solution is diluted to 30000, 10000, 3000, 1000, 300, 100, 50, 30, and 10 ng/mL by using acetonitrile:water (1:1), to obtain standard curve working solutions. 5 µL of the working solution is added to 45 µL of blank canine plasma; and well mixing is performed through vortex, to prepare into standard curve samples equivalent to plasma concentrations of 3000, 1000, 300, 100, 30, 10, 5, 3, and 1 ng/mL. 30 µL of the standard curve sample and the collected plasma sample (plasma collected at 5min, 15min, and 30min of intravenous administration is diluted 5-fold) are added to 150 µL of a propranolol acetonitrile solution (internal standard, 50 ng/mL) to precipitate protein, then 100 µL of water is added to well mix through vortex; centrifugation is performed for 5 min at 4000 rpm; and supernatant is taken for LC-MS analysis. LC-MS detection conditions include the following.

Chromatographic column: Waters ACQUITYTM PREMIER HSS T3, 50*2.1mm, 1.8µm.

Mobile phase A: water (0.1% formic acid); mobile phase B: acetonitrile; flow rate: 0.5 mL/min; and gradient elution is shown in Table 3 below.

**Table 3**

| Time (min) | A(%) | B(%) |
|---|---|---|
| 0 | 95% | 5% |
| 1 | 10% | 90% |
| 2.5 | 5% | 95% |
| 2.51 | 95% | 5% |
| 3.0 | 95% | 5% |

### 6. Data Processing

After LC-MS detection of blood drug concentration, and a non-compartment model of WinNonlin 6.1 software is adopted to calculate pharmacokinetic parameters of beagle dogs after administration. Results are shown in Table 4 below.

**Table 4 Pharmacokinetic parameters in dogs of the compound of the present invention (IV and PO administration)**

| Compound serial number | Administration method and dosage (mg/kg) | Cₘₐₓ (ng/ml) | AUCₗₐₛₜ(h*ng/ ml) | Cl (ml/min/kg) | F% |
|---|---|---|---|---|---|
| Comparativ e example 1 | IV 1 | 657 | 1530 | 10.7 | 188.7 |
| | PO 5 | 2000 | 14400 | | |
| 1 | IV 1 | 1510 | 2870 | 6.15 | 229.3 |
| | PO 5 | 5550 | 32900 | | |

From the experiment results of Table 4, it may be learned that, compared with the compound of Comparative example 1, the canine PK of the compound of the present invention with the serial number being 1 has higher oral exposure and bioavailability.

### Embodiment 50: p38α/MK2 complex kinase screening experiment

**Table 5 Reagent:**

| Material | Supplier | Cat# |
|---|---|---|
| dithiothreitol (DTT) | aladdin | D104860-1G |
| ATP | Sigma-Aldrich | A7699 |
| MAPKAPK2(MK2), Active | SignalChem | M40-11G |
| MAPKAPK2(MK2), Unactive | SignalChem | M40-14G |
| p38 alpha, Unactive | SignalChem | M39-14G |
| MEK6(MKK6), Active | SignalChem | M07-10G |
| Fluorescently labeled HSP27 peptide | GenScript | / |
| IMAP FP IPP Explorer Kit | Molecular Devices | R8124 |
| SD 0006 | MCE | HY-11087 |
| MK2-IN-3 | MCE | HY-131249 |
| 384-well Plate | Corning | REF 3575 |

**Table 6 Instrument:**

| Device name | Device serial number | Brand | Model |
|---|---|---|---|
| Multifunctional microwell detection plate system | MEO-18-02 | German BMG | PHERASTER FSX |
| Shaker | MEH-02-02 | Kylin Bell | QB-9002 |
| Vortex oscillator | MEH-12-11 | IKA | VORTEX2 S025 |
| Centrifuge | MEC-01-08 | eppendorf | 5424R |
| Ultrapure water machine | MEO-03-02 | Thermo | GenPure UV/UF |
| Centrifuge | MEC-01-03 | eppendorf | 5810R |

### Drug preparation:

A certain amount of a drug is weighted and prepared into a 10 mM of mother solution by using DMSO; the mother solution is diluted to 100 µM (100X working solution) in two steps; and then, the solution is diluted 3-fold to 33.33 µM, 11.11 µM, 3.70 µM, 1.23 µM, 0.41 µM, 0.14 µM, 0.046 µM, 0.015 µM, and 0.005 µM, successively. DMSO control is also arranged.

Then, a series of diluted DMSO drugs are diluted 25 folds with 1X IMAP Reaction Buffer as a 4X drug working solution.

### Experimental method:

a), the 1X IMAP Reaction Buffer is used to prepare an enzyme (MEK6, active), p38α (unactive), MK2(unactive), a substrate (HSP27) and ATP.
b), 10 µL of a 2X enzyme &substrate working solution and 5 µL of a 4X drug working solution are successively added in a black 384-well plate; and after rapid centrifugation, 5 µL of an ATP working solution is added to start enzymatic reaction. 10 gradient concentrations and 2 duplicated wells are set to each group of compounds; and a DMSO control group and a negative control group (DMSO, no ATP) are additionally set. The final concentration of each component is shown in Table 7 below.

**Table7**

| | |
|---|---|
| p38α (nM) | 0.2 |
| MK2, unactive (nM) | 1 |
| HSP27 substrate (µM) | 1 |
| ATP (µM) | 10 |

c), after 1h of incubation at room temperature, 1X Progressive Binding Solution is added to end the enzymatic reaction; after 30 min of incubation, a BMGPHERASTER FSX multifunctional microplate reader is used to detect a Fluorescence Polarization (FP) signal [FP(Ex485/ Em520/Em520nm)].

### Statistical method:

A GraphPad Prism 7 nonlinear fitting formula is used to calculate the compound IC₅₀, and results are shown in Table 8.

**Table 8: IC₅₀ value that the compound of the present invention inhibits p38α/MK2 complex**

| Embodiment compound | IC₅₀ (nM) |
|---|---|
| 1 | 2.60 |
| 1A | 0.53 |
| 16 | 3.35 |
| 17 | 1.33 |
| 26 | 2.26 |
| 40 | 2.85 |
| 41 | 1.58 |
| 42 | 3.14 |
| Comparative example 1 | 8.33 |
| Comparative example 1A | 2.41 |

From the experimental results of Table 8, it may be learned that, the compound of the present invention has an obvious inhibitory activity on p38α/MK2 complex, so that related diseases such as inflammatory response may be regulated.

### Embodiment 51: p38α kinase screening experiment

**Table 9 Reagent:**

| Reagent | Supplier | Article number |
|---|---|---|
| ADP-Glo Kinase Assay | Promega | V9102 |
| MAPK14 (p38 α) | SignalChem | M39-10BG |
| MAPK11 (p38 β) | SignalChem | M39-10BG |
| p38 Substrate | SignalChem | P03-58 |
| ATP | Promega | V910B |
| DMSO | Sigma | D8418 |

**Table 10 Instrument:**

| Consumables and instruments | Supplier | Article number or model |
|---|---|---|
| 384-well plate, white, low volume, round-bottom | Greiner | 784075 |
| 384-Well Polypropylene microplate, Clear, FlatBottom,Bar Code | Labcyte | P-05525-BC |
| 96-well polypropylene plate | Nunc | 249944 |
| Plate shaker | Thermo | 4625-1 CECN/THZ Q |
| Centrifuge | Eppendorf | 5810R |
| Envision 2104 multi-label Reader | PerkinElmer | 44473 |
| Echo | Labcyte | 655 |

### Drug preparation:

A certain amount of the compound is weighed, prepared into a 10/50 mM mother solution with DMSO, diluted 3 folds with DMSO, and diluted to 10 concentration points as a compound working solution.

### Experimental method:

50 nL of the diluted compound working solution is transferred to each well of a reaction plate (784075, Greiner) with Echo 655, then 2.5 µL (4 ng/µL) of a p38α kinase solution is added; after 10 minutes at room temperature, 2.5 µL of a mixed solution of a kinase substrate (0.2 mg/mL) and ATP (150 µM) is added, for reaction for 60 min at room temperature; and 4 µL of an ADP Glo reagent is added, for incubation for 40 min at room temperature. 8 µL of a kinase detection reagent is added, for incubation for 40 min at room temperature; and finally a light-emitting signal is read on Envision 2104.

### Data analysis

A GraphPad Prism 8 nonlinear fitting formula is used to calculate the compound IC₅₀, and test results are shown in Table 11.

**Table 11: IC₅₀ value that the compound of the present invention inhibits p38α**

| Embodiment compound | IC₅₀ (nM) | Activity multiple relative to p38α/(p38α/MK2 complex) |
|---|---|---|
| 1 | 577.9 | 222 |
| Comparative example 1 | 853.8 | 103 |

From the experiment results of Table 11, it may be learned that, compared with the prior art, the compound of the present invention has higher activity multiple relative to p38α/(p38α/MK2 complex) and better selectivity.

Note: p38α/MK2 complex kinase data is from Embodiment 50.

### Embodiment 52: p38β kinase screening experiment

**Table 12 Reagent:**

| Reagent | Supplier | Article number |
|---|---|---|
| ADP-Glo Kinase Assay | Promega | V9102 |
| MAPK14 (p38 α) | SignalChem | M39-10BG |
| MAPK11 (p38 β) | SignalChem | M39-10BG |
| p38 Substrate | SignalChem | P03-58 |
| ATP | Promega | V910B |
| DMSO | Sigma | D8418 |

**Table 13 Instrument:**

| Consumables and instruments | Supplier | Article number or model |
|---|---|---|
| 384-well plate, white, low volume, round-bottom | Greiner | 784075 |
| 384-Well Polypropylene microplate, Clear, FlatBottom,Bar Code | Labcyte | P-05525-BC |
| 96-well polypropylene plate | Nunc | 249944 |
| Plate shaker | Thermo | 4625-1 CECN/THZ Q |
| Centrifuge | Eppendorf | 5810R |
| Envision 2104 multi-label Reader | PerkinElmer | 44473 |
| Echo | Labcyte | 655 |

### Drug preparation:

A certain amount of the compound is weighed, prepared into a 10/50 mM mother solution with DMSO, diluted 3 folds with DMSO, and diluted to 10 concentration points as a compound working solution.

### Experimental method:

50 nL of the diluted compound working solution is transferred to each well of a reaction plate (784075, Greiner) with Echo 655, then 2.5 µL (2 ng/µL) of a p38β kinase solution is added; after 10 minutes at room temperature, 2.5 µL of a mixed solution of a kinase substrate (0.4 mg/mL) and ATP (100 µM) is added, for reaction for 60 min at room temperature; and 4 µL of an ADP Glo reagent is added, for incubation for 40 min at room temperature. 8 µL of a kinase detection reagent is added, for incubation for 40 min at room temperature; and finally a light-emitting signal is read on Envision 2104.

### Data analysis

A GraphPad Prism 8 nonlinear fitting formula is used to calculate the compound IC₅₀, and test results are shown in Table 14.

**Table 14: IC₅₀ value that the compound of the present invention inhibits p38β**

| Embodiment compound | IC₅₀ (nM) | Activity multiple relative to p38β/(p38α/MK2 complex) |
|---|---|---|
| 1 | 4095 | 1575 |
| Comparative example 1 | 5972 | 720 |

From the experiment results of Table 14, it may be learned that, compared with the prior art, the compound of the present invention has higher activity multiple relative to p38β/(p38α/MK2 complex) and better selectivity.

Note: p38α/MK2 complex kinase data is from Embodiment 50.

## Claims

1. A compound shown in a general formula (I), or a racemate, or a geometric or stereoisomeric isomer, or a pharmaceutically acceptable salt thereof, wherein 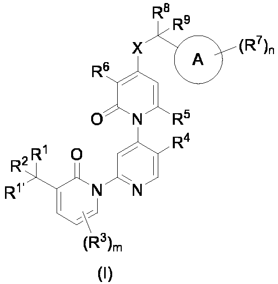
R¹ and R^{1'} are independently selected from hydrogen, halogen, alkyl, cycloalkyl, heterocycloalkyl, alkylcycloalkyl, alkyl heterocycloalkyl, phenyl, heteroaryl, haloalkyl, or cyano, or R¹ and R^{1'} are cyclized to the cycloalkyl or the heterocycloalkyl together;
R² is selected from hydrogen, halogen, hydroxyl, cyano, alkyl, alkoxy, alkoxyalkyl, or haloalkyl;
R³ is independently selected from hydrogen, alkyl, cycloalkyl, heterocycloalkyl, halogen, alkoxy, cyano, haloalkoxy, or sulfonyl;
m is 0, 1, 2, or 3;
R⁴ is selected from hydrogen, alkyl, cycloalkyl, alkoxy, or haloalkyl;
R⁵ is selected from hydrogen, alkyl, cycloalkyl, alkoxy, or haloalkyl;
R⁶ is selected from hydrogen, halogen, cyano, alkyl, cycloalkyl, or haloalkyl;
an A ring is selected from an aromatic ring or a heteroaromatic ring;
R⁷ is independently selected from hydrogen, halogen, cyano, alkyl, cycloalkyl, haloalkyl, alkoxy, haloalkoxy, or sulfonyl;
n is 0, 1, 2, 3, 4, or 5;
X is O, CH₂ or NH; and
R⁸ and R⁹ are independently selected from hydrogen, alkyl, cycloalkyl, alkylcycloalkyl, or haloalkyl, or R⁸ and R⁹ are cyclized to the cycloalkyl or the heterocycloalkyl together.

2. The compound as claimed in claim 1, or a racemate, or a geometric or stereoisomeric isomer, or a pharmaceutically acceptable salt thereof, wherein
the alkyl is selected from alkyl of C₁₋₆, and the alkyl of C₁₋₆ is selected from methyl, ethyl, propyl, isopropyl, n-butyl, isobutyl, sec-butyl, tert-butyl, n-pentyl, sec-pentyl, 1-ethylpropyl, 2-methylbutyl , tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, or 1-ethylbutyl;
alkoxy is selected from alkoxy of C₁₋₆, and the alkoxy of C₁₋₆ is selected from methoxy, ethoxy, propoxy, isopropoxy, n-butoxy, isobutoxy, sec-butoxy, tert-butoxy, n-pentyloxy, sec-pentyloxy, 1-ethylpropoxy, 2-methylbutoxy, tert-pentyloxy, 1,2-dimethylpropoxy, isopentyloxy, neopentyloxy, n-hexyloxy, isohexyloxy, sec-hexyloxy, tert-hexyloxy, neohexyloxy, 2-methylpentyloxy, 1,2-dimethylbutoxy, or 1-ethylbutoxy; and alkoxyalkyl is selected from C₁₋₄alkoxy-C₁₋₄alkyl, and the C₁₋₄alkoxy-C₁₋₄alkylis further selected from methoxymethyl, methoxyethyl, methoxypropyl, methoxybutyl, ethoxymethyl, ethoxyethyl, ethoxypropyl, ethoxybutyl, propoxymethyl, propoxyethyl, propoxypropyl, propoxybutyl, butoxymethyl, butoxyethyl, butoxypropyl, or butoxybutyl.

3. The compound as claimed in claim 1, or a racemate, or a geometric or stereoisomeric isomer, or a pharmaceutically acceptable salt thereof, wherein the cycloalkyl is selected from cycloalkylof C₃₋₆, and the cycloalkylof C₃₋₆ is selected from cyclopropyl, cyclobutyl, cyclopentyl, or cyclohexyl.

4. The compound as claimed in claim 1, or a racemate, or a geometric or stereoisomeric isomer, or a pharmaceutically acceptable salt thereof, wherein the aromatic ring is selected from a four-membered ring, a fused ring containing the four-membered ring, a five-membered ring, a fused ring containing the five-membered ring, a six-membered ring, a fused ring containing the six-membered ring, a biphenyl type aromatic ring; and the heteroaromatic ring means that one or more carbon atoms on the aromatic ring are substituted by a heteroatom.

5. The compound as claimed in claim 1, or a racemate, or a geometric or stereoisomeric isomer, or a pharmaceutically acceptable salt thereof, wherein the halogen is selected from fluorine, chlorine, bromine, or iodine; the haloalkyl means that one or more hydrogen atoms on the alkyl are substituted by the halogen; the haloalkoxy means that one or more hydrogen atoms on the alkoxy are substituted by the halogen; and the heterocycloalkyl means that one or more carbon atoms on the cycloalkyl are substituted by a heteroatom; and the alkylcycloalkylmeans that one or more hydrogen atoms on the cycloalkyl are substituted by an alkyl, and the alkyl heterocycloalkyl means that one or more hydrogen atoms on the heterocycloalkyl are substituted by an alkyl.

6. The compound as claimed in claim 1, or a racemate, or a geometric or stereoisomeric isomer, or a pharmaceutically acceptable salt thereof, wherein a heteroatom is selected from nitrogen, oxygen, or sulfur, and there are one or more heteroatoms.

7. The compound as claimed in claim 1, or a racemate, or a geometric or stereoisomeric isomer, or a pharmaceutically acceptable salt thereof, wherein the A ring is selected from

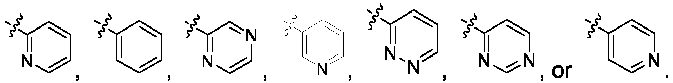

8. The compound as claimed in claim 1, or a racemate, or a geometric or stereoisomeric isomer, or a pharmaceutically acceptable salt thereof, selected from: 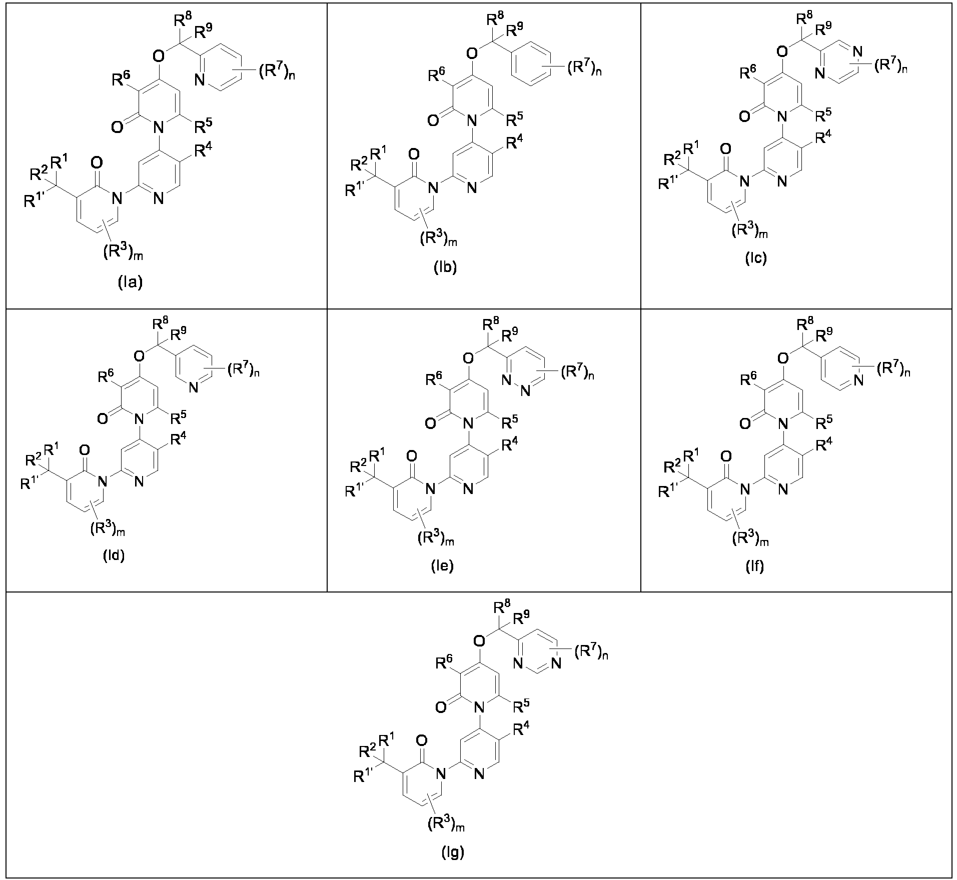
wherein, m is 0, 1, 2, or 3;
n is 0, 1, 2, 3, 4, or 5;
R¹, R^{1'}, R², R³, R⁴, R⁵, R⁶, R⁷, R⁸, and R⁹ are defined as above.

9. The compound as claimed in claim 1, or a racemate, or a geometric or stereoisomeric isomer, or a pharmaceutically acceptable salt thereof, wherein
m is 0 or 1;
n is 0, 1, or 2;
the A ring is selected from 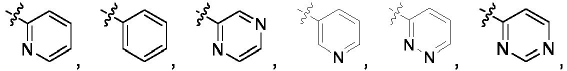 or 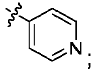
R¹ and R^{1'} are selected from hydrogen, methyl, or ethyl, and R¹ and R^{1'} are cyclized to 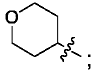
R² is selected from hydrogen, hydroxyl, difluoromethyl, or cyano;
R³ is selected from hydrogen, methyl, methoxy, chlorine, or bromine;
R⁴ is selected from methyl, cyclopropyl, methoxy, ethyl, fluoromethyl, or trifluoromethyl;
R⁵ is selected from methyl;
R⁶ is selected from chlorine, hydrogen, or bromine;
R⁷ is selected from hydrogen, fluorine, chlorine, bromine, cyclopropyl, methoxy, cyano, methyl, or trifluoromethyl; and
R⁸ and R⁹ are hydrogen.

10. The compound as claimed in claim 1, or a racemate, or a geometric or stereoisomeric isomer, or a pharmaceutically acceptable salt thereof, selected from:
| No. | Compound structure | No. | Compound structure |
|---|---|---|---|
| 1 | 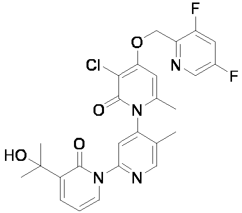 | 2 | 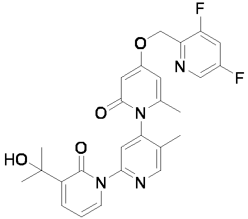 |
| 1A | 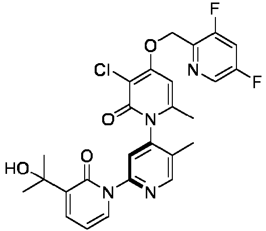 | 1B | 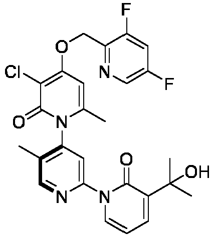 |
| 3 | 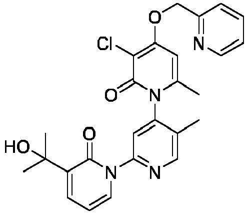 | 4 | 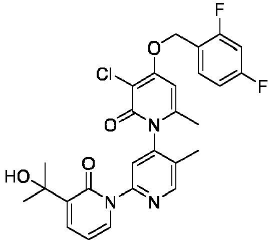 |
| 5 | 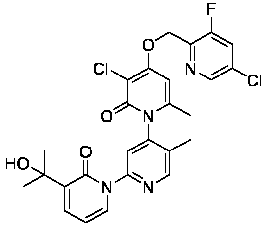 | 6 | 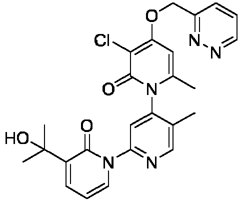 |
| 7 | 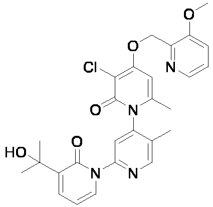 | 8 | 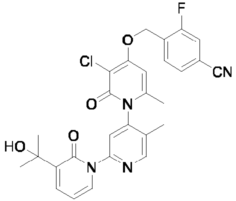 |
| 9 | 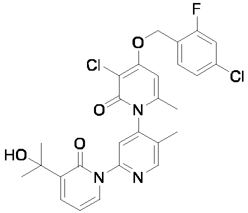 | 10 | 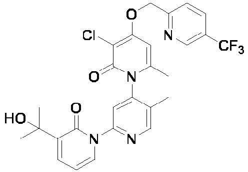 |
| 11 | 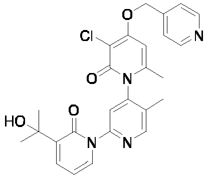 | 12 | 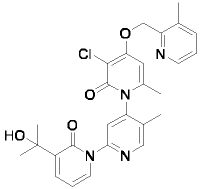 |
| 13 | 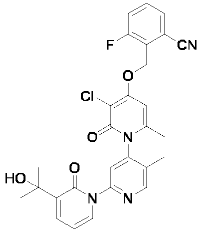 | 14 | 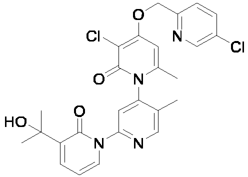 |
| 15 | 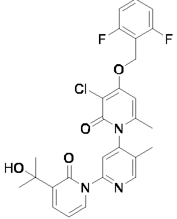 | 16 | 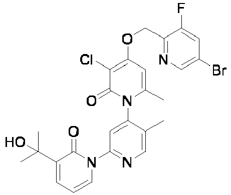 |
| 17 | 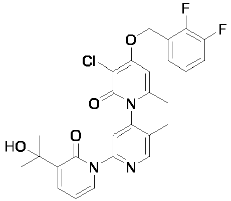 | 18 | 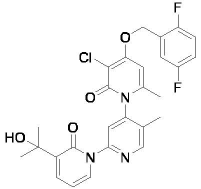 |
| 19 | 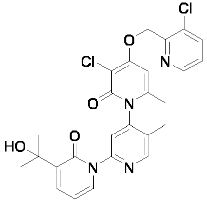 | 20 | 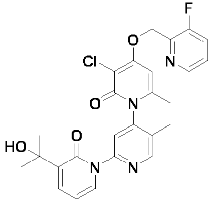 |
| 21 | 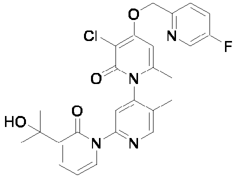 | 22 | 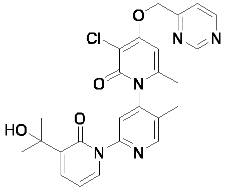 |
| 23 | 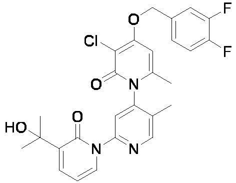 | 24 | 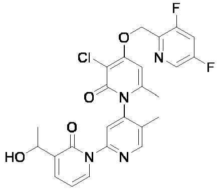 |
| 25 | 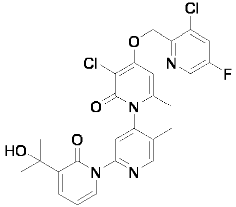 | 26 | 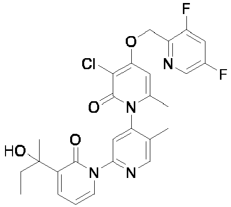 |
| 27 | 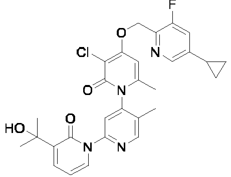 | 28 | 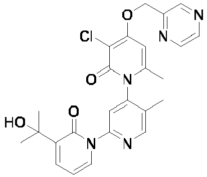 |
| 29 | 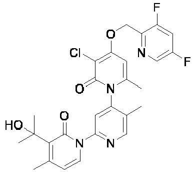 | 30 | 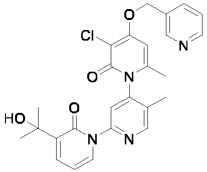 |
| 31 | 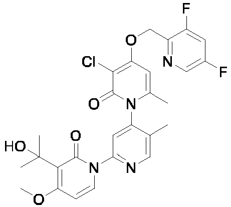 | 32 | 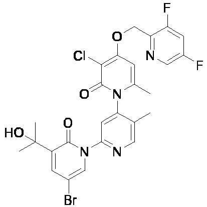 |
| 33 | 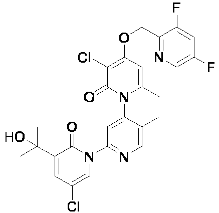 | 34 | 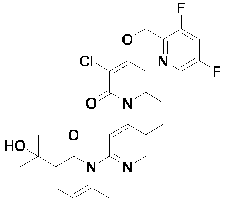 |
| 35 | 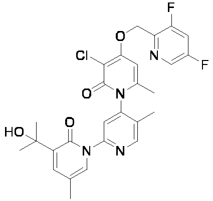 | 36 | 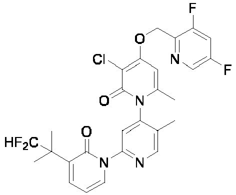 |
| 37 | 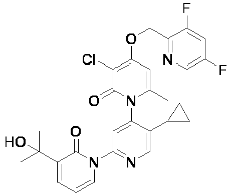 | 38 | 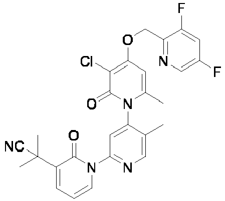 |
| 39 | 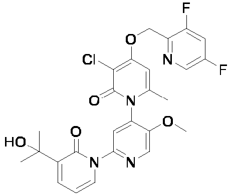 | 40 | 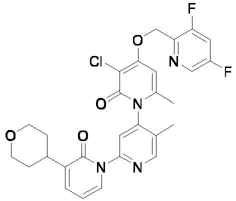 |
| 41 | 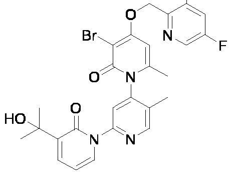 | 42 | 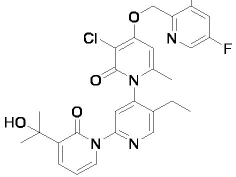 |
| 43 | 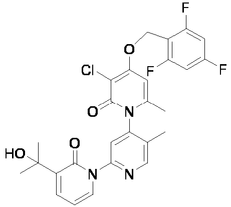 | 44 | 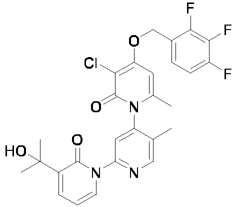 |
| 45 | 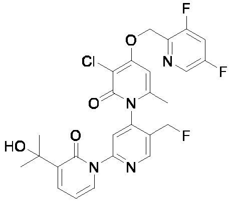 | 46 | 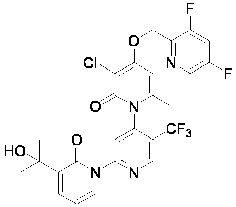 |

11. The compound as claimed in claim 1, or a racemate, or a geometric or stereoisomeric isomer, or a pharmaceutically acceptable salt thereof, wherein the pharmaceutically acceptable salt refers to a salt prepared by the compound, or a racemate or an isomer thereof, and a pharmaceutically acceptable acid or base.

12. A pharmaceutical composition, comprising a therapeutically effective amount of the compound as claimed in any of claims 1 to 11, or a racemate, or a geometric or stereoisomeric isomer, or a pharmaceutically acceptable salt thereof and a pharmaceutically acceptable carrier.

13. A compound as claimed in any of claims 1 to 11, or a racemate, or a geometric or stereoisomeric isomer, or a pharmaceutically acceptable salt thereof for use in the treatment of diseases.

14. A compound for use according to claim 13, wherein the diseases are p38/MK2 related diseases and selected from a chronic inflammatory disease and an acute inflammatory disease.

15. A compound for use according to claim 14, wherein the chronic inflammatory disease is rheumatoid arthritis.

## Patentansprüche

1. Verbindung mit der allgemeinen Formel (I) oder ein Racemat oder ein geometrisches oder stereoisomeres Isomer oder ein pharmazeutisch akzeptables Salz davon, wobei 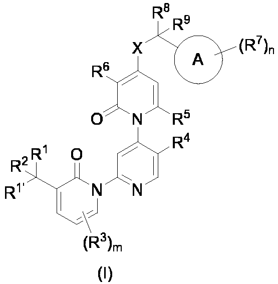
R¹ und R^{1'} unabhängig voneinander ausgewählt sind aus Wasserstoff, Halogen, Alkyl, Cycloalkyl, Heterocycloalkyl, Alkylcycloalkyl, Alkylheterocycloalkyl, Phenyl, Heteroaryl, Halogenalkyl oder Cyano, oder R¹ und R^{1'} zusammen zu Cycloalkyl oder Heterocycloalkyl cyclisiert sind;
R² ausgewählt ist aus Wasserstoff, Halogen, Hydroxyl, Cyano, Alkyl, Alkoxy, Alkoxyalkyl oder Halogenalkyl;
R³ unabhängig ausgewählt ist aus Wasserstoff, Alkyl, Cycloalkyl, Heterocycloalkyl, Halogen, Alkoxy, Cyano, Halogenalkoxy oder Sulfonyl;
m 0, 1, 2 oder 3 ist;
R⁴ ausgewählt ist aus Wasserstoff, Alkyl, Cycloalkyl, Alkoxy oder Halogenalkyl;
R⁵ ausgewählt ist aus Wasserstoff, Alkyl, Cycloalkyl, Alkoxy oder Halogenalkyl;
R⁶ ausgewählt ist aus Wasserstoff, Halogen, Cyano, Alkyl, Cycloalkyl oder Halogenalkyl;
ein Ring A ausgewählt ist aus einem aromatischen Ring oder einem heteroaromatischen Ring;
R⁷ unabhängig ausgewählt ist aus Wasserstoff, Halogen, Cyano, Alkyl, Cycloalkyl, Halogenalkyl, Alkoxy, Halogenalkoxy oder Sulfonyl;
n 0, 1, 2, 3, 4 oder 5 ist;
X O, CH₂ oder NH ist; und
R⁸ und R⁹ unabhängig voneinander ausgewählt sind aus Wasserstoff, Alkyl, Cycloalkyl, Alkylcycloalkyl oder Halogenalkyl, oder R⁸ und R⁹ zusammen zu Cycloalkyl oder Heterocycloalkyl cyclisiert sind.

2. Verbindung nach Anspruch 1 oder ein Racemat oder ein geometrisches oder stereoisomeres Isomer oder ein pharmazeutisch akzeptables Salz davon, wobei
das Alkyl ausgewählt ist aus einem C₁₋₆Alkyl, und das C₁₋₆Alkyl ausgewählt ist aus Methyl, Ethyl, Propyl, Isopropyl, n-Butyl, Isobutyl, sec-Butyl, tert-Butyl, n-Pentyl, sec-Pentyl, 1-Ethylpropyl, 2-Methylbutyl, tert-Pentyl, 1,2-Dimethylpropyl, Isopentyl, Neopentyl, n-Hexyl, Isohexyl, sec-Hexyl, tert-Hexyl, Neohexyl, 2-Methylpentyl, 1,2-Dimethylbutyl oder 1-Ethylbutyl;
Alkoxy ausgewählt ist aus einem C₁₋₆Alkoxy, und das C₁₋₆Alkoxy ausgewählt ist aus Methoxy, Ethoxy, Propoxy, isopropoxy, n-Butoxy, isobutoxy, sec-Butoxy, tert-Butoxy, n-Pentyloxy, sec-Pentyloxy, 1-Ethylpropoxy, 2-Methylbutoxy, tert-Pentyloxy, 1,2-Dimethylpropoxy, Isopentyloxy, Neopentyloxy, n-Hexyloxy, Isohexyloxy, sec-Hexyloxy, tert-Hexyloxy, Neohexyloxy, 2-Methylpentyloxy, 1,2-Dimethylbutoxy, oder 1-Ethylbutoxy; und Alkoxyalkyl ausgewählt ist aus C₁₋₄Alkoxy-C₁₋₄Alkyl, und das C₁₋₄Alkoxy-C₁₋₄Alkyl ferner ausgewählt ist aus Methoxymethyl, Methoxyethyl, Methoxypropyl, Methoxybutyl, Ethoxymethyl, Ethoxyethyl, Ethoxypropyl, Ethoxybutyl, Propoxymethyl, Propoxyethyl, Propoxypropyl, Propoxybutyl, Butoxymethyl, Butoxyethyl, Butoxypropyl oder Butoxybutyl.

3. Verbindung nach Anspruch 1 oder ein Racemat oder ein geometrisches oder stereoisomeres Isomer oder ein pharmazeutisch annehmbares Salz davon, wobei das Cycloalkyl ausgewählt ist aus einem C₃₋₆Cycloalkyl und das C₃₋₆Cycloalkyl ausgewählt ist aus Cyclopropyl, Cyclobutyl, Cyclopentyl oder Cyclohexyl.

4. Verbindung nach Anspruch 1 oder ein Racemat oder ein geometrisches oder stereoisomeres Isomer oder ein pharmazeutisch annehmbares Salz davon, wobei der aromatische Ring ausgewählt ist aus einem viergliedrigen Ring, einem kondensierten Ring, der den viergliedrigen Ring enthält, einem fünfgliedrigen Ring, einem kondensierten Ring, der den fünfgliedrigen Ring enthält, einem sechsgliedrigen Ring, einem kondensierten Ring, der den sechsgliedrigen Ring enthält, einem aromatischen Ring vom Biphenyl-Typ; und der heteroaromatische Ring bedeutet, dass ein oder mehrere Kohlenstoffatome des aromatischen Rings durch ein Heteroatom substituiert sind.

5. Verbindung nach Anspruch 1 oder ein Racemat oder ein geometrisches oder stereoisomeres Isomer oder ein pharmazeutisch verträgliches Salz davon, wobei das Halogen ausgewählt ist aus Fluor, Chlor, Brom oder lod; das Halogenalkyl bedeutet, dass ein oder mehrere Wasserstoffatome des Alkyls durch das Halogen substituiert sind; das Halogenalkoxy bedeutet, dass ein oder mehrere Wasserstoffatome des Alkoxys durch das Halogen substituiert sind; und das Heterocycloalkyl bedeutet, dass ein oder mehrere Kohlenstoffatome des Cycloalkyls durch ein Heteroatom substituiert sind; und das Alkylcycloalkyl bedeutet, dass ein oder mehrere Wasserstoffatome des Cycloalkyls durch ein Alkyl substituiert sind, und das Alkylheterocycloalkyl bedeutet, dass ein oder mehrere Wasserstoffatome des Heterocycloalkyls durch ein Alkyl substituiert sind.

6. Verbindung nach Anspruch 1 oder ein Racemat oder ein geometrisches oder stereoisomeres Isomer oder ein pharmazeutisch akzeptables Salz davon, wobei ein Heteroatom aus Stickstoff, Sauerstoff oder Schwefel ausgewählt ist und ein oder mehrere Heteroatome vorhanden sind.

7. Verbindung nach Anspruch 1 oder ein Racemat oder ein geometrisches oder stereoisomeres Isomer oder ein pharmazeutisch akzeptables Salz davon, wobei der A-Ring ausgewählt ist aus:

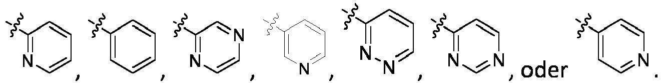

8. Verbindung nach Anspruch 1 oder ein Racemat oder ein geometrisches oder stereoisomeres Isomer oder ein pharmazeutisch akzeptables Salz davon, ausgewählt aus: 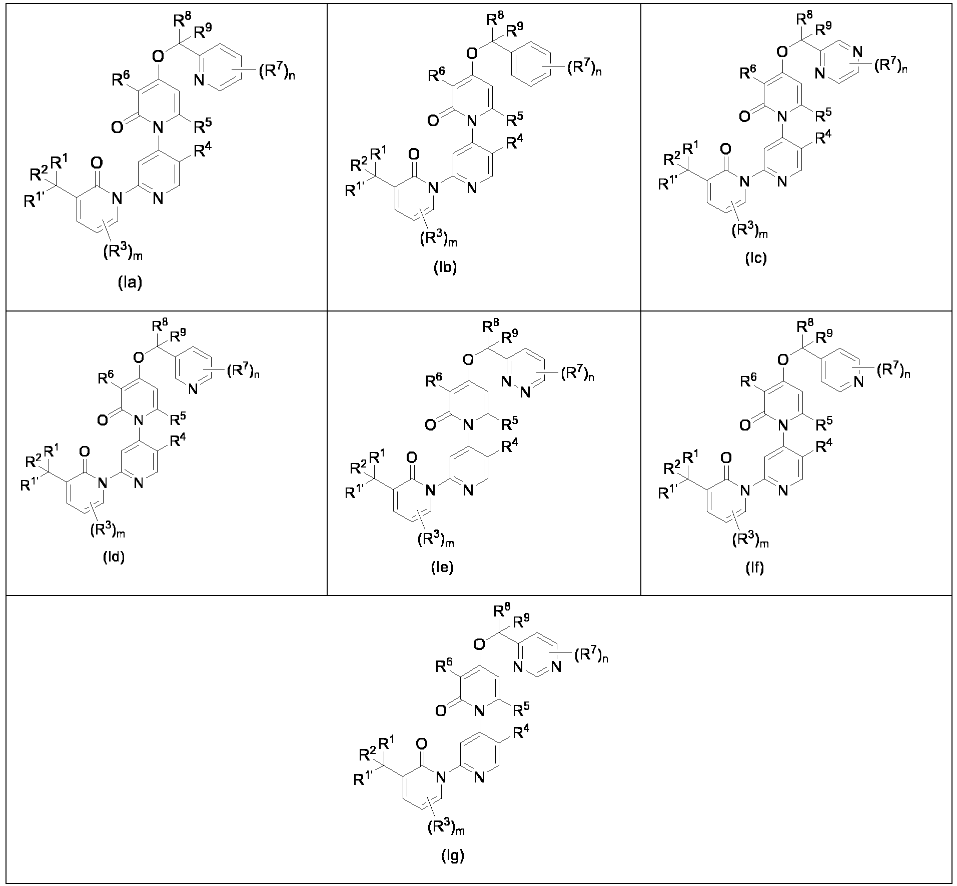 wobei
m 0, 1, 2 oder 3 ist;
n 0, 1, 2, 3, 4 oder 5 ist;
R¹, R^{1'}, R², R³, R⁴, R⁵, R⁶, R⁷, R⁸, und R⁹ wie oben definiert sind.

9. Verbindung nach Anspruch 1 oder ein Racemat oder ein geometrisches oder stereoisomeres Isomer oder ein pharmazeutisch akzeptables Salz davon, wobei
m 0 oder 1 ist;
n 0, 1 oder 2 ist;
der Ring A ausgewählt ist aus 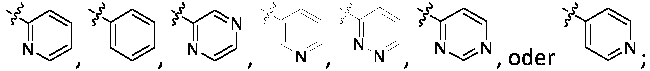
R¹ und R^{1'} ausgewählt sind aus Wasserstoff, Methyl oder Ethyl, und R¹ und R^{1'} cyclisiert sind zu: 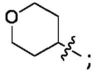
R² ausgewählt ist aus Wasserstoff, Hydroxyl, Difluormethyl oder Cyano;
R³ ausgewählt ist aus Wasserstoff, Methyl, Methoxy, Chlor oder Brom;
R⁴ ausgewählt ist aus Methyl, Cyclopropyl, Methoxy, Ethyl, Fluormethyl oder Trifluormethyl;
R⁵ ausgewählt ist aus Methyl;
R⁶ ausgewählt ist aus Chlor, Wasserstoff, oder Brom;
R⁷ ausgewählt ist aus Wasserstoff, Fluor, Chlor, Brom, Cyclopropyl, Methoxy, Cyano, Methyl oder Trifluormethyl; und
R⁸ und R⁹ Wasserstoff sind.

10. Verbindung nach Anspruch 1 oder ein Racemat oder ein geometrisches oder stereoisomeres Isomer oder ein pharmazeutisch akzeptables Salz davon, ausgewählt aus:
| Nr. | Struktur der Verbindung | Nr. | Struktur der Verbindung |
|---|---|---|---|
| 1 | 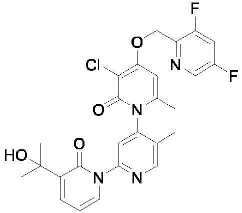 | 2 | 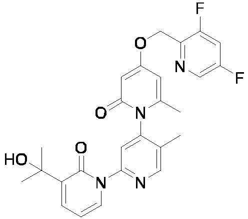 |
| 1A | 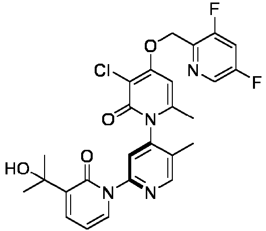 | 1B | 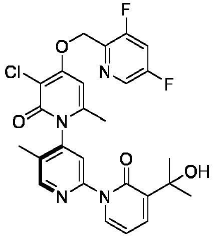 |
| 3 | 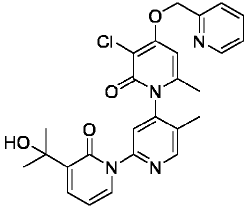 | 4 | 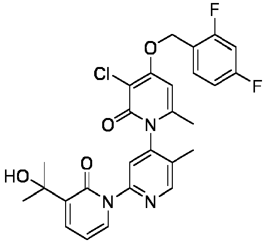 |
| 5 | 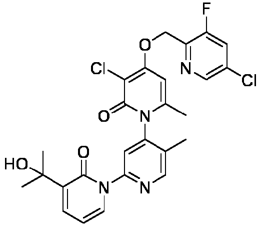 | 6 | 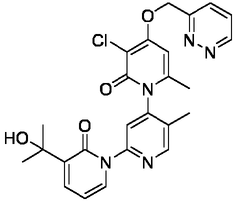 |
| 7 | 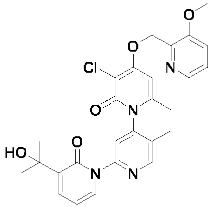 | 8 | 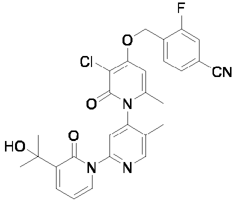 |
| 9 | 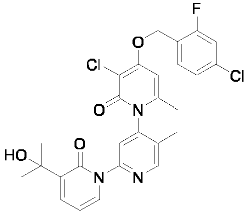 | 10 | 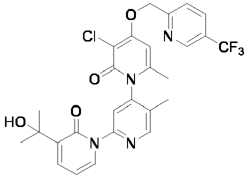 |
| 11 | 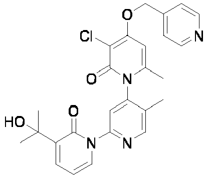 | 12 | 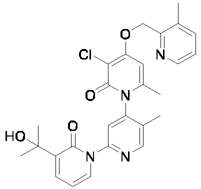 |
| 13 | 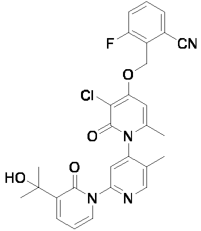 | 14 | 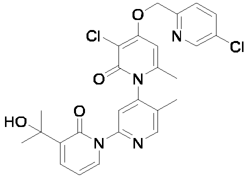 |
| 15 | 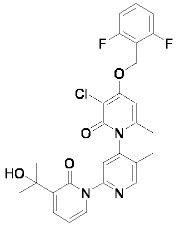 | 16 | 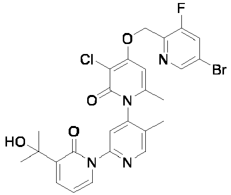 |
| 17 | 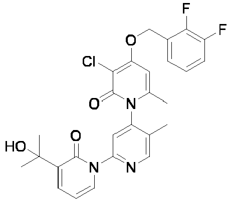 | 18 | 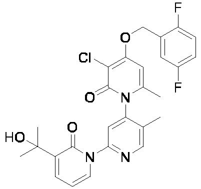 |
| 19 | 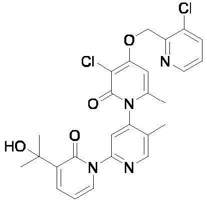 | 20 | 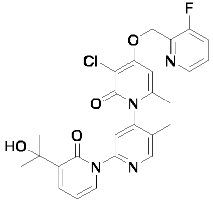 |
| 21 | 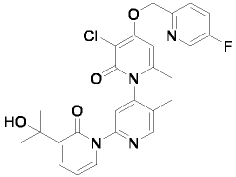 | 22 | 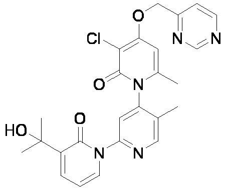 |
| 23 | 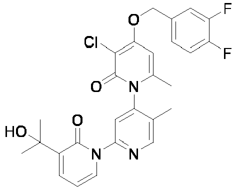 | 24 | 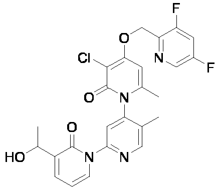 |
| 25 | 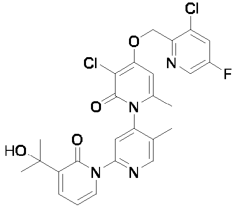 | 26 | 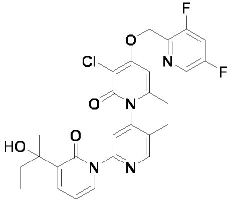 |
| 27 | 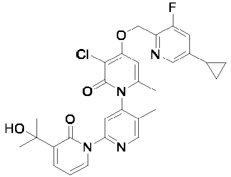 | 28 | 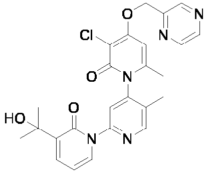 |
| 29 | 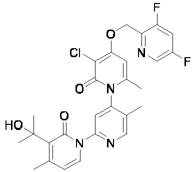 | 30 | 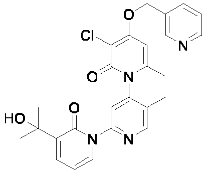 |
| 31 | 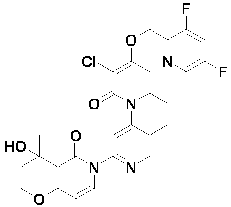 | 32 | 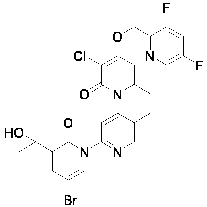 |
| 33 | 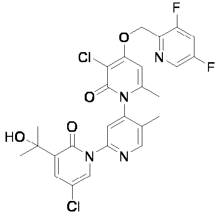 | 34 | 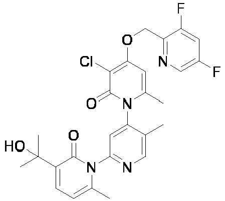 |
| 35 | 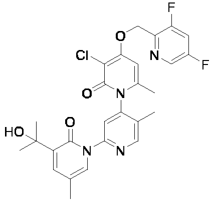 | 36 | 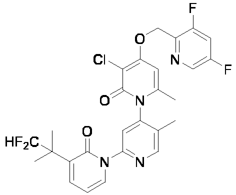 |
| 37 | 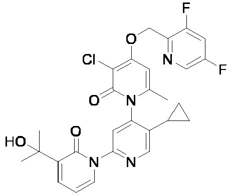 | 38 | 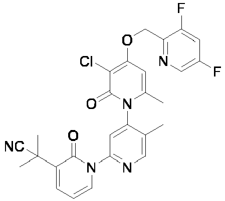 |
| 39 | 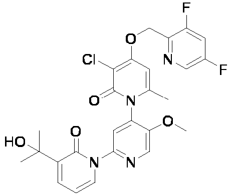 | 40 | 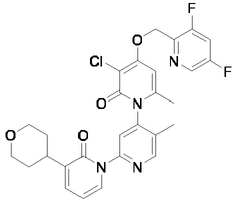 |
| 41 | 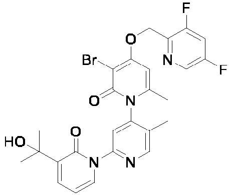 | 42 | 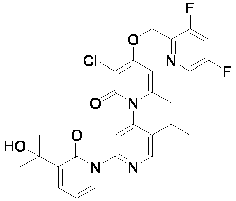 |
| 43 | 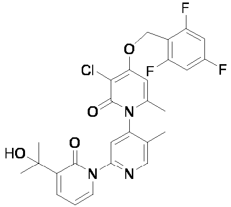 | 44 | 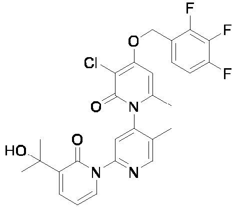 |
| 45 | 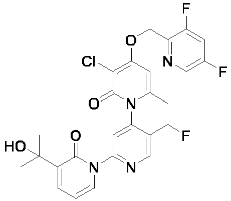 | 46 | 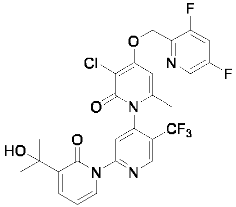 |

11. Verbindung nach Anspruch 1 oder ein Racemat oder ein geometrisches oder stereoisomeres Isomer oder ein pharmazeutisch verträgliches Salz davon, wobei sich das pharmazeutisch verträgliche Salz auf ein Salz bezieht, das aus der Verbindung oder einem Racemat oder einem Isomer davon und einer pharmazeutisch verträglichen Säure oder Base hergestellt wird.

12. Pharmazeutische Zusammensetzung, die eine therapeutisch wirksame Menge der Verbindung nach einem der Ansprüche 1 bis 11 oder ein Racemat oder ein geometrisches oder stereoisomeres Isomer oder ein pharmazeutisch akzeptables Salz davon und einen pharmazeutisch akzeptablen Träger umfasst.

13. Verbindung nach einem der Ansprüche 1 bis 11 oder ein Racemat oder ein geometrisches oder stereoisomeres Isomer oder ein pharmazeutisch akzeptables Salz davon zur Verwendung bei der Behandlung von Krankheiten.

14. Verbindung zur Verwendung nach Anspruch 13, wobei die Krankheiten mit p38/MK2 zusammenhängende Krankheiten sind und ausgewählt sind aus einer chronischen Entzündungskrankheit und einer akuten Entzündungskrankheit.

15. Verbindung zur Verwendung nach Anspruch 14, wobei die chronische Entzündungskrankheit rheumatoide Arthritis ist.

## Revendications

1. Composé représenté par la formule générale (I), ou racémate, ou isomère géométrique ou stéréo-isomère, ou sel pharmaceutiquement acceptable de celui-ci, dans lequel 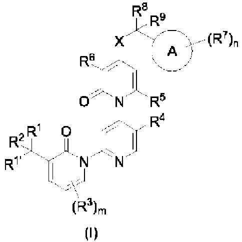
R¹ et R^{1'}sont indépendamment choisis parmi hydrogène, halogène, alkyle, cycloalkyle, hétérocycloalkyle, alkylcycloalkyle, alkyle hétérocycloalkyle, phényle, hétéroaryle, haloalkyle ou cyano, ou R¹ et R^{1'} sont cyclisés en cycloalkyle ou hétérocycloalkyle ensemble ;
R² est choisi parmi hydrogène, halogène, hydroxyle, cyano, alkyle, alcoxy, alcoxyalkyle ou haloalkyle ;
R³ est indépendamment choisi parmi hydrogène, alkyle, cycloalkyle, hétérocycloalkyle, halogène, alcoxy, cyano, haloalcoxy ou sulfonyle ;
m est 0, 1,2 ou 3 ;
R⁴ est choisi parmi hydrogène, alkyle, cycloalkyle, alcoxy ou haloalkyle ;
R⁵ est choisi parmi hydrogène, alkyle, cycloalkyle, alcoxy ou haloalkyle ;
R⁶ est choisi parmi hydrogène, halogène, cyano, alkyle, cycloalkyle ou haloalkyle ;
un cycle A est choisi parmi un cycle aromatique ou un cycle hétéroaromatique ;
R⁷ est indépendamment choisi parmi hydrogène, halogène, cyano, alkyle, cycloalkyle, haloalkyle, alcoxy, haloalcoxy ou sulfonyle ;
n est 0, 1, 2, 3, 4, ou 5 ;
X est O, CH₂ ou NH ; et
R⁸ et R⁹ sont indépendamment choisis parmi hydrogène, alkyle, cycloalkyle, alkylcycloalkyle ou haloalkyle, ou R⁸ et R⁹ sont cyclisés en cycloalkyle ou en hétérocycloalkyle ensemble.

2. Composé selon la revendication 1, ou racémate, ou isomère géométrique ou stéréo-isomère, ou un sel pharmaceutiquement acceptable de celui-ci, dans lequel
l'alkyle est choisi parmi un alkyle de C₁ à C₆ et l'alkyle de C₁ à C₆ est choisi parmi méthyle, éthyle, propyle, isopropyle, n-butyle, isobutyle, sec-butyle, tert-butyle, n-pentyle, sec-pentyle, 1-éthylpropyle, 2-méthylbutyle, tert-pentyle, 1,2-diméthylpropyle, isopentyle, néopentyle, n-hexyle, isohexyle, sec-hexyle, tert-hexyle, néohexyle, 2-méthylpentyle, 1,2-diméthylbutyle ou 1-éthylbutyle ;
l'alcoxy est choisi parmi un alcoxy de C₁ à C₆, et l'alcoxy de C₁ à C₆ est choisi parmi méthoxy, éthoxy, propoxy, isopropoxy, n-butoxy, isobutoxy, sec-butoxy, tert-butoxy, n-pentyloxy, sec-pentyloxy, 1-éthylpropoxy, 2-méthylbutoxy, tert-pentyloxy, 1,2-diméthylpropoxy, isopentyloxy, néopentyloxy, n-hexyloxy, isohexyloxy, sec-hexyloxy, tert-hexyloxy, néohexyloxy, 2-méthylpentyloxy, 1,2-diméthylbutoxy ou 1-éthylbutoxy ; et l'alcoxyalkyle est choisi parmi un alcoxy en C₁₋₄ (alkyle en C ₁₋₄), et l'alcoxy en C₁₋₄(alkyle en C ₁₋₄) est en outre choisi parmi méthoxyméthyle, méthoxyéthyle, méthoxypropyle, méthoxybutyle, éthoxyméthyle, éthoxyéthyle, éthoxypropyle, éthoxybutyle, propoxyméthyle, propoxyéthyle, propoxypropyle, propoxybutyle, butoxyméthyle, butoxyéthyle, butoxypropyle ou butoxybutyle.

3. Composé selon la revendication 1, ou racémate, ou isomère géométrique ou stéréo-isomère, ou un sel pharmaceutiquement acceptable de celui-ci, dans lequel le cycloalkyle est choisi parmi un cycloalkyle de C₃ à C₆, et le cycloalkyle de C₃ à C₆ est choisi parmi cyclopropyle, cyclobutyle, cyclopentyle ou cyclohexyle.

4. Composé selon la revendication 1, ou un racémate, ou un isomère géométrique ou stéréo-isomère, ou un sel pharmaceutiquement acceptable de celui-ci, dans lequel le cycle aromatique est choisi parmi un cycle à quatre éléments, un cycle fusionné contenant le cycle à quatre éléments, un cycle à cinq éléments, un cycle fusionné contenant le cycle à cinq éléments, un cycle à six éléments, un cycle fusionné contenant le cycle à six éléments, un cycle aromatique de type biphényle ; et le cycle hétéroaromatique signifie qu'un ou plusieurs atomes de carbone sur le cycle aromatique sont substitués par un hétéroatome.

5. Composé selon la revendication 1, ou racémate, ou isomère géométrique ou stéréo-isomère, ou un sel pharmaceutiquement acceptable de celui-ci, dans lequel l'halogène est choisi parmi fluor, chlore, brome ou iode ; l'haloalkyle signifie qu'un ou plusieurs atomes d'hydrogène sur l'alkyle sont substitués par l'halogène ; l'haloalcoxy signifie qu'un ou plusieurs atomes d'hydrogène sur l'alcoxy sont substitués par l'halogène ; et l'hétérocycloalkyle signifie qu'un ou plusieurs atomes de carbone sur le cycloalkyle sont substitués par un hétéroatome ; et l'alkylcycloalkyle signifie qu'un ou plusieurs atomes d'hydrogène sur le cycloalkyle sont substitués par un alkyle, et l'alkylhétérocycloalkyle signifie qu'un ou plusieurs atomes d'hydrogène sur l'hétérocycloalkyle sont substitués par un alkyle.

6. Composé selon la revendication 1, ou racémate, ou un isomère géométrique ou stéréo-isomère, ou un sel pharmaceutiquement acceptable de celui-ci, dans lequel un hétéroatome est choisi parmi azote, oxygène ou soufre, et il existe un ou plusieurs hétéroatomes.

7. Composé selon la revendication 1, ou racémate, ou isomère géométrique ou stéréo-isomère, ou un sel pharmaceutiquement acceptable de celui-ci, dans lequel le cycle A est choisi parmi

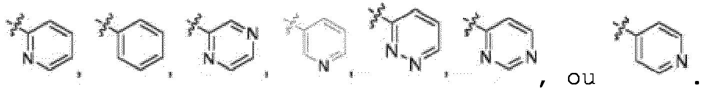

8. Composé selon la revendication 1, ou racémate, ou isomère géométrique ou stéréo-isomère, ou un sel pharmaceutiquement acceptable de celui-ci, choisi parmi : 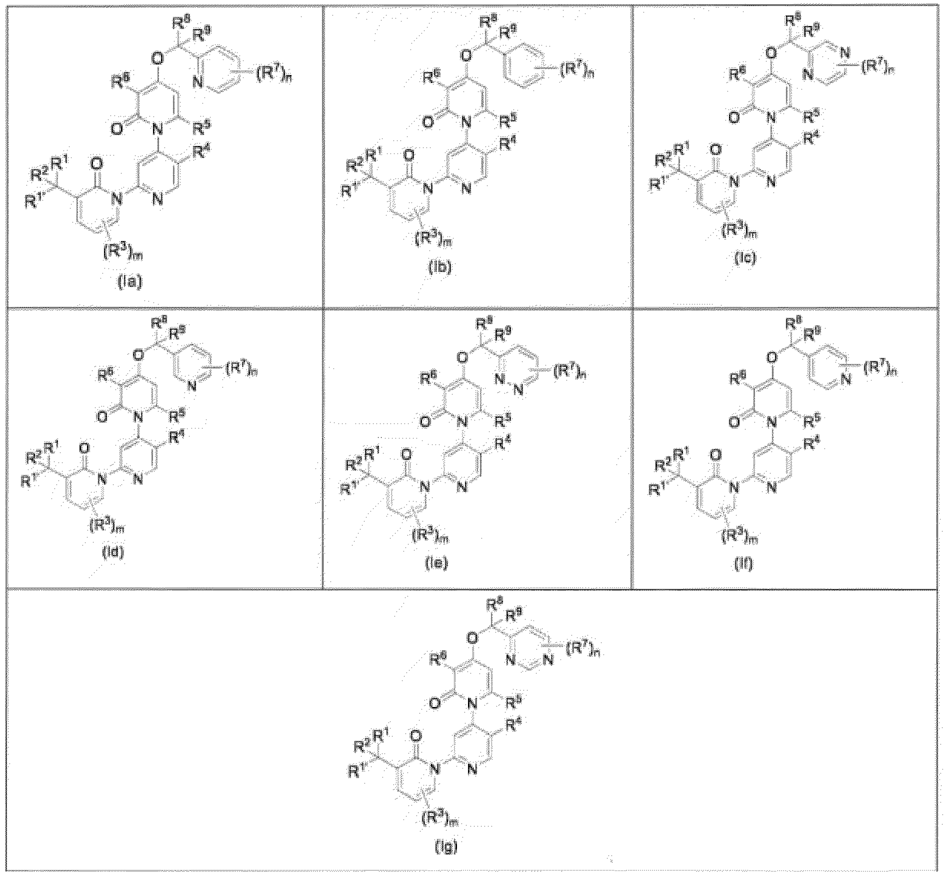
où, m est 0, 1, 2 ou 3 ;
n est 0, 1, 2, 3, 4, ou 5 ;
R¹, R^{1'}, R², R³, R⁴, R⁵, R⁶, R⁷, R⁸ et R⁹ sont définis comme ci-dessus.

9. Composé selon la revendication 1, ou racémate, ou isomère géométrique ou stéréo-isomère, ou un sel pharmaceutiquement acceptable de celui-ci, dans lequel
m est 0 ou 1 ;
n est 0, 1 ou 2 ;
le cycle A est sélectionné parmi 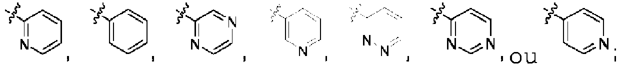
R¹ et R^{1'}sont choisis parmi hydrogène, méthyle ou éthyle, et R¹ et R^{1'} sont cyclisés en 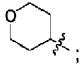
R² est choisi parmi hydrogène, hydroxyle, difluorométhyle ou cyano ;
R³ est choisi parmi hydrogène, méthyle, méthoxy, chlore ou brome ;
R⁴ est choisi parmi méthyle, cyclopropyle, méthoxy, éthyle, fluorométhyle ou trifluorométhyle ;
R⁵ est choisi parmi méthyle ;
R⁶ est choisi parmi chlore, hydrogène ou brome ;
R⁷ est choisi parmi hydrogène, fluor, chlore, brome, cyclopropyle, méthoxy, cyano, méthyle ou trifluorométhyle ; et
R⁸ et R⁹ sont un hydrogène.

10. Composé selon la revendication 1, ou racémate, ou isomère géométrique ou stéréo-isomère, ou un sel pharmaceutiquement acceptable de celui-ci, choisi parmi :
| N° | Structure du composé | N° | Structure du composé |
|---|---|---|---|
| 1 | 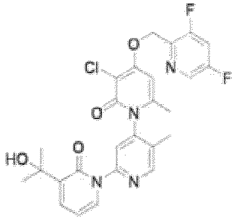 | 2 | 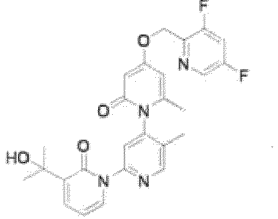 |
| 1A | 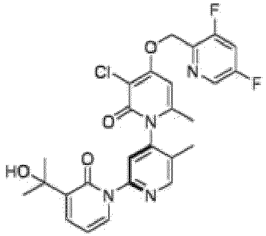 | 1B | 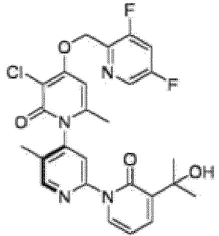 |
| 3 | 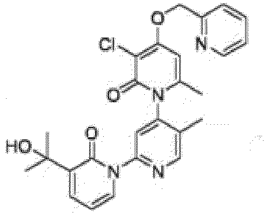 | 4 | 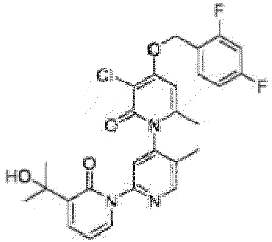 |
| 5 | 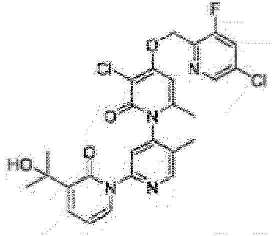 | 6 | 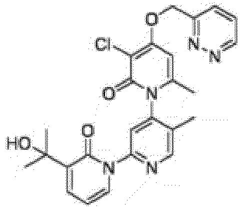 |
| 7 | 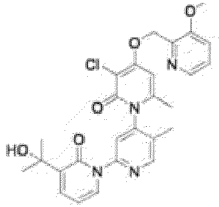 | 8 | 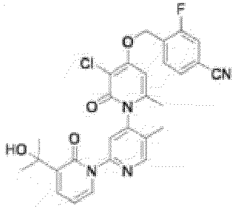 |
| 9 | 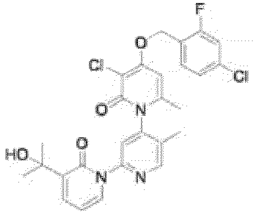 | 10 | 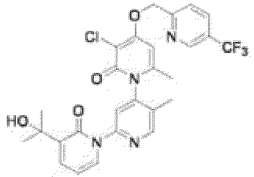 |
| 11 | 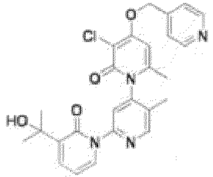 | 12 | 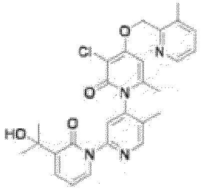 |
| 13 | 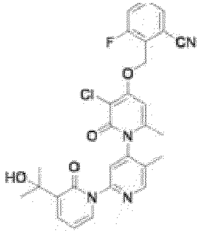 | 14 | 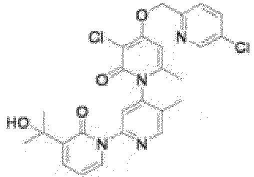 |
| 15 | 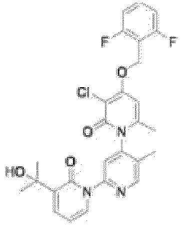 | 16 | 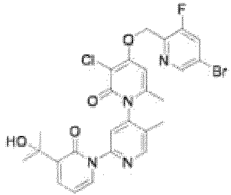 |
| 17 | 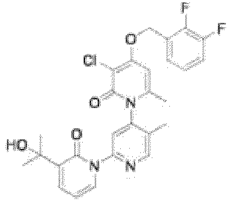 | 18 | 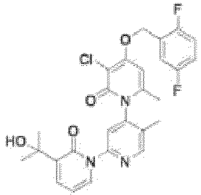 |
| 19 | 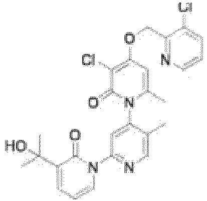 | 20 | 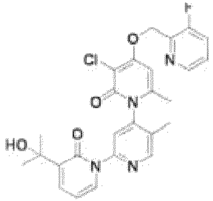 |
| 21 | 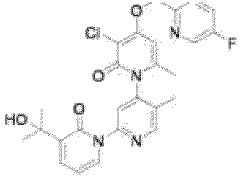 | 22 | 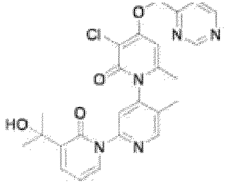 |
| 23 | 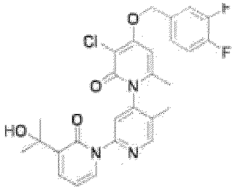 | 24 | 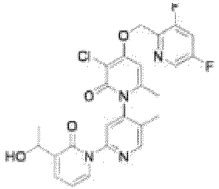 |
| 25 | 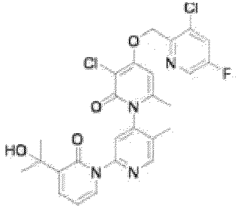 | 26 | 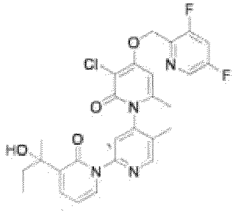 |
| 27 | 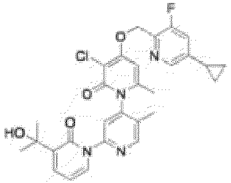 | 28 | 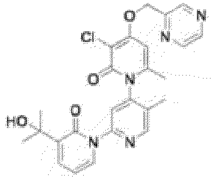 |
| 29 | 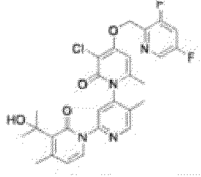 | 30 | 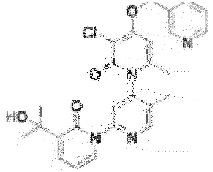 |
| 31 | 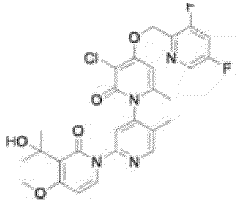 | 32 | 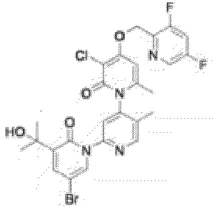 |
| 33 | 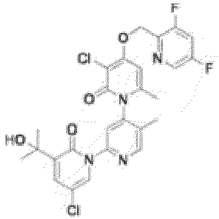 | 34 | 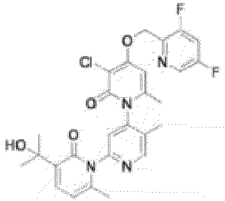 |
| 35 | 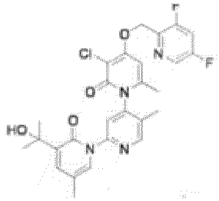 | 36 | 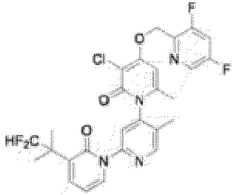 |
| 37 | 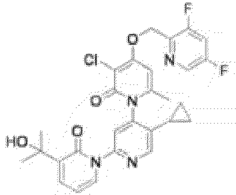 | 38 | 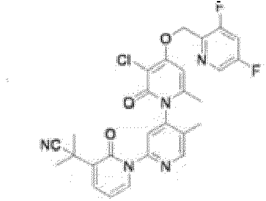 |
| 39 | 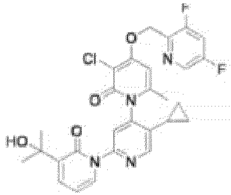 | 40 | 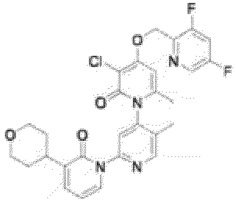 |
| 41 | 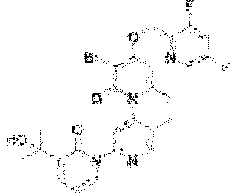 | 42 | 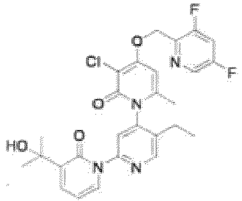 |
| 43 | 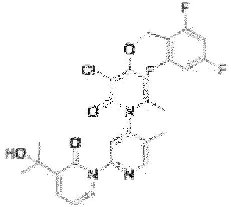 | 44 | 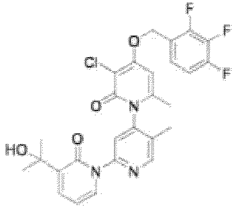 |
| 45 | 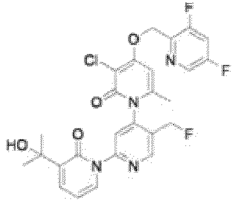 | 46 | 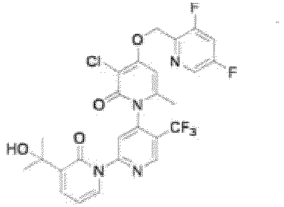 |

11. Composé selon la revendication 1, ou racémate, ou isomère géométrique ou stéréo-isomère, ou un sel pharmaceutiquement acceptable de celui-ci, dans lequel le sel pharmaceutiquement acceptable se réfère à un sel préparé par le composé, ou un racémate ou un isomère de celui-ci, et un acide ou une base pharmaceutiquement acceptable.

12. Composition pharmaceutique, comprenant une quantité thérapeutiquement efficace du composé selon l'une quelconque des revendications 1 à 11, ou un racémate, ou un isomère géométrique ou stéréo-isomère, ou un sel pharmaceutiquement acceptable de celui-ci et un support pharmaceutiquement acceptable.

13. Composé selon l'une quelconque des revendications 1 à 11, ou racémate, ou isomère géométrique ou stéréo-isomère, ou sel pharmaceutiquement acceptable de celui-ci destiné à être utilisé dans le traitement de maladies.

14. Composé destiné à être utilisé selon la revendication 13, dans lequel les maladies sont des maladies liées à p38/MK2 et choisies parmi une maladie inflammatoire chronique et une maladie inflammatoire aiguë.

15. Composé destiné à être utilisé selon la revendication 14, dans lequel la maladie inflammatoire chronique est la polyarthrite rhumatoïde.
